# EUROPEAN PATENT SPECIFICATION

(11) **EP 2 244 729 B1**
(45) Date of publication and mention of the grant of the patent: **02.11.2016**
(21) Application number: 09702787.4
(22) Date of filing: 16.01.2009
(51) Int. Cl.: A61K 39/00, C07K 16/00, A61K 51/10, C07K 16/28, A61K 47/48

(54) **CYSTEINE ENGINEERED ANTIBODIES FOR SITE-SPECIFIC CONJUGATION**
CYSTEIN-MANIPULIERTE ANTIKÖRPER ZUR STELLENSPEZIFISCHEN KONJUGATION
ANTICORPS MODIFIÉS PAR L'INTRODUCTION DE CYSTEINE POUR CONJUGAISON SPÉCIFIQUE

(30) Priority: 18.01.2008 US 22073 P
(43) Date of publication of application: 03.11.2010
(73) Proprietor: MedImmune, LLC, Gaithersburg, MD 20878 (US)
(72) Inventor: DIMASI, Nazzareno, Gaithersburg, MD 20878 (US); GAO, Changshou, Potomac, MD 20854 (US); WU, Herren, Boyds, MD 20841 (US)
(74) Representative: Winter, Christopher Spencer
(86) International application number: PCT/US2009/031294
(87) International publication number: WO 2009/092011

(56) References cited:
- WO-A2-2006/034488
- US-A1- 2007 036 799
- US-A1- 2007 092 940
- US-A1- 2007 197 439
- SHOPES B: "A genetically engineered human IgG mutant with enhanced cytolytic activity.", JOURNAL OF IMMUNOLOGY (BALTIMORE, MD. : 1950) 1 MAY 1992, vol. 148, no. 9, 1 May 1992 (1992-05-01), pages 2918-2922, XP002714200, ISSN: 0022-1767
- STIMMEL J B ET AL: "Site-specific conjugation on serine right-arrow cysteine variant monoclonal antibodies.", THE JOURNAL OF BIOLOGICAL CHEMISTRY 29 SEP 2000, vol. 275, no. 39, 29 September 2000 (2000-09-29), pages 30445-30450, XP002714201, ISSN: 0021-9258
- ALBRECHT H ET AL: "Monospecific bivalent scFv-SH: Effects of linker length and location of an engineered cysteine on production, antigen binding activity and free SH accessibility", JOURNAL OF IMMUNOLOGICAL METHODS, ELSEVIER SCIENCE PUBLISHERS B.V.,AMSTERDAM, NL, vol. 310, no. 1-2, 20 March 2006 (2006-03-20), pages 100-116, XP028017570, ISSN: 0022-1759, DOI: 10.1016/J.JIM.2005.12.012 [retrieved on 2006-03-20]
- ALBRECHT H ET AL: "Production of Soluble ScFvs with C-Terminal-Free Thiol for Site-Specific Conjugation or Stable Dimeric ScFvs on Demand", BIOCONJUGATE CHEMISTRY, ACS, WASHINGTON, DC, US, vol. 15, 1 January 2004 (2004-01-01), pages 16-26, XP002458164, ISSN: 1043-1802, DOI: 10.1021/BC030018+
- XIONG CHENG-YI ET AL: "Development of tumor targeting anti-MUC-1 multimer: effects of di-scFv unpaired cysteine location on PEGylation and tumor binding.", PROTEIN ENGINEERING, DESIGN & SELECTION : PEDS AUG 2006, vol. 19, no. 8, August 2006 (2006-08), pages 359-367, XP002714202, ISSN: 1741-0126
- LYONS A ET AL: "SITE-SPECIFIC ATTACHMENT TO RECOMBINANT ANTIBODIES VIA INTRODUCED SURFACE CYSTEINE RESIDUES", PROTEIN ENGINEERING, OXFORD UNIVERSITY PRESS, SURREY, GB, vol. 3, no. 8, 1 January 1990 (1990-01-01) , pages 703-708, XP001000052, ISSN: 0269-2139
- VIDARTE L ET AL: "Serine 132 is the C3 covalent attachment point on the CH1 domain of human IgG1.", THE JOURNAL OF BIOLOGICAL CHEMISTRY 12 OCT 2001, vol. 276, no. 41, 12 October 2001 (2001-10-12), pages 38217-38223, ISSN: 0021-9258

### SEQUENCE LISTING

<110> MedImmune, LLC.
<120> Cysteine Engineered Antibodies for Site-Specific Conjugation
<130> MED0032.EP
<140> EP09702787.4
   <141> 2009-01-16
<150> 61/022,073
   <151> 2008-01-18
<160> 11
<170> PatentIn version 3.5
<210> 1
   <211> 214
   <212> PRT
   <213> Artificial Sequence
<220>
   <223> synthetic construct
<400> 1
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Ala
               20                  25                  30
   Leu Ala Trp Tyr Gln Gln Lys Pro Gly Lys Ala Pro Glu Leu Leu Ile
           35                  40                  45
   Tyr Asp Ala Ser Ser Leu Glu Ser Gly Val Pro Ser Arg Phe Ser Gly
       50                  55                  60
   Ser Gly Ser Gly Thr Asp Phe Ile Leu Thr Ile Ser Ser Leu Gln Pro
   65                  70                  75                  80
   Glu Asp Phe Ala Thr Tyr Tyr Cys Gln Gln Phe Asn Ser Tyr Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 2
   <211> 453
   <212> PRT
   <213> Artificial Sequence
<220>
   <223> Synthetic construct
<400> 2
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Phe Tyr
               20                  25                  30
   Gln Met Gly Trp Val Arg Gln Ala Pro Gly Lys Gly Leu Glu Trp Val
           35                  40                  45
   Ser Tyr Ile Ser Pro Ser Gly Gly Gly Thr Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asn Leu Tyr Ser Gly Tyr Asp Pro Thr Leu Asp Tyr
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Arg Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Glu Glu Met Thr Lys Asn Gln
           355                 360                 365
   Val Ser Leu Thr Cys Leu Val Lys Gly Phe Tyr Pro Ser Asp Ile Ala
       370                 375                 380
   Val Glu Trp Glu Ser Asn Gly Gln Pro Glu Asn Asn Tyr Lys Thr Thr
   385                 390                 395                 400
   Pro Pro Val Leu Asp Ser Asp Gly Ser Phe Phe Leu Tyr Ser Lys Leu
                   405                 410                 415
   Thr Val Asp Lys Ser Arg Trp Gln Gln Gly Asn Val Phe Ser Cys Ser
               420                 425                 430
   Val Met His Glu Ala Leu His Asn His Tyr Thr Gln Lys Ser Leu Ser
           435                 440                 445
   Leu Ser Pro Gly Lys
   450
<210> 3
   <211> 97
   <212> PRT
   <213> Artificial Sequence
<220>
   <223> synthetic construct
<400> 3
   Ser Thr Lys Gly Pro Ser Val Phe Pro Leu Ala Pro Cys Ser Arg Ser
   1               5                   10                  15
   Thr Ser Glu Ser Thr Ala Ala Leu Gly Cys Leu Val Lys Asp Tyr Phe
               20                  25                  30
   Pro Glu Pro Val Thr Val Ser Trp Asn Ser Gly Ala Leu Thr Ser Gly
           35                  40                  45
   Val His Thr Phe Pro Ala Val Leu Gln Ser Ser Gly Leu Tyr Ser Leu
       50                  55                  60
   Ser Ser Val Val Thr Val Pro Ser Ser Asn Phe Gly Thr Gln Thr Tyr
   65                  70                  75                  80
   Thr Cys Asn Val Asp His Lys Pro Ser Asn Thr Lys Val Asp Lys Thr
                   85                  90                  95
   Val
<210> 4
   <211> 97
   <212> PRT
   <213> Artificial Sequence
<220>
   <223> synthetic construct
<400> 4
   Ser Thr Lys Gly Pro Ser Val Phe Pro Leu Ala Pro Cys Ser Arg Ser
   1               5                   10                  15
   Thr Ser Glu Ser Thr Ala Ala Leu Gly Cys Leu Val Lys Asp Tyr Phe
               20                  25                  30
   Pro Glu Pro Val Thr Val Ser Trp Asn Ser Gly Ala Leu Thr Ser Gly
           35                  40                  45
   Val His Thr Phe Pro Ala Val Leu Gln Ser Ser Gly Leu Tyr Ser Leu
       50                  55                  60
   Ser Ser Val Val Thr Val Pro Ser Ser Ser Leu Gly Thr Lys Thr Tyr
   65                  70                  75                  80
   Thr Cys Asn Val Asp His Lys Pro Ser Asn Thr Lys Val Asp Lys Arg
                   85                  90                  95
   Val
<210> 5
   <211> 97
   <212> PRT
   <213> Artificial Sequence
<220>
   <223> synthetic construct
<400> 5
   Ser Thr Lys Gly Pro Ser Val Phe Pro Leu Ala Pro Ser Ser Lys Ser
   1               5                   10                  15
   Thr Ser Gly Gly Thr Ala Ala Leu Gly Cys Leu Val Lys Asp Tyr Phe
               20                  25                  30
   Pro Glu Pro Val Thr Val Ser Trp Asn Ser Gly Ala Leu Thr Ser Gly
           35                  40                  45
   Val His Thr Phe Pro Ala Val Leu Gln Ser Ser Gly Leu Tyr Ser Leu
       50                  55                  60
   Ser Ser Val Val Thr Val Pro Ser Ser Ser Leu Gly Thr Gln Thr Tyr
   65                  70                  75                  80
   Ile Cys Asn Val Asn His Lys Pro Ser Asn Thr Lys Val Asp Lys Lys
                   85                  90                  95
   Val
<210> 6
   <211> 97
   <212> PRT
   <213> Artificial Sequence
<220>
   <223> synthetic construct
<400> 6
   Ser Thr Lys Gly Pro Ser Val Phe Pro Leu Ala Pro Cys Ser Arg Ser
   1               5                   10                  15
   Thr Ser Gly Gly Thr Ala Ala Leu Gly Cys Leu Val Lys Asp Tyr Phe
               20                  25                  30
   Pro Glu Pro Val Thr Val Ser Trp Asn Ser Gly Ala Leu Thr Ser Gly
           35                  40                  45
   Val His Thr Phe Pro Ala Val Leu Gln Ser Ser Gly Leu Tyr Ser Leu
       50                  55                  60
   Ser Ser Val Val Thr Val Pro Ser Ser Ser Leu Gly Thr Gln Thr Tyr
   65                  70                  75                  80
   Ile Cys Asn Val Asn His Lys Pro Ser Asn Thr Lys Val Asp Lys Lys
                   85                  90                  95
   Val
<210> 7
   <211> 102
   <212> PRT
   <213> Artificial Sequence
<220>
   <223> synthetic construct
<400> 7
   Ser Thr Gln Ser Pro Ser Val Phe Pro Leu Thr Arg Cys Cys Lys Asn
   1               5                   10                  15
   Ile Pro Ser Asn Ala Thr Ser Val Thr Leu Gly Cys Leu Ala Thr Gly
               20                  25                  30
   Tyr Phe Pro Glu Pro Val Met Val Thr Cys Asp Thr Gly Ser Leu Asn
           35                  40                  45
   Gly Thr Thr Met Thr Leu Pro Ala Thr Thr Leu Thr Leu Ser Gly His
       50                  55                  60
   Tyr Ala Thr Ile Ser Leu Leu Thr Val Ser Gly Ala Trp Ala Lys Gln
   65                  70                  75                  80
   Met Phe Thr Cys Arg Val Ala His Thr Pro Ser Ser Thr Asp Trp Val
                   85                  90                  95
   Asp Asn Lys Thr Phe Ser
               100
<210> 8
   <211> 103
   <212> PRT
   <213> Artificial Sequence
<220>
   <223> synthetic consrtruct
<400> 8
   Ser Ala Ser Ala Pro Thr Leu Phe Pro Leu Val Ser Cys Glu Asn Ser
   1               5                   10                  15
   Pro Ser Asp Thr Ser Ser Val Ala Val Gly Cys Leu Ala Gln Asp Phe
               20                  25                  30
   Leu Pro Asp Ser Ile Thr Phe Ser Trp Lys Tyr Lys Asn Asn Ser Asp
           35                  40                  45
   Ile Ser Ser Thr Arg Gly Phe Pro Ser Val Leu Arg Gly Gly Lys Tyr
       50                  55                  60
   Ala Ala Thr Ser Gln Val Leu Leu Pro Ser Lys Asp Val Met Gln Gly
   65                  70                  75                  80
   Thr Asp Glu His Val Val Cys Lys Val Gln His Pro Asn Gly Asn Lys
                   85                  90                  95
   Glu Lys Asn Val Pro Leu Pro
               100
<210> 9
   <211> 101
   <212> PRT
   <213> Artificial Sequence
<220>
   <223> synthetic construct
<400> 9
   Ser Pro Thr Ser Pro Lys Val Phe Pro Leu Ser Leu Cys Ser Thr Gln
   1               5                   10                  15
   Pro Asp Gly Asn Val Val Ile Ala Cys Leu Val Gln Gly Phe Phe Pro
               20                  25                  30
   Gln Glu Pro Leu Ser Val Thr Trp Ser Glu Ser Gly Gln Gly Val Thr
           35                  40                  45
   Ala Arg Asn Phe Pro Pro Ser Gln Asp Ala Ser Gly Asp Leu Tyr Thr
       50                  55                  60
   Thr Ser Ser Gln Leu Thr Leu Pro Ala Thr Gln Cys Leu Ala Gly Lys
   65                  70                  75                  80
   Ser Val Thr Cys His Val Lys His Tyr Thr Asn Pro Ser Gln Asp Val
                   85                  90                  95
   Thr Val Pro Cys Pro
               100
<210> 10
   <211> 101
   <212> PRT
   <213> Artificial Sequence
<220>
   <223> synthetic construct
<400> 10
   Ser Pro Thr Ser Pro Lys Val Phe Pro Leu Ser Leu Asp Ser Thr Pro
   1 5 10 15
   Gln Asp Gly Asn Val Val Val Ala Cys Leu Val Gln Gly Phe Phe Pro
   20 25 30
   Gln Glu Pro Leu Ser Val Thr Trp Ser Glu Ser Gly Gln Asn Val Thr
   35 40 45
   Ala Arg Asn Phe Pro Pro Ser Gln Asp Ala Ser Gly Asp Leu Tyr Thr
   50 55 60
   Thr Ser Ser Gln Leu Thr Leu Pro Ala Thr Gln Cys Pro Asp Gly Lys
   65 70 75 80
   Ser Val Thr Cys His Val Lys His Tyr Thr Asn Pro Ser Gln Asp Val
   85 90 95
   Thr Val Pro Cys Pro
   100
<210> 11
   <211> 100
   <212> PRT
   <213> Artificial Sequence
<220>
   <223> synthetic construct
<400> 11
   Pro Thr Lys Ala Pro Asp Val Phe Pro Ile Ile Ser Gly Cys Arg His
   1 5 10 15
   Pro Lys Asp Asn Ser Pro Val Val Leu Ala Cys Leu Ile Thr Gly Tyr
   20 25 30
   His Pro Thr Ser Val Thr Val Thr Trp Tyr Met Gly Thr Gln Ser Gln
   35 40 45
   Pro Gln Arg Thr Phe Pro Glu Ile Gln Arg Arg Asp Ser Tyr Tyr Met
   50 55 60
   Thr Ser Ser Gln Leu Ser Thr Pro Leu Gln Gln Trp Arg Gln Gly Glu
   65 70 75 80
   Tyr Lys Cys Val Val Gln His Thr Ala Ser Lys Ser Lys Lys Glu Ile
   85 90 95
   Phe Arg Trp Pro
   100

## Description

### 1. FIELD OF THE INVENTION

The invention relates to antibodies comprising cysteine engineered CH1 domains which result in free thiol groups for conjugation reactions. Also provided are methods of design, modification, production, and use of such antibodies.

### 2. BACKGROUND OF THE INVENTION

### 2.1 CANCER AND CANCER THERAPIES

More than 1.2 million Americans develop cancer each year. Cancer is the second leading case of death in the United States and if current trends continue, cancer is expected to be the leading cause of the death by the year 2010. Lung and prostate cancer are the top cancer killers for men in the United States. Lung and breast cancer are the top cancer killers for women in the United States. One in two men in the United States will be diagnosed with cancer at some time during his lifetime. One in three women in the United States will be diagnosed with cancer at some time during her lifetime. Current treatment options, such as surgery, chemotherapy and radiation treatment, are often either ineffective or present serious side effects.

One barrier to the development of anti-metastasis agents has been the assay systems that are used to design and evaluate these drugs. Most conventional cancer therapies target rapidly growing cells. However, cancer cells do not necessarily grow more rapidly but instead survive and grow under conditions that are non-permissive to normal cells (Lawrence and Steeg, 1996, World J. Urol. 14:124-130). These fundamental differences between the behaviors of normal and malignant cells provide opportunities for therapeutic targeting. The paradigm that micrometastatic tumors have already disseminated throughout the body emphasizes the need to evaluate potential chemotherapeutic drugs in the context of a foreign and three-dimensional microenvironment. Many standard cancer drug assays measure tumor cell growth or survival under typical cell culture conditions (i.e., monolayer growth). However, cell behavior in two-dimensional assays often does not reliably predict tumor cell behavior in vivo.

Currently, cancer therapy may involve surgery, chemotherapy, hormonal therapy and/or radiation treatment to eradicate neoplastic cells in a patient (see, for example, Stockdale, 1998, "Principles of Cancer Patient Management", in Scientific American: Medicine, vol. 3, Rubenstein and Federman, eds., Chapter 12, Section IV). All of these approaches pose significant drawbacks for the patient. Surgery, for example, may be contraindicated due to the health of the patient or may be unacceptable to the patient. Additionally, surgery may not completely remove the neoplastic tissue. Radiation therapy is only effective when the neoplastic tissue exhibits a higher sensitivity to radiation than normal tissue, and radiation therapy can also often elicit serious side effects. Hormonal therapy is rarely given as a single agent and although can be effective, is often used to prevent or delay recurrence of cancer after other treatments have removed the majority of the cancer cells.

With respect to chemotherapy, there are a variety of chemotherapeutic agents available for treatment of cancer. A significant majority of cancer chemotherapeutics act by inhibiting DNA synthesis (see, for example, Gilman et al., Goodman and Gilman's: The Pharmacological Basis of Therapeutics, Eighth Ed. (Pergamon Press, New York, 1990)). As such, chemotherapy agents are inherently nonspecific. In addition almost all chemotherapeutic agents are toxic, and chemotherapy causes significant, and often dangerous, side effects, including severe nausea, bone marrow depression, immunosuppression, etc. (see, for example, Stockdale, 1998, "Principles Of Cancer Patient Management" in Scientific American Medicine, vol. 3, Rubenstein and Federman, eds., ch. 12, sect. 10). Furthermore, even with administration of combinations of chemotherapeutic agents, many tumor cells are resistant or develop resistance to the chemotherapeutic agents.

Cancer therapy can now also involve biological therapy or immunotherapy. Biological therapies/immunotherapies are limited in number and although more specific then chemotherapeutic agents many still target both health and cancerous cells. In addition, such therapies may produce side effects such as rashes or swellings, flu-like symptoms, including fever, chills and fatigue, digestive tract problems or allergic reactions.

### 2.2 ANTIBODIES FOR THE TREATMENT OF CANCER

Antibodies are immunological proteins that bind a specific antigen. In most mammals, including humans and mice, antibodies are constructed from paired heavy and light polypeptide chains. Each chain is made up of two distinct regions, referred to as the variable (Fv) and constant (Fc) regions. The light and heavy chain Fv regions contain the antigen binding determinants of the molecule and are responsible for binding the target antigen. The Fc regions define the class (or isotype) of antibody (IgG for example) and are responsible for binding a number of natural proteins to elicit important biochemical events.

The Fc region of an antibody interacts with a number of ligands including Fc receptors and other ligands, imparting an array of important functional capabilities referred to as effector functions. An important family of Fc receptors for the IgG class are the Fc gamma receptors (FcγRs). These receptors mediate communication between antibodies and the cellular arm of the immune system (Raghavan et al., 1996, Annu Rev Cell Dev Biol 12:181-220; Ravetch et al., 2001, Annu Rev Immunol 19:275-290). In humans this protein family includes FcγRI (CID64), including isoforms FcγRIA, FcγRIB, and FcvRIC; FcγRII (CD32), including isoforms FcγRIIA, FcγRIIB, and FcγRIIC; and FcγRIII (CD 16), including isoforms FcγRIIIA and FcγRIIB (Jefferis et al., 2002, Immunol Lett 82:57-65). These receptors typically have an extracellular domain that mediates binding to Fc, a membrane spanning region, and an intracellular domain that may mediate some signaling event within the cell. These different FcγR subtypes are expressed on different cell types (reviewed in Ravetch et al., 1991, Annu Rev Immunol 9:457-492). For example, in humans, FcγRIIIB is found only on neutrophils, whereas FcγRIIIA is found on macrophages, monocytes, natural killer (NK) cells, and a subpopulation of T-cells.

Formation of the Fc/FcγR complex recruits effector cells to sites of bound antigen, typically resulting in signaling events within the cells and important subsequent immune responses such as release of inflammation mediators, B cell activation, endocytosis, phagocytosis, and cytotoxic attack. The ability to mediate cytotoxic and phagocytic effector functions is a potential mechanism by which antibodies destroy targeted cells. The cell-mediated reaction wherein nonspecific cytotoxic cells that express FcγRs recognize bound antibody on a target cell and subsequently cause lysis of the target cell is referred to as antibody dependent cell-mediated cytotoxicity (ADCC) (Raghavan et al., 1996, Annu Rev Cell Dev Biol 12:181-220; Ghetie et al., 2000, Annu Rev Immunol 18:739-766; Ravetch et al., 2001, Annu Rev Immunol 19:275-290). Notably, the primary cells for mediating ADCC, NK cells, express only FcγRIIIA, whereas monocytes express FcγRI, FcγRII and FcγRIII (Ravetch et al., 1991, supra).

Another important Fc ligand is the complement protein C1q. Fc binding to C1q mediates a process called complement dependent cytotoxicity (CDC) (reviewed in Ward et al., 1995, Ther Immunol 2:77-94). C1q is capable of binding six antibodies, although binding to two IgGs is sufficient to activate the complement cascade. C1q forms a complex with the C1r and C1s serine proteases to form the C1 complex of the complement pathway.

Several key features of antibodies including but not limited to, specificity for target, ability to mediate immune effector mechanisms, and long half-life in serum, make antibodies and related immunoglobulin molecules powerful therapeutics. Numerous monoclonal antibodies are currently in development or are being used therapeutically for the treatment of a variety of conditions including cancer. Examples of these include Vitaxin® (MedImmune), a humanized Integrin αvβ3 antibody *(e.g.,* PCT publication WO 2003/075957), Herceptin® (Genentech), a humanized anti-Her2/neu antibody approved to treat breast cancer *(e.g.,* U.S. 5,677,171), CNTO 95 (Centocor), a human Integrin αv antibody (PCT publication WO 02/12501), Rituxan® (IDEC/Genentech/Roche), a chimeric anti-CD20 antibody approved to treat Non-Hodgkin's lymphoma (*e.g.*, U.S. 5,736,137) and Erbitux® (ImClone), a chimeric anti-EGFR antibody (*e.g.*, U.S. 4,943,533).

There are a number of possible mechanisms by which antibodies destroy tumor cells, including anti-proliferation via blockage of needed growth pathways, intracellular signaling leading to apoptosis, enhanced down regulation and/or turnover of receptors, ADCC, CDC, and promotion of an adaptive immune response (Cragg et al., 1999, Curr Opin Immunol 11:541-547; Glennie et al., 2000, Immunol Today 21:403-410). However, despite widespread use, antibodies are not yet optimized for clinical use and many have suboptimal anticancer potency. Thus, there is a significant need to enhance the capacity of antibodies to destroy targeted cancer cells.

### 2.3 ANTIBODY CONJUGATES

The use of antibody conjugates , i.e. immunoconjugates, for the local delivery of cytotoxic or cytostatic agents, i.e. drugs to kill or inhibit tumor cells in the treatment of cancer (Lambert, J. (2005) Curr. Opinion in Pharmacology 5:543-549; Wu et al.(2005) Nature Biotechnology 23(9): 1137-1146; Payne, G. (2003) Cancer Cell 3:207-212; Syrigos and Epenetos (1999) Anticancer Research 19:605-614; Niculescu-Duvaz and Springer (1997) Adv. Drug Del. Rev. 26:151-172; U.S. Pat. No. 4,975,278) theoretically allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al (ed.s), pp. 475-506). Maximal efficacy with minimal toxicity is sought thereby. Efforts to design and refine antibody conjugates have focused on the selectivity of monoclonal antibodies (mAbs) as well as drug-linking and drug-releasing properties (Lambert, J. (2005) Curr. Opinion in Pharmacology 5:543-549). Both polyclonal antibodies and monoclonal antibodies have been reported as useful in these strategies (Rowland et al (1986) Cancer Immunol. Immunother., 21:183-87). Drugs used in these methods include daunomycin, doxorubicin, methotrexate, and vindesine (Rowland et al (1986)). Toxins used in antibody-toxin conjugates include bacterial toxins such as diphtheria toxin, plant toxins such as ricin, small molecule toxins such as geldanamycin (Mandler et al (2000) J. of the Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may effect their cytotoxic and cytostatic effects by mechanisms including tubulin binding, DNA binding, or topoisomerase inhibition. Some cytotoxic drugs tend to be inactive or less active when conjugated to large antibodies or protein receptor ligands.

Several antibody conjugates have been approved by the FDA or are in clinical trials. For instance, ZEVALIN® (ibritumomab tiuxetan, Biogen/Idec) is composed of a murine IgG1 kappa monoclonal antibody directed against the CD20 antigen found on the surface of normal and malignant B lymphocytes and ¹¹¹In or ⁹⁰Y radioisotope bound by a thiourea linker-chelator (Wiseman et al (2000) Eur. J. Nucl. Med. 27(7):766-77; Wiseman et al (2002) Blood 99(12):4336-42; Witzig et al (2002) J. Clin. Oncol. 20(10):2453-63; Witzig et al (2002) J. Clin. Oncol. 20(15):3262-69). Although ZEVALIN® has activity against B-cell non-Hodgkin's Lymphoma (NHL), administration results in severe and prolonged cytopenias in most patients. MYLOTARG® (gemtuzumab ozogamicin, Wyeth Pharmaceuticals), an antibody-drug conjugate composed of a human CD33 antibody linked to calicheamicin, was also approved in 2000 for the treatment of acute myeloid leukemia by injection (Drugs of the Future (2000) 25(7):686; U.S. Pat. Nos. 4,970,198; 5,079,233; 5,585,089; 5,606,040; 5,693,762; 5,739,116; 5,767,285; 5,773,001). Cantuzumab mertansine (Immunogen, Inc.), an antibody-drug conjugate composed of the human C242 antibody linked via the disulfide linker SPP to the maytansinoid drug moiety, DM1 (Xie et al (2004) J. of Pharm. and Exp. Ther. 308(3):1073-1082), is advancing in clinical trials for the treatment of cancers that express CanAg, such as colon, pancreatic, gastric, and others. MLN-2704 (Millennium Pharm., BZL Biologics, Immunogen Inc.), an antibody-drug conjugate composed of the anti-prostate specific membrane antigen (PSMA) monoclonal antibody linked to the maytansinoid drug moiety, DM1, is under development for the potential treatment of prostate tumors.

The auristatin peptides, auristatin E (AE) and monomethylauristatin (MMAE), synthetic analogs of dolastatin (WO 02/088172), have been conjugated to: (i) chimeric monoclonal antibodies cBR96 (specific to Lewis Y on carcinomas); (ii) cAC10 which is specific to CD30 on hematological malignancies (Klussman, et al (2004), Bioconjugate Chemistry 15(4):765-773; Doronina et al (2003) Nature Biotechnology 21(7):778-784; Francisco et al (2003) Blood 102(4):1458-1465; US 2004/0018194; (iii) anti-CD20 antibodies such as RITUXAN®(WO 04/032828) for the treatment of CD20-expressing cancers and immune disorders; (iv) anti-EphB2R antibodies 2H9 and anti-IL-8 for treatment of colorectal cancer (Mao et al (2004) Cancer Research 64(3):781-788); (v) E-selectin antibody (Bhaskar et al (2003) Cancer Res. 63:6387-6394); and (vi) other anti-CD30 antibodies (WO 03/043583). Variants of auristatin E are disclosed in U.S. Pat. No. 5,767,237 and U.S. Pat. No. 6,124,431. Monomethyl auristatin E conjugated to monoclonal antibodies are disclosed in Senter et al, Proceedings of the American Association for Cancer Research, Volume 45, Abstract Number 623, presented Mar. 28, 2004. Auristatin analogs MMAE and MMAF have been conjugated to various antibodies (WO 2005/081711).

Conventional means of attaching, i.e. linking through covalent bonds, a drug moiety to an antibody generally leads to a heterogeneous mixture of molecules where the drug moieties are attached at a number of sites on the antibody. For example, cytotoxic drugs have typically been conjugated to antibodies through the often-numerous lysine or cysteine residues of an antibody, generating a heterogeneous antibody-drug conjugate mixture. Depending on reaction conditions, the heterogeneous mixture typically contains a distribution of antibodies with from 0 to about 8, or more, attached drug moieties. In addition, within each subgroup of conjugates with a particular integer ratio of drug moieties to antibody, is a potentially heterogeneous mixture where the drug moiety is attached at various sites on the antibody. Analytical and preparative methods are inadequate to separate and characterize the antibody-drug conjugate species molecules within the heterogeneous mixture resulting from a conjugation reaction. Antibodies are large, complex and structurally diverse biomolecules, often with many reactive functional groups. Their reactivities with linker reagents and drug-linker intermediates are dependent on factors such as pH, concentration, salt concentration, and co-solvents. Furthermore, the multistep conjugation process may be nonreproducible due to difficulties in controlling the reaction conditions and characterizing reactants and intermediates.

Cysteine thiols are reactive at neutral pH, unlike most amines which are protonated and less nucleophilic near pH 7. Since free thiol (R-SH, sulfhydryl) groups are relatively reactive, proteins with cysteine residues often exist in their oxidized form as disulfide-linked oligomers or have internally bridged disulfide groups. Extracellular proteins generally do not have free thiols (Garman, 1997, Non-Radioactive Labelling: A Practical Approach, Academic Press, London, at page 55). The amount of free thiol in a protein may be estimated by the standard Ellman's assay. IgM is an example of a disulfide-linked pentamer, while IgG is an example of a protein with internal disulfide bridges bonding the subunits together. In proteins such as this, reduction of the disulfide bonds with a reagent such as dithiothreitol (DTT) or selenol (Singh et al (2002) Anal. Biochem. 304:147-156) is required to generate the reactive free thiol. This approach may result in loss of antibody tertiary structure and antigen binding specificity.

Antibody cysteine thiol groups are generally more reactive, i.e. more nucleophilic, towards electrophilic conjugation reagents than antibody amine or hydroxyl groups. Cysteine residues have been introduced into proteins by genetic engineering techniques to form covalent attachments to ligands or to form new intramolecular disulfide bonds (Better et al (1994) J. Biol. Chem. 13:9644-9650; Bernhard et al (1994) Bioconjugate Chem. 5:126-132; Greenwood et al (1994) Therapeutic Immunology 1:247-255; Tu et al (1999) Proc. Natl. Acad. Sci USA 96:4862-4867; Kanno et al (2000) J. of Biotechnology, 76:207-214; Chmura et al (2001) Proc. Nat. Acad. Sci. USA 98(15):8480-8484; U.S. Pat. No. 6,248,564). However, designing in cysteine thiol groups by the mutation of various amino acid residues of a protein to cysteine amino acids is potentially problematic, particularly in the case of unpaired (free Cys) residues or those which are relatively accessible for reaction or oxidation. In concentrated solutions of the protein, whether in the periplasm of *E. coli,* culture supernatants, or partially or completely purified protein, unpaired Cys residues on the surface of the protein can pair and oxidize to form intermolecular disulfides, and hence protein dimers or multimers. Disulfide dimer formation renders the new Cys unreactive for conjugation to a drug, ligand, or other label. Furthermore, if the protein oxidatively forms an intramolecular disulfide bond between the newly engineered Cys and an existing Cys residue, both Cys groups are unavailable for active site participation and interactions. Also, the protein may be rendered inactive or nonspecific, by misfolding or loss of tertiary structure (Zhang et al (2002) Anal. Biochem. 311:1-9).

Previous attempts to engineer conjugation sites into antibodies have been attempted. US Patent No. 5,219,916 describes the modification of "surface pocket" residues such as Ser 156 or Thr 173 (according to Kabat et al., Sequences of Immunological Interest, 4th ed., US Dept. of Health and Human Services, 1987). In the related study, the researchers determined that only residues on "surface pockets" were capable of supporting the substitution of cysteine in an effort to engineer a conjugation site (Lyons et al. (1990) Protein Eng. 3:8 pg 703-708).

WO 2006/034488 discloses antibodies engineered by replaying one replacing one or more amino acids with cysteine to form cysteine engineered antibody fragments (ThioFab) having thiol reactivity. The amino acids are substituted at positions, for example; S119C, A121C, S122C, A144C, A175C. The cysteine engineered antibodies may be conjugated with chemotherapeutic drugs, toxins, affinity ligands and detection labels.

Thus, there is a need to develop stable cysteine engineered antibodies which provide free thiol groups capable of conjugation to various agents.

Citation or discussion of a reference herein shall not be construed as an admission that such is prior art to the present invention.

### 3. SUMMARY OF THE INVENTION

The present invention provides antibodies comprising modified CH1 domains such that they contain free cysteine residues capable of conjugation to various agents. Cysteine engineered antibodies of the invention comprise one or more amino acids from the 131-139 region of the heavy chain of an antibody substituted with one or more non-naturally occurring cysteine amino acids whereby the substituted cysteine amino acid provides a free thiol group capable for conjugation. In one embodiment, the cysteine engineered antibodies of the invention comprise 1, 2, 3, 4, 5, 6, 7, 8, or more substituted cysteine amino acids. In another embodiment, the 131-139 region of the CH1 domain of the antibody comprises substitutions of cysteine for serine or threonine residues.

The cysteine engineered antibodies of the invention may comprise a non-naturally occurring disulfide bond connecting the modified CH1 domain with another antibody chain. In one embodiment, cysteine engineered antibodies of the invention comprise one or more free thiol groups that are formed as a result of the formation of the non-naturally occurring disulfide bond connecting the modified CH1 domain with another antibody chain.

Another aspect of the invention provides nucleic acids, vectors and host cells for the generation of cysteine engineered antibodies.

Another aspect of the invention provides antibody conjugates and methods of making such conjugates comprising the cysteine engineered antibodies of the invention coupled to a drug where the drug may be a cytotoxic agent, chemotherapeutic agent, peptide, peptidomimetic, protein scaffold, enzyme, toxin, radionuclide, DNA, RNA, siRNA, microRNA, peptidonucleic acid, fluorescent tag, or biotin.

Another aspect of the invention provides antibodies that are capable of internalizing when bound to cell surface receptors. In such aspects, antibodies of the invention are useful for cytoplasmic delivery of cargo molecules and/or agents.

Another aspect of the invention provides methods of treating, detecting, and diagnosing cancer, autoimmune, inflammatory, or infectious diseases with the antibody conjugates of the invention.

### 4. BRIEF DESCRIPTION OF THE FIGURES

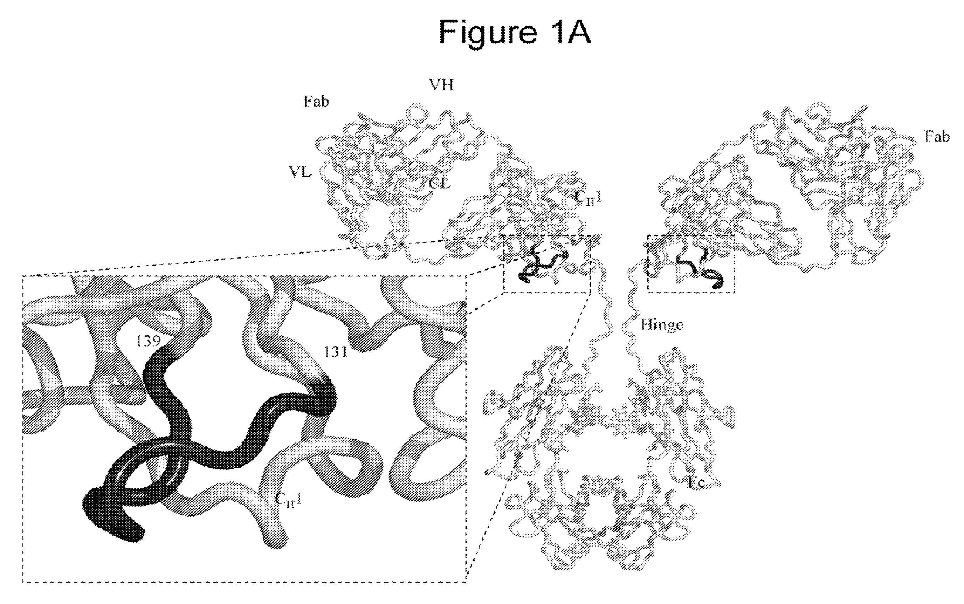

FIGURE 1A is a schematic representation of the IgG structure. The region from residue 131 to residue 139 of the CH1 domain, which was mutagenized, is shown in dark black. The insert shows a zoom view of the IgG region from residue 131 to residue 139 of the CH1 domain.

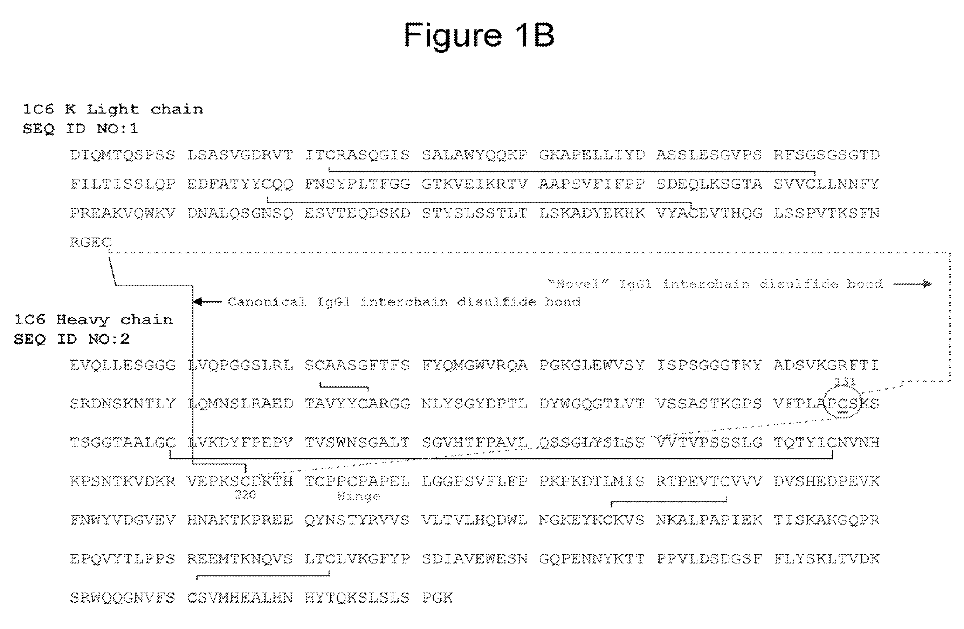

FIGURE 1B is a representation of the cysteine engineering strategy for position 131 employed to create a free thiol in an antibody capable of conjugation. Specifically, the Kappa light chain and heavy chain of an EphB4 specific antibody, 1C6 are presented with the cysteine residues represented in bold typeface. In addition, the relative disulfide bonds naturally occurring in an IgG1 antibody format are presented as solid lines. The specific engineering of position 131 from serine to cysteine (represented in the Figure as bold and underlined) represents a potential interchain disulfide bond that would form, replacing the canonical interchain disulfide bond connecting the light chain carboxy terminal cysteine to cysteine 220 of the heavy chain. The potential disulfide bonds that may be formed with the inclusion of 131Cys are presented (dashed lines). The disulfide bonds as presented herein were confirmed by experimental results

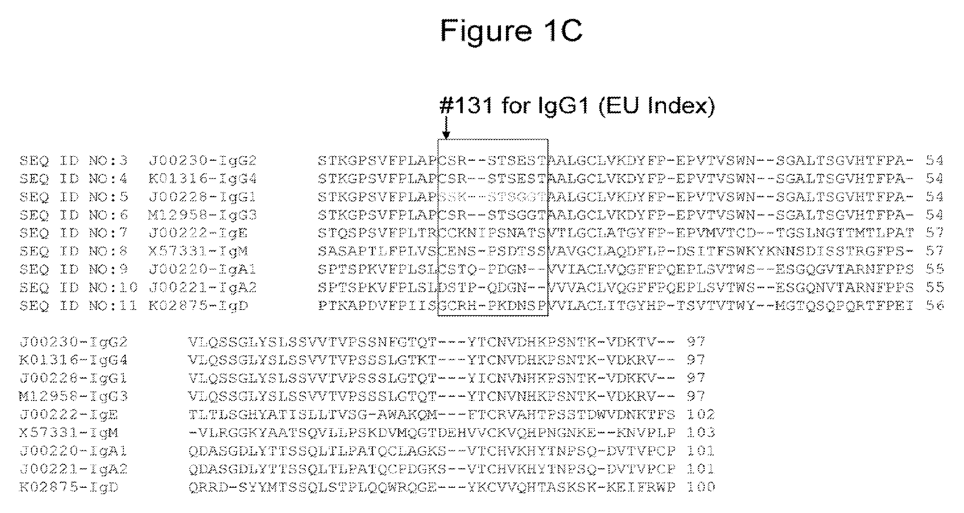

FIGURE 1C is an alignment of CH1 domains from various antibody formats outlining the equivalent region containing candidate serine or threonine residues for cysteine engineering. The boxed region represents the equivalent region of the CH1 domain of IgG1 in the other antibody formats.

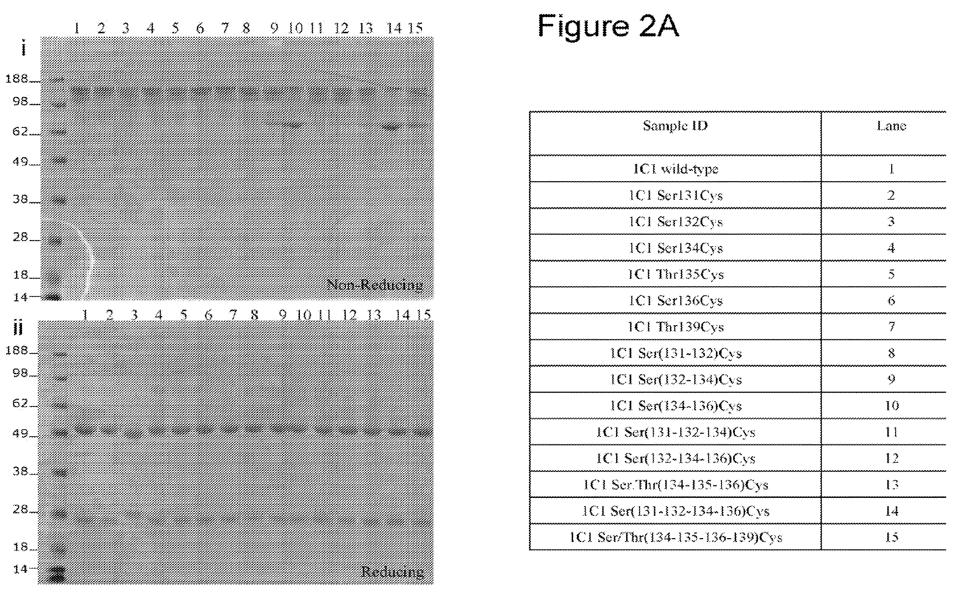

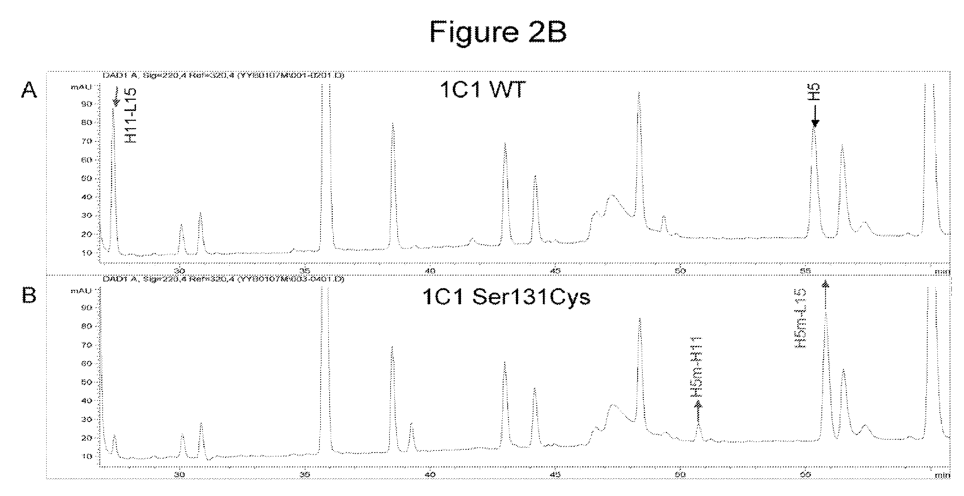

FIGURE 2A is a Coomassie stained PAGE gel documenting the expression and purification of antibodies run under non-reducing (inset i) and reducing (inset ii) conditions. Antibodies presented in this panel include 1C1 (lane 1) and various cysteine engineered antibodies derived from 1C1 (lanes 2-15). Antibodies were loaded at a concentration of 2 µg/well.
FIGURE 2B is a comparison of non-reducing peptide mapping of IgG1 wt and Ser131 to Cys mutant. 1C1 is an EphA2 specific antibody of the IgG1 subclass. Inset A is the 1C1 WT antibody which showed the regular disulfide bond linkage between heavy chain hinge region and light chain C-terminus (H11-L15) and the peptide containing Ser131 (H5). Inset B is the 1C1 Ser131Cys mutant which showed the decrease of the regular disulfide bond between Light and heavy chain (H11-L15) and 1C1 wt peptide H5, and the appearance of new formed disulfide bond linkages between mutated cysteine to light chain C-terminus (H5m-L15) and to hinge-region (H5m-H11). Only trace amounts of free cysteine was observed for mutated Cys131.

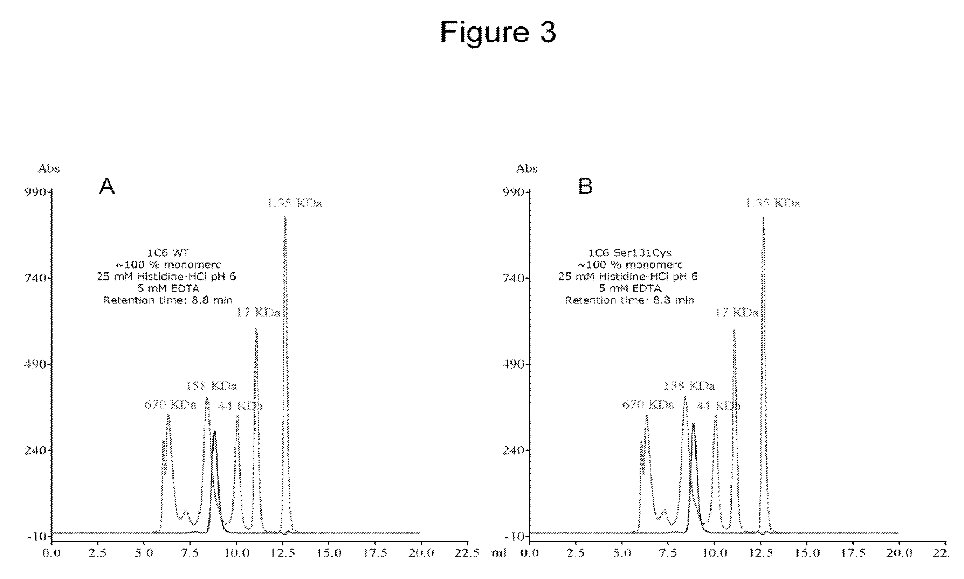

FIGURE 3 represents the results from a Size Exclusion Chromatography (SEC) analysis performed on purified 1C6 WT (A) and 1C6 Ser131Cys (B) antibodies. The antibodies were expressed and purified and subjected to SEC chromatography. The dotted tracing represents a set of defined molecular weight markers used to establish the apparent molecular weight of the antibodies. As demonstrated in (A) the 1C6 WT antibody elutes off the SEC column with a molecular weight corresponding to a monomeric antibody. The SEC analysis of the cysteine engineered 1C6 antibody (B) demonstrates that this antibody also exists in monomeric form, similar to wild type antibody.

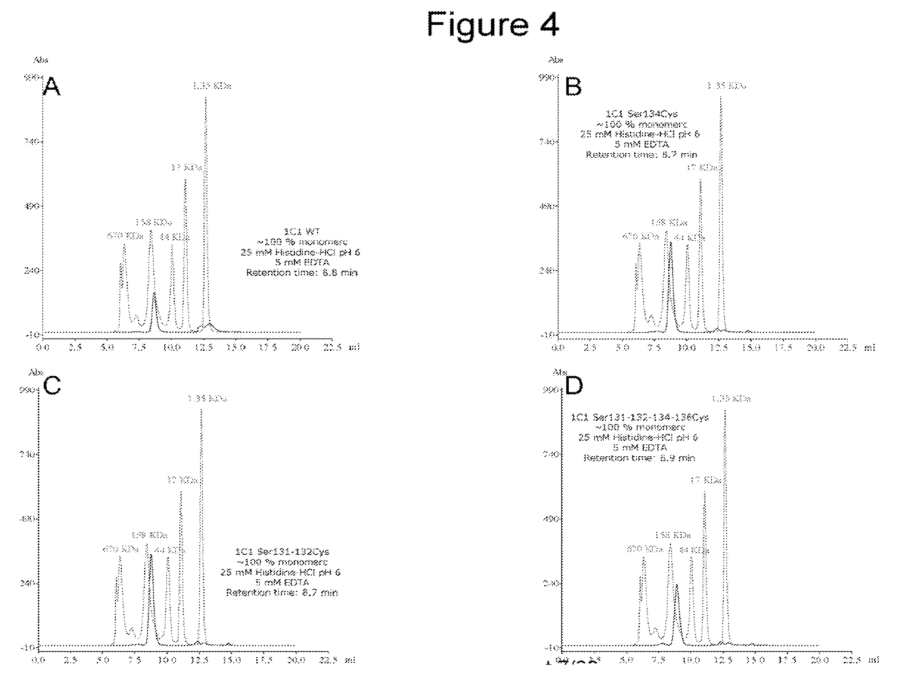

FIGURE 4 represents the results from a Size Exclusion Chromatography (SEC) analysis performed on purified antibodies namely 1C1 WT (A), 1C1 Ser134Cys (B), 1C1 Ser132Cys (C), and 1C1 Ser131-132-134-136Cys (D). The dotted tracing represents a set of defined molecular weight markers used to establish the apparent molecular weight of the antibodies. As demonstrated in (A) the 1C1 WT antibody elutes off the SEC column with a molecular weight corresponding to a monomeric antibody. The SEC analysis of the cysteine engineered antibodies 1C1 Ser134Cys (B), 1C1 Ser132Cys (C), and 1C1 Ser131-132-134-136Cys (D) demonstrated that these antibodies also exists in monomeric form, similar to wild type antibody.

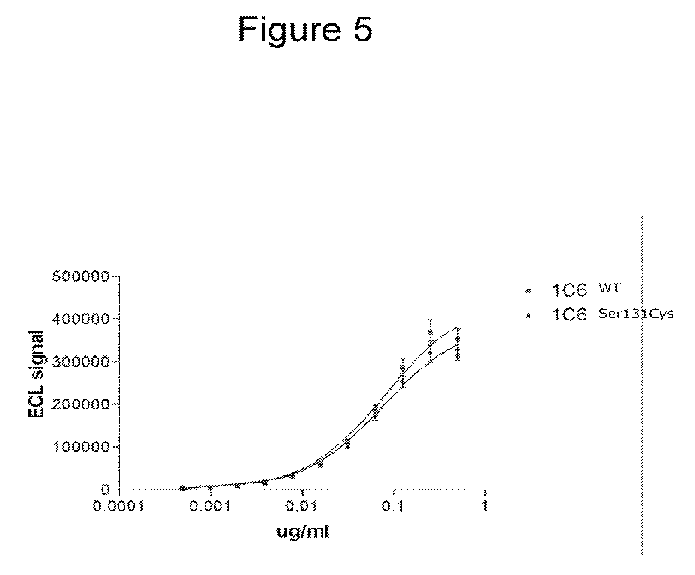

FIGURE 5 represents the results from an ELISA based antigen binding assay performed on purified antibodies namely 1C6 WT and 1C6 Ser131Cys. These antibodies specifically recognize the EphB4 receptor. As demonstrated in the Figure, the binding affinity profile measured in an ELISA format of the WT antibody and the cysteine engineered Ser131Cys antibody are very similar.

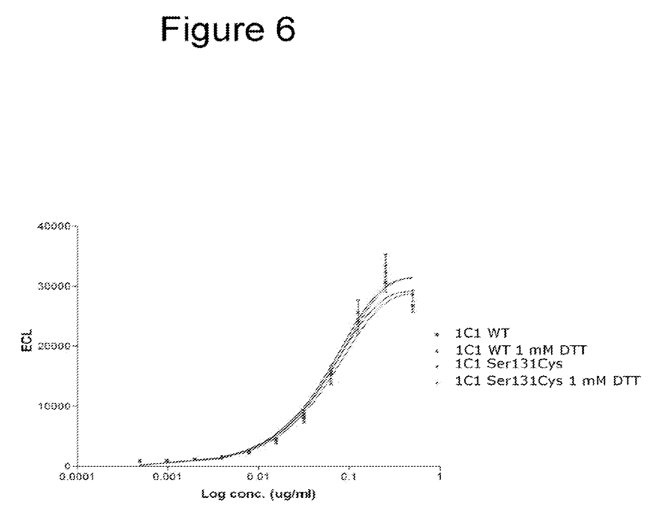

FIGURE 6 represents the results from an ELISA based antigen binding assay performed on purified antibodies namely 1C1 WT and 1C1 Ser131Cys. These antibodies specifically recognize the EphA2 receptor. As demonstrated in the Figure, the binding affinity profile measured in an ELISA format of the WT antibody and the cysteine engineered Ser131Cys antibody are very similar. In addition, the inclusion of 1 mM DTT did not have a measurable effect on the binding profile.

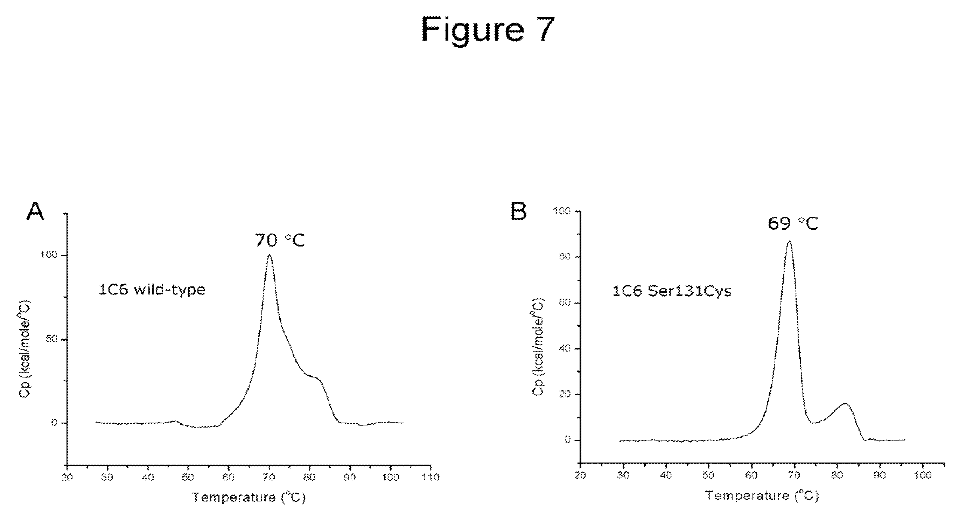

FIGURE 7 represents Differential Scanning Calorimetry (DSC) thermograms of the 1C6 WT antibody (A) and 1C6 Ser131Cys antibody (B). Both antibodies exhibit very similar melting temperatures (Tm) of 70°C and 69°C respectively.

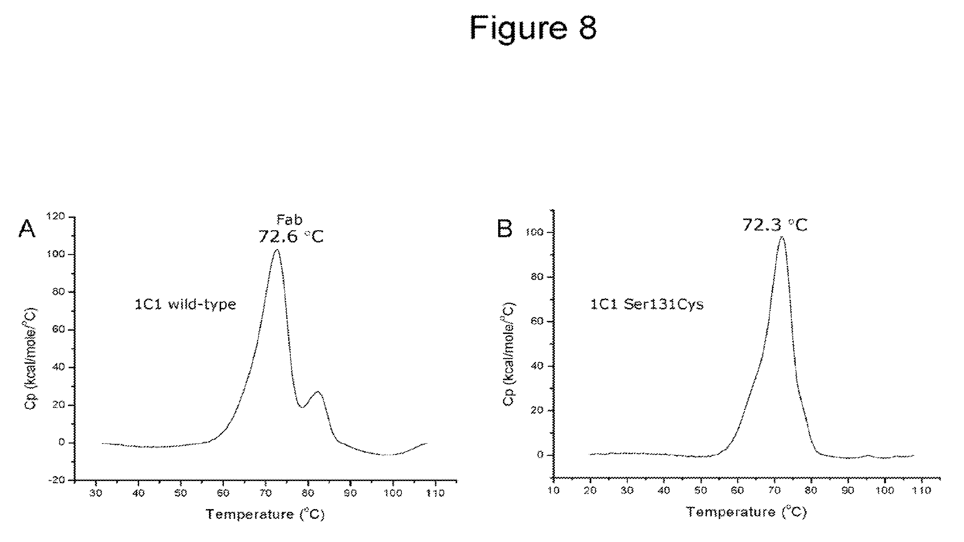

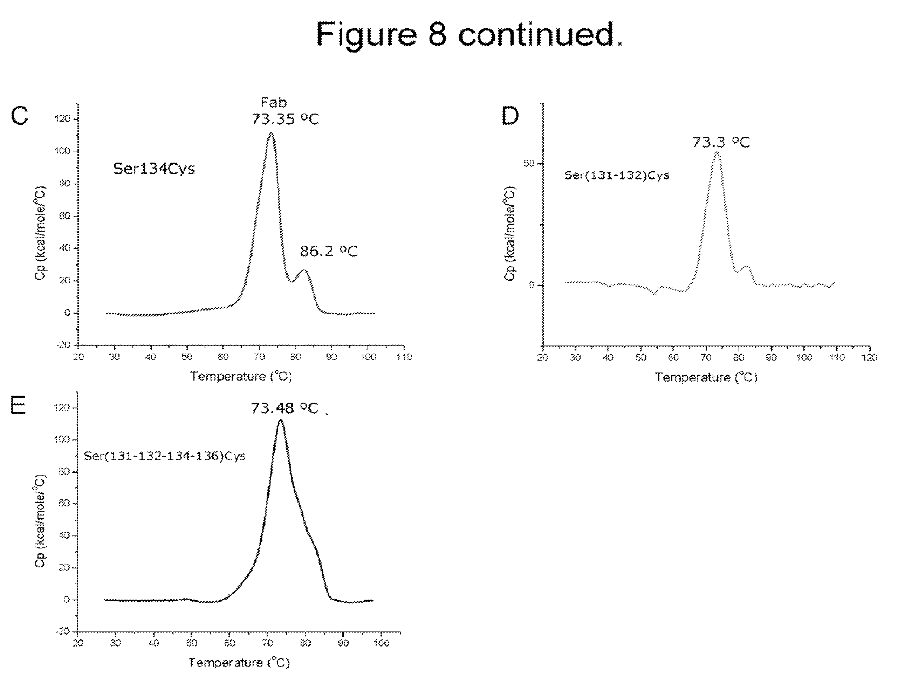

FIGURE 8 represents Differential Scanning Calorimetry (DSC) thermograms of the 1C1 WT (A), 1C1 Ser131Cys (B), 1C1 Ser134Cys (C), 1C1 Ser(131-132)Cys (D), and 1C1 Ser(131-132-134-136)Cys antibodies. All of the antibodies exhibit a very similar melting temperature (Tm).

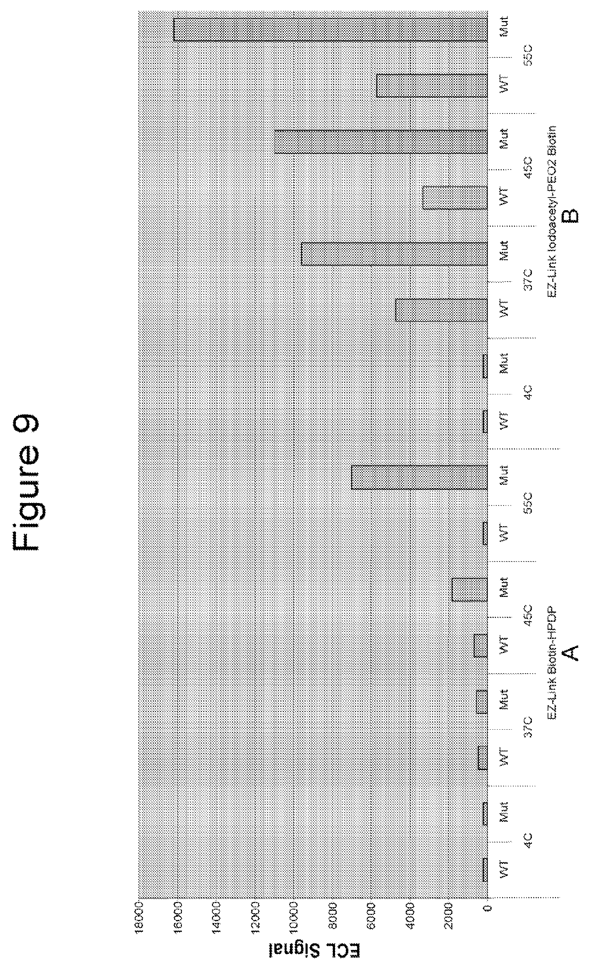

FIGURE 9 represents the results from a biotin conjugation study of 1C6 (WT) antibody and the 1C6 Ser131Cys (Mut) antibody under various conditions. In panel A, the 1C6 and 1C6 Ser131Cys antibodies were subjected to a conjugation reaction with EZ-Link Biotin-HPDP (Pierce) at various temperatures (4°C, 37°C, 45°C, and 55°C). The resultant biotin conjugation efficiency was measured and plotted. The 1C6 Ser131Cys antibody exhibits a higher efficiency of site-specific biotin conjugation than the 1C6 antibody. In panel B, the 1C6 and 1C6 Ser131Cys antibodies were subjected to a conjugation reaction with EZ-Link iodoacetyl-PEO2 Biotin at various temperatures (4°C, 37°C, 45°C, and 55°C). The resultant site-specific biotin conjugation efficiency was measured and plotted. The 1C6 Ser131Cys antibody exhibits a higher efficiency of site-specific biotin conjugation than the 1C6 antibody.

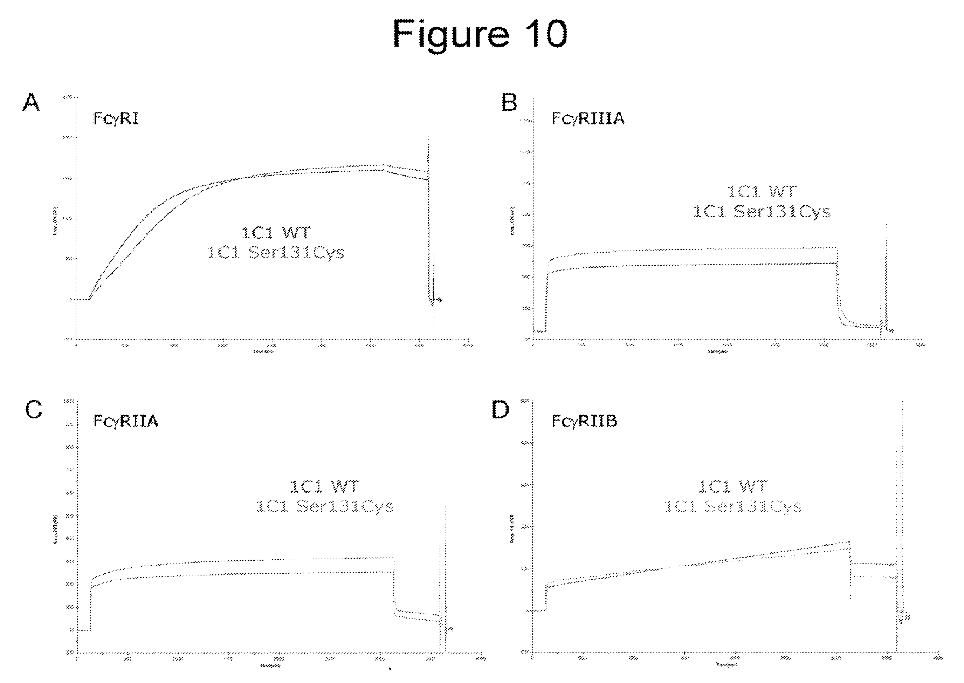

FIGURE 10 represents the results from a BIACORE® assay measuring the relative affinities for the 1C1 WT and 1C1 Ser131Cys antibodies for various Fcγ receptors. The various Fcγ receptors studied were FcγRI (A), FcγRIIIA (B), FcγRIIA (C), FcγRIIB (D). The 1C1 WT and 1C1 Ser131Cys antibodies exhibit very similar binding affinities for various Fcγ receptors.

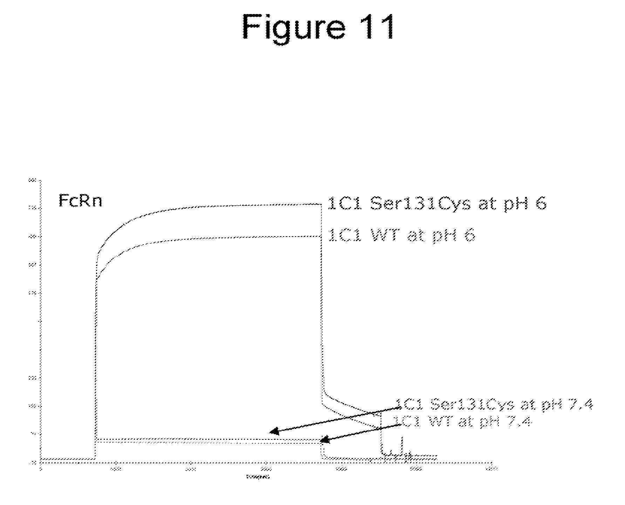

FIGURE 11 represents the results from a BIACORE® assay measuring the relative affinities for the 1C1 WT and 1C1 Ser131Cys antibodies for the FcRn receptor at pH 6.0 and pH 7.4. The 1C1 Ser131Cys antibody binds the FcRn receptor with a similar binding profile to the 1C1 WT antibody at both pH 6.0 and pH 7.4.

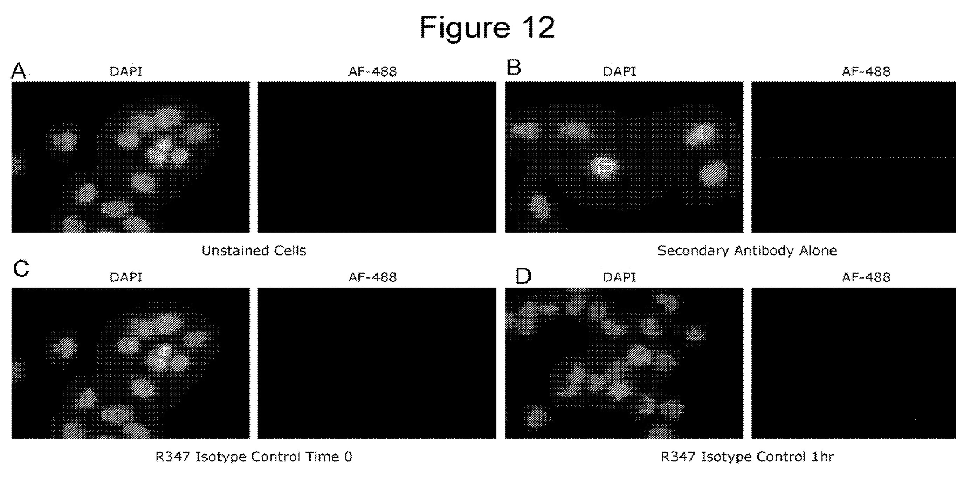

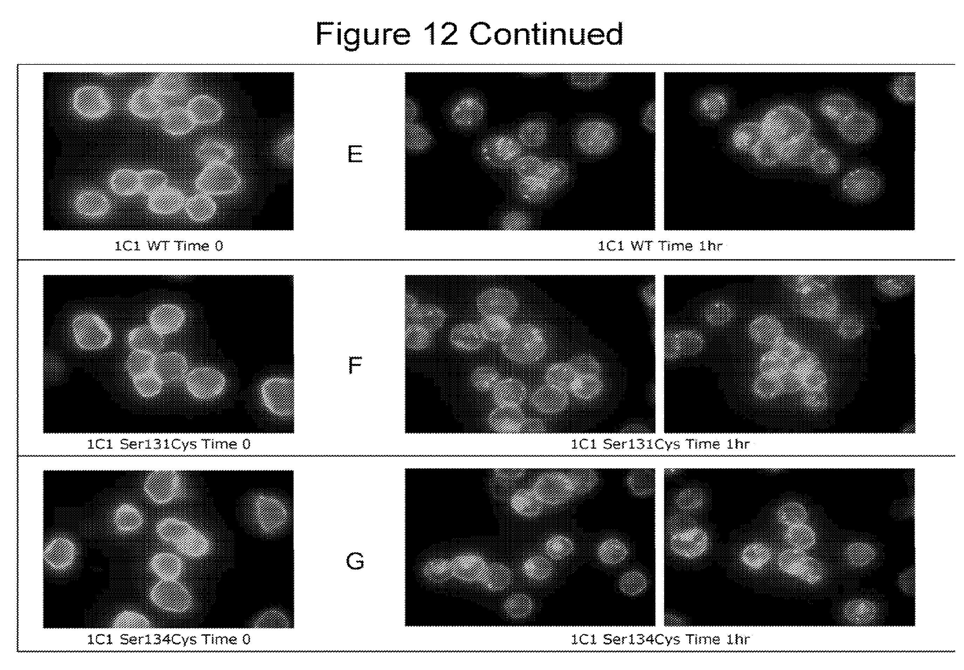

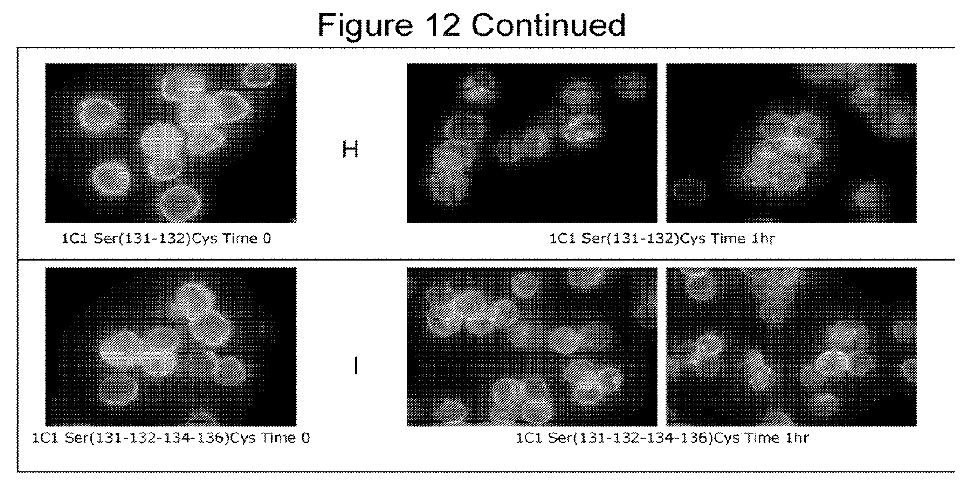

FIGURE 12 represents the results from an antibody internalization study performed on PC3 cells. A set of controls are presented in the first panel. In (A) unstained cells are counterstained with DAPI. In (B) cells stained with secondary antibody alone are counterstained with DAPI. In (C) a control primary antibody, R347 is incubated with the cells as well as counterstaining with DAPI. In (D) the cells are incubated for one hour and subsequently stained with R347. None of the controls (A-D) exhibit any antibody specific cell staining. In (E) cells are incubated with 1C1 wt antibody at time zero and for one hour. Two representative images at one hour indicate internalization of the 1C1 WT antibody. In (F) cells are incubated with 1C1 Ser131Cys antibody at time zero and for one hour. Two representative images at one hour indicate internalization of the 1C1 Ser131Cys antibody. In (G) cells are incubated with 1C1 Ser134Cys antibody at time zero and for one hour. Two representative images at one hour indicate internalization of the 1C1 Ser134Cys antibody. In (H) cells are incubated with 1C1 Ser(131-132)Cys antibody at time zero and for one hour. Two representative images at one hour indicate internalization of the 1C1 Ser(131-132)Cys antibody. In (I) cells are incubated with 1C1 Ser(131-132-134-136)Cys antibody at time zero and for one hour. Two representative images at one hour indicate internalization of the 1C1 Ser(131-132-134-136)Cys antibody. All of the cysteine engineered antibodies internalized to a similar extent as compared to the wild type antibody.

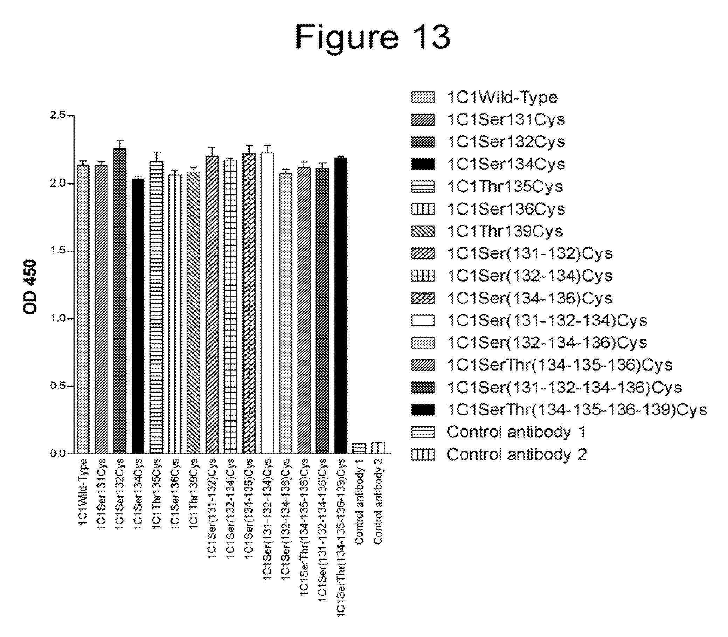

FIGURE 13 represents the results from a binding specificity experiment in which the cysteine engineered antibodies displayed an equivalent binding specificity for EphA2 compared with the wild type 1C1 prior to cysteine engineering. The use of 2 unrelated antibodies (Control antibody 1 and 2) confirms the specificity of this ELISA experiment for EphA2. Also, multiple substitutions of cysteine residues do not alter the binding specificity of the antibody for its cognate antigen. These results demonstrate that the cysteine engineering of antibodies does not alter the binding specificities as compared to the antibody prior to cysteine engineering.

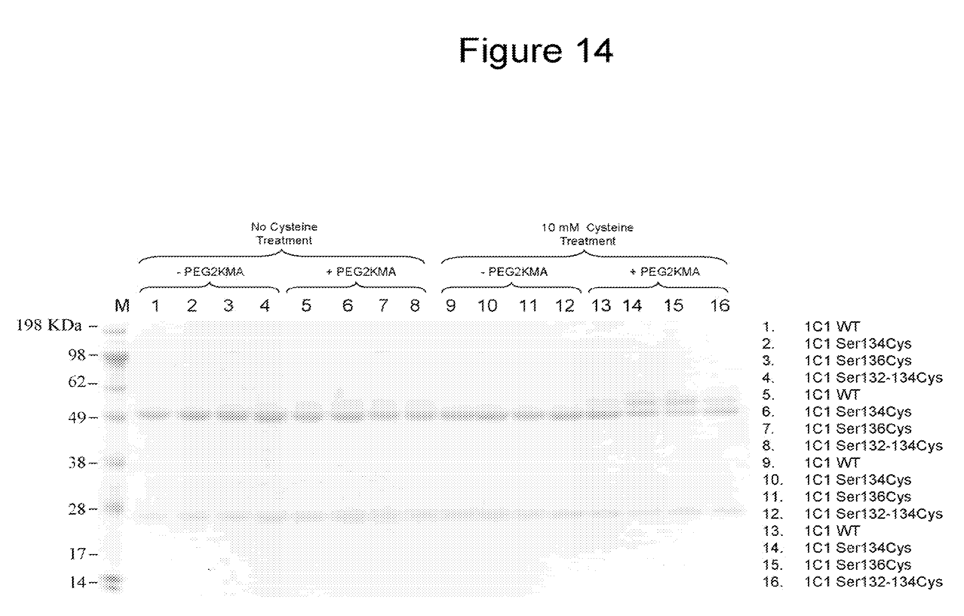

FIGURE 14 represents the results from a conjugation reaction with PRG using various cysteine engineered antibodies before (lanes 1-8) or after (lanes 9-Ï6) treatment with free cysteine. The non-cysteine treatment lanes demonstrate a lowered level of PEGylation (higher molecular weight band) as compared with a treatment of the cysteine engineered antibodies with 10 mM free Cysteine. Control wells containing antibodies prior to cysteine engineering exhibit no detectable level of pegylation in either condition (lanes 1,5, 9, and 13). The pegylation reaction was performed for 120 minutes at 37<0>C. Samples were run on a 10% NuPage MOP Gel

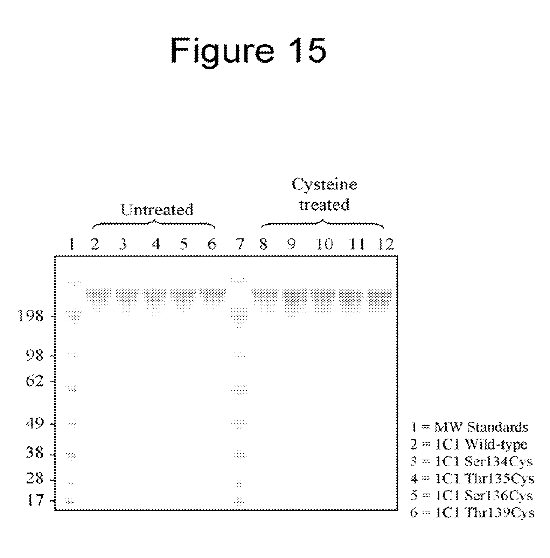

FIGURE 15 represents the results from an experiment in which cysteine engineered antibodies were subjected to an uncapping reaction. The uncapping reaction frees the engineered cysteine for conjugation without disrupting the overall antibody structure. The antibodies 1C1 (wild type) (lanes 2, 8), 1C1 Ser134Cys (lanes 3, 9), 1C1Thr135Cys (lanes 4, 10), 1C1Ser136Cys (lanes 5, 11), 1C1Thr139Cys (lanes 6, 12) were subjected to the uncapping reaction and analyzed by non-reducing PAGE. The protein profiles presented demonstrate that the uncapping reaction does not destabilize the overall antibody structure (lanes 8-12) as compared with the antibodies prior to the uncapping reaction (lanes 2-6).

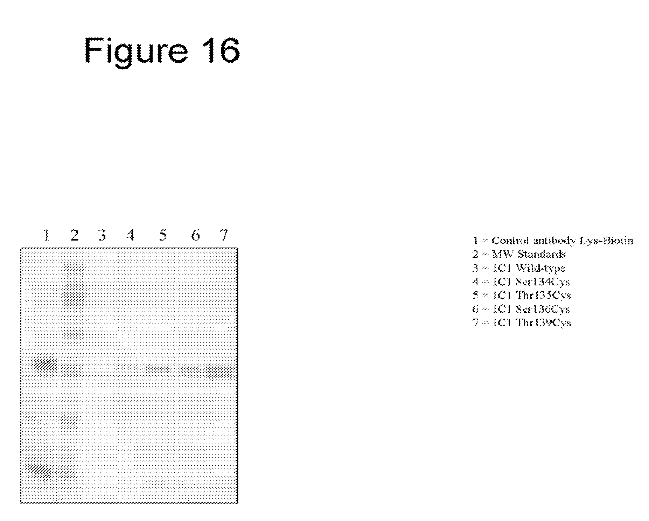

FIGURE 16 represents the results from an experiment in which various cysteine engineered antibodies were conjugated with Biotin. Briefly, the cysteine engineered antibodies and controls were subjected to an uncapping reaction and then placed in a conjugation reaction with Malemide-PEG2-biotin. The un reacted conjugant was subsequently removed. The positive control exhibits strong biotin staining by Western blot analysis (Lane 1). The control 1C1 antibody displayed no detectable conjugated biotin (Lane 3). The cysteine engineered antibodies 1C1 Ser134Cys (lane 4), 1C1 Phr135Cys (lane 5), 1C1 Ser136Cys (lane 6), and 1C1Thr139Cys (lane 7) display strong staining for conjugated biotin. Also, it is evident from the Figure that the light chain of the cysteine engineered antibodies do not exhibit any biotin conjugation. The lower band of the control antibody (Lane 1) demonstrates a high level of staining whereas the lower bands of the cysteine engineered antibodies and wt counterpart do not exhibit any significant staining on the lower (light chain) band (Lanes 3-7).

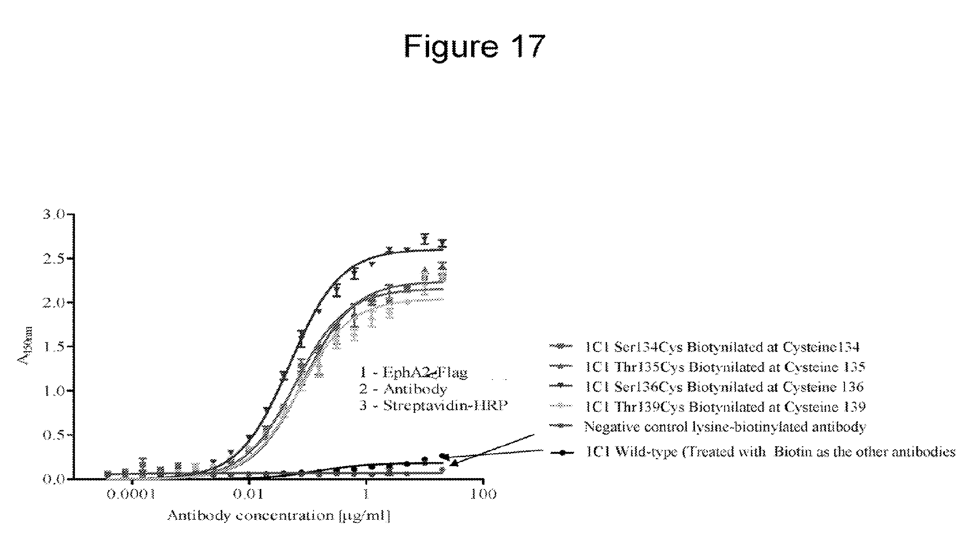

FIGURE 17 represents the results from an experiment that demonstrates that cysteine engineered antibodies retain binding specificity for their cognate antigens after conjugation to a heterologous agent. In this experiment, cysteine engineered antibodies conjugated with biotin were analyzed for retention of antigen binding specificity as compared to the parental antibodies prior to cysteine engineering. The ELISA based assay demonstrates that conjugated cysteine engineered antibodies 1C1 Ser1 34Cys, 1C1 Thr135Cys, 1C1 Ser136Cys, and 1C1 Thr139Cys exhibit a very similar binding profile to the cognate EphA2 antigen as the parental antibody prior to cysteine engineering and conjugation.

### 5. DETAILED DESCRIPTION

The invention is based on the finding that residues present on the surface of the CH1 domain of antibodies (see FIGURE 1A)are suitable for the substitution of cysteine in an effort to engineer a site capable of conjugation to various agents.

The compounds of the invention include cysteine engineered antibodies where 1, 2, 3, 4, 5, 6, 7, or more amino acids of the 131-139 region of the CH1 domain wherein the numbering system of the constant region is that of the EU index as set forth in Kabat et al. (1991, NIH Publication 91-3242, National Technical Information Service, Springfield, VA) of a parent or wild type antibody are substituted with a cysteine amino acid. It should be noted that a single substitution of a cysteine residue results in the display of two cysteine residues in the resultant antibody due to the dimeric nature of IgG molecules. The resultant cysteine engineered antibodies of the invention may display at least 1, 2, 3, 4, 5, 6, 7, 8, 9, 10, 11, 12, 13, 14, 15, 16, 17, 18 or more free thiols for the purpose of conjugation to a drug or compound.

In some embodiments, the cysteine engineered antibodies of the invention comprise a serine substitution to cysteine. In other embodiments, the cysteine engineered antibodies of the invention comprise a threonine to cysteine substitution. In some embodiments, the cysteine engineered antibodies of the invention comprise both a serine and a threonine to cysteine substitution.

In some embodiments, the cysteine engineered antibodies of the invention comprise at least one substitution at positions selected from: 131, 132, 133, 134, 135, 136, 137, 138, and 139 of the CH1 domain of an antibody, wherein the numbering system of the constant region is that of the EU index as set forth in Kabat et al. (*supra*). In other embodiments, the cysteine engineered antibodies of the invention comprise at least two substitutions selected from the positions 131, 132, 133, 134, 135, 136, 137, 138, and 139 of the CH1 domain of an antibody. In other embodiments, the cysteine engineered antibodies of the invention comprise at least three substitutions selected from the positions 131, 132, 133, 134, 135, 136, 137, 138, and 139 of the CH1 domain of an antibody. In other embodiments, the cysteine engineered antibodies of the invention comprise at least four substitutions selected from the positions 131, 132, 133, 134, 135, 136, 137, 138, and 139 of the CH1 domain of an antibody. In other embodiments, the cysteine engineered antibodies of the invention comprise at least five substitutions selected from the positions 131, 132, 133, 134, 135, 136, 137, 138, and 139 of the CH1 domain of an antibody. In other embodiments, the cysteine engineered antibodies of the invention comprise at least six substitutions selected from the positions 131, 132, 133, 134, 135, 136, 137, 138, and 139 of the CH1 domain of an antibody. In other embodiments, the cysteine engineered antibodies of the invention comprise at least seven substitutions selected from the positions 131, 132, 133, 134, 135, 136, 137, 138, and 139 of the CH1 domain of an antibody. In other embodiments, the cysteine engineered antibodies of the invention comprise at least eight substitutions selected from the positions 131, 132, 133, 134, 135, 136, 137, 138, and 139 of the CH1 domain of an antibody. In other embodiments, the cysteine engineered antibodies of the invention comprise substitutions of the positions 131, 132, 133, 134, 135, 136, 137, 138, and 139 of the CH1 domain of an antibody.

In some embodiments, the cysteine engineered antibodies of the invention do not comprise a substitution at positions 132 and/or 138. In other embodiments, cysteine engineered antibodies of the invention comprises substitutions at only threonine and/or serine amino acids naturally occurring in the 131 to 139 region of the CH1 domain of an IgG 1 molecule, or equivalents thereof.

In one embodiment, the cysteine engineered antibodies of the invention include an IgG1 having a serine and/or a threonine substituted for a cysteine at a position selected from the group consisting of: 131, 132, 133, 134, 135, 136, 137, 138, and 139. In other embodiments, the cysteine engineered antibodies of the invention are derived from an IgG1, IgG2, IgG3 or an IgG4 format. In yet other embodiments, the cysteine engineered antibodies of the invention are derived from non-IgG formats such as IgA1, IgA2 IgM, IgD, or IgE. In other embodiments, antibodies of the invention comprise cysteine engineering of residues corresponding to the 131-139 region of the CH1 region of IgG1. In another embodiment, antibodies of the invention comprise cysteine engineering of the residues outlined in the various antibody formats presented in Figure 1C. In yet other embodiments, the antibodies of the invention comprise antibody fragments including, but not limited to Fab and Fab2 molecule formats.

The 131-139 region of the CH1 domain of the IgG1 molecule is solvent exposed as illustrated in Figure 1A. As such, it is envisaged that the 131-139 loop may be expanded (in other words, inclusion of additional amino acids) to facilitate a surface for site-specifie conjugation of various agents. In some embodiments, the 131 -139 loop of the CH1 domain of the antibodies of the invention are expanded by at least 1, at least 2. at least 3, at least 4, at least 5, at least 6, at least 7, at least 8, at least 9, at least 10, at least 11. at least 12, at least 13, at least 14, or at least 15 amino acids. In some embodiments, an expansion of the 131-139 loop of the CH1 domain of antibodies of the invention occurs after the 131, 132, .133, 134, 135, 136, 137, 138, or 139 residue. In other embodiments, an expansion of the 131-139 loop of the CH1 domain of antibodies of the invention occurs after the 131, 132, 133, 134, 135, 136, 137. 138, and 139 residue.

In other embodiments, an expansion of the 131-139 loop of a CH1 domain of an IgG1 molecule may comprise any amino acid, hi other embodiments, said expansion comprises at least one non-naturally occurring cysteine amino acid. In other embodiments, said expansion comprises threonine and/or serine residues. In other embodiments, said expansion is also coupled with the substitution of naturally occurring cysteine residues for non-cystεine residues.

In another embodiment, cysteine engineered antibodies comprise the formation of at least one nors-naturally occurring disulfide bond. The non-naturally occurring disulfide bond may be intrachain or interchain bond. The non-naturally occurring disulfide bond may link two separate antibody molecules together. The formation of a non-naturally occurring disulfide bond may liberate at least one free thiol group previously linked to another cysteine residue.

The engineering of cysteine residues to display free thiol groups may lead to a mixture of antibody species, displaying a high degree of variability of positions of disulfide bonds. For example, the naturally occurring "canonical" disulfide bond (illustrated in Figure 1 B) may only be represented in some of the antibodies present in a sample, ït is understood that the engineering of other non-naturally occurring cysteines may lead to the formation of disulfide bonds other than the "canoncical" disulfide bond. In some embodiments, a disulfide bond is formed between the light chain and any non-naiurally occurring cysteine residue present in the 131 -139 region of the heavy chain. In other embodiments, a disulfide bond is formed between the light chain and any non- naturally occurring cysteine residue present m the 131, 132, 133, 134, 135, 136, 137, 138, ami/or 139 position of the heavy chain.

In an effort to limit the variability of disulfide positions present in antibodies? in a sample, cysteine engineered antibodies may comprise compensatory substitutions of naturally occurring cysteine residues to another residue, to ablate a disulfide bond, hi a specific embodiment, the cysteine engineered antibodies of the invention comprise the substitution of a naturally occurring cysteine residue, such as the cysteine occurring at position 220 of the heavy chain, for another amino acid residue to ablate a disulfide bond.

The formation of at least one non-naturally occurring disulfide bond may influence the stability of the cysteine engineered antibody of the invention in comparison to the antibody prior to modification. In some embodiments, the non-vaturally occurring disulfide bond may increase stability of the cysteine engineered antibody as compared to the same antibody prior to cysteine engineering. In other embodiments, the non-naturally occurring disulfide bond may decrease stability of the cysteine engineered antibody as compared to the same antibody prior to cysteine engineering.

Cysteine engineered antibodies of the invention retain the antigen binding capability of their wild type counterpart. In one embodiment, the cysteine engineered antibodies of the invention exhibit essentially the same affinity as compared to an antibody prior to cysteine engineering. In another embodiment, cysteine engineered antibodies of the invention exhibit a reduced affinity as compared to an antibody prior to cysteine engineering. In another embodiment, cysteine engineered antibodies of the invention exhibit an enhanced affinity as compared to an antibody prior to cysteine engineering.

Antibodies of the invention may have a high binding affinity to one or more of its cognate antigens. For example, an antibody described herein may have an association rate constant or kₒₙ rate (antibody (Ab) + antigen->Ab-Ag) of at least 2 X 10⁵ M⁻¹s⁻¹, at least 5 X 10⁵ M⁻¹s⁻¹, at least 10⁶ M⁻¹s⁻¹, at least 5 X 10⁶ M⁻¹s⁻¹, at least 10⁷ M⁻¹s⁻¹, at least 5 X 10⁷ M⁻¹s⁻¹, or at least 10⁸ M⁻¹s⁻¹.

In another embodiment, an antibody may have a k_{off} rate (Ab-Ag -> Ab + Ag) of less than 5x10⁻¹ s⁻¹, less than 10⁻¹ s⁻¹, less than 5x10⁻²s⁻¹, less than 10⁻² s⁻¹, less than 5x10⁻³ s⁻¹, less than 10⁻³ s⁻¹, less than 5x10⁻⁴s⁻¹, or less than 10⁻⁴ s^{-1.} In a another embodiment, an antibody of the invention has a k_{off} of less than 5x10⁻⁵ s⁻¹, less than 10⁻⁵ s⁻¹, less than 5x10⁻⁶ s⁻¹, less than 10⁻⁶ s⁻¹, less than 5x10⁻⁷ s⁻¹, less than 10⁻⁷ s⁻¹, less than 5x10⁻⁸ s⁻¹, less than 10⁻⁸ s⁻¹, less than 5x10⁻⁹ s⁻¹, less than 10⁻⁹ s⁻¹, or less than 10⁻¹⁰ s⁻¹.

In another embodiment, an antibody may have an affinity constant or Kₐ (kₒₙ/k_{off}) of at least 10² M⁻¹, at least 5 X 10² M⁻¹, at least 10³ M⁻¹, at least 5 X 10³ M⁻¹, at least 10⁴ M⁻¹, at least 5 X 10⁴ M⁻¹, at least 10⁵ M⁻¹, at least 5 X 10⁵ M⁻¹, at least 10⁶ M⁻¹, at least 5 X 10⁶ M⁻¹, at least 10⁷ M⁻¹, at least 5 X 10⁷ M⁻¹, at least 10⁸ M⁻¹, at least 5 X 10⁸ M⁻¹, at least 10⁹ M⁻¹, at least 5 X 10⁹ M⁻¹, at least 10¹⁰ M⁻¹, at least 5 X 10¹⁰ M⁻¹, at least 10¹¹ M⁻¹, at least 5 X 10¹¹ M⁻¹, at least 10¹² M⁻¹, at least 5 X 10¹² M⁻¹, at least 10¹³ M⁻¹, at least 5 X 10¹³ M⁻¹, at least 10¹⁴ M⁻¹, at least 5 X 10¹⁴ M⁻¹, at least 10¹⁵ M⁻¹, or at least 5 X 10¹⁵ M⁻¹. In yet another embodiment, an antibody may have a dissociation constant or K_{d} (k_{off}/kₒₙ) of less than 5x10⁻² M, less than 10⁻² M, less than 5x10⁻³ M, less than 10⁻³ M, less than 5x10⁻⁴ M, less than 10⁻⁴ M, less than 5x10⁻⁵ M, less than 10⁻⁵ M, less than 5x10⁻⁶ M, less than 10⁻⁶ M, less than 5x10⁻⁷ M, less than 10⁻⁷ M, less than 5x10⁻⁸ M, less than 10⁻⁸ M, less than 5x10⁻⁹ M, less than 10⁻⁹ M, less than 5x10⁻¹⁰ M, less than 10⁻¹⁰ M, less than 5x10⁻¹¹ M, less than 10⁻¹¹ M, less than 5x10⁻¹² M, less than 10⁻¹² M, less than 5x10⁻¹³ M, less than 10⁻¹³ M, less than 5x10⁻¹⁴ M, less than 10⁻¹⁴ M, less than 5x10⁻¹⁵ M, or less than 10⁻¹⁵ M.

An antibody used in accordance with a method described herein may have a dissociation constant (K_{d}) of less than 3000 pM, less than 2500 pM, less than 2000 pM, less than 1500 pM, less than 1000 pM, less than 750 pM, less than 500 pM, less than 250 pM, less than 200 pM, less than 150 pM, less than 100 pM, less than 75 pM as assessed using a method described herein or known to one of skill in the art *(e.g.,* a BIAcore assay, ELISA) (Biacore International AB, Uppsala, Sweden).

### Modulation of the Fc region

The invention also provides cysteine engineered antibodies with altered Fc regions (also referred to herein as "variant Fc regions"). Accordingly, in one embodiment, antibodies of the invention comprise a variant Fc region (i.e., Fc regions that have been altered as discussed below). Antibodies of the invention comprising a variant Fc region are also referred to here as "Fc variant protein(s)."

In the description of variant Fc regions, it is understood that the Fc regions of the antibodies of the invention comprise the numbering scheme according to the EU index as in Kabat et al. (1991, NIH Publication 91-3242, National Technical Information Service, Springfield, VA).

It is known that variants of the Fc region (*e.g.,* amino acid substitutions and/or additions and/or deletions) enhance or diminish effector function (see Presta et al., 2002, Biochem Soc Trans 30:487-490; U.S. patents 5,624,821, 5,885,573 and PCT publication Nos. WO 00/42072, WO 99/58572 and WO 04/029207). Accordingly, in one embodiment, the antibodies of the invention comprise variant Fc regions. In one embodiment, the variant Fc regions of antibodies exhibit a similar level of inducing effector function as compared to the native Fc. In another embodiment, the variant Fc region exhibits a higher induction of effector function as compared to the native Fc. In another embodiment, the variant Fc region exhibits lower induction of effector function as compared to the native Fc. In another embodiment, the variant Fc region exhibits higher induction of ADCC as compared to the native Fc. In another embodiment, the variant Fc region exhibits lower induction of ADCC as compared to the native Fc. In another embodiment, the variant Fc region exhibits higher induction of CDC as compared to the native Fc. In another embodiment, the variant Fc region exhibits lower induction of CDC as compared to the native Fc. Specific embodiments of variant Fc regions are detailed *infra.*

It is also known in the art that the glycosylation of the Fc region can be modified to increase or decrease effector function (see for examples, Umana et al, 1999, Nat. Biotechnol 17:176-180; Davies et al., 2001, Biotechnol Bioeng 74:288-294; Shields et al, 2002, J Biol Chem 277:26733-26740; Shinkawa et al., 2003, J Biol Chem 278:3466-3473) U.S. Pat. No. 6,602,684; U.S. Ser. No. 10/277,370; U.S. Ser. No. 10/113,929; PCT WO 00/61739A1; PCT WO 01/292246A1; PCT WO 02/311140A1; PCT WO 02/30954A1; Potillegent™ technology (Biowa, Inc. Princeton, N.J.); GlycoMAb™ glycosylation engineering technology (GLYCART biotechnology AG, Zurich, Switzerland).

Accordingly, in one embodiment, the Fc regions of antibodies of the invention comprise altered glycosylation of amino acid residues. In another embodiment, the altered glycosylation of the amino acid residues results in lowered effector function. In another embodiment, the altered glycosylation of the amino acid residues results in increased effector function. In a specific embodiment, the Fc region has reduced fucosylation. In another embodiment, the Fc region is afucosylated (see for examples, U.S. Patent Application Publication No.2005/0226867).

Recent research suggests that the addition of sialic acid to the oligosaccharides on IgG molecules enhances their anti-inflammatory activity and alter their cytotoxicity (Keneko et al., Science 313, 670-673(2006), Scallon et al., Mol. Immuno. 2007 Mar;44(7):1524-34). Thus, the efficacy of antibody therapeutics may be optimized by selection of a glycoform that is best suited to the intended application. The two oligosaccharide chains interposed between the two CH2 domains of antibodies are involved in the binding of the Fc region to its receptors. The studies referenced above demonstrate that IgG molecules with increased sialylation have anti-inflammatory properties whereas IgG molecules with reduced sialylation have increased immunostimulatory properties. Therefore, an antibody therapeutic can be "tailor-made" with an appropriate sialylation profile for a particular application. Methods for modulating the sialylation state of antibodies are presented in WO2007/005786 entitled "Methods And Compositions With Enhanced Therapeutic Activity", and WO2007/117505 entitled "Polypeptides With Enhanced Anti-Inflammatory And Decreased Cytotoxic Properties And Related Methods".

In one embodiment, the Fc regions of antibodies of the invention comprise an altered sialylation profile compared to a reference unaltered Fc region. In one embodiment, the Fc regions of antibodies of the invention comprise an increased sialylation profile compared to a reference unaltered Fc region. In some embodiments the Fc regions of antibodies of the invention comprise an increase in sialylation of about 5%, about 10%, about 15%, about 20%, about 25%, about 30%, about 35%, about 40%, about 45%, about 50%, about 60%, about 65%, about 70%, about 80%, about 85%, about 90%, about 95%, about 100%, about 125%, about 150% or more as compared to a reference unaltered Fc region. In some embodiments the Fc regions of antibodies of the invention comprise an increase in sialylation of about 2 fold, about 3 fold, about 4 fold, about 5 fold, about 10 fold, about 20 fold, about 50 fold or more as compared to an unaltered reference Fc region.

In another embodiment, the Fc regions of antibodies of the invention comprise a decreased sialylation profile compared to a reference unaltered Fc region. In some embodiments, the Fc regions of antibodies of the invention comprise a decrease in sialylation of about 5%, about 10%, about 15%, about 20%, about 25%, about 30%, about 35%, about 40%, about 45%, about 50%, about 60%, about 65%, about 70%, about 80%, about 85%, about 90%, about 95%, about 100%, about 125%, about 150% or more as compared to a reference unaltered Fc region. In some embodiments the Fc regions of antibodies of the invention comprise a decrease in sialylation of about 2 fold, about 3 fold, about 4 fold, about 5 fold, about 10 fold, about 20 fold, about 50 fold or more as compared to an unaltered reference Fc region.

It is also known in the art that the Fc region can be modified to increase the half-lives of proteins. The increase in half-life allows for the reduction in amount of drug given to a patient as well as reducing the frequency of administration. Accordingly, antibodies of the invention with increased half-lives may be generated by modifying (for example, substituting, deleting, or adding) amino acid residues identified as involved in the interaction between the Fc and the FcRn receptor (see, for examples, PCT publication Nos. 97/34631 and 02/060919 e). In addition, the half-life of antibodies of the invention may be increase by conjugation to PEG or Albumin by techniques widely utilized in the art. In some embodiments the Fc regions of antibodies of the invention comprise an increase in half-life of about 5%, about 10%, about 15%, about 20%, about 25%, about 30%, about 35%, about 40%, about 45%, about 50%, about 60%, about 65%, about 70%, about 80%, about 85%, about 90%, about 95%, about 100%, about 125%, about 150% or more as compared to a reference unaltered Fc region. In some embodiments the Fc regions of antibodies of the invention comprise an increase in half-life of about 2 fold, about 3 fold, about 4 fold, about 5 fold, about 10 fold, about 20 fold, about 50 fold or more as compared to an unaltered reference Fc region.

In an alternate embodiment, the Fc regions of antibodies of the invention comprise a decrease in half-life. In some embodiments the Fc regions of antibodies of the invention comprise a decrease in half-life of about 5%, about 10%, about 15%, about 20%, about 25%, about 30%, about 35%, about 40%, about 45%, about 50%, about 60%, about 65%, about 70%, about 80%, about 85%, about 90%, about 95%, about 100%, about 125%, about 150% or more as compared to a reference unaltered Fc region. In some embodiments the Fc regions of antibodies of the invention comprise a decrease in half-life of about 2 fold, about 3 fold, about 4 fold, about 5 fold, about 10 fold, about 20 fold, about 50 fold or more as compared to an unaltered reference Fc region.

The present invention encompasses Fc variant proteins which have altered binding properties for an Fc ligand (e.g., an Fc receptor, C1q) relative to a comparable molecule (e.g., a protein having the same amino acid sequence except having a wild type Fc region). Examples of binding properties include but are not limited to, binding specificity, equilibrium dissociation constant (K_{D}), dissociation and association rates (k_{off} and kₒₙ respectively), binding affinity and/or avidity. It is generally understood that a binding molecule (e.g., a Fc variant protein such as an antibody) with a low K_{D} may be preferable to a binding molecule with a high K_{D}. However, in some instances the value of the kₒₙ or k_{off} may be more relevant than the value of the K_{D}. One skilled in the art can determine which kinetic parameter is most important for a given antibody application.

The affinities and binding properties of an Fc region for its ligand may be determined by a variety of in vitro assay methods (biochemical or immunological based assays) known in the art for determining Fc-FcγR interactions, i.e., specific binding of an Fc region to an FcγR including but not limited to, equilibrium methods (e.g., enzyme-linked immunoabsorbent assay (ELISA), or radioimmunoassay (RIA)), or kinetics (e.g., BIACORE® analysis), and other methods such as indirect binding assays, competitive inhibition assays, fluorescence resonance energy transfer (FRET), gel electrophoresis and chromatography (e.g., gel filtration). These and other methods may utilize a label on one or more of the components being examined and/or employ a variety of detection methods including but not limited to chromogenic, fluorescent, luminescent, or isotopic labels. A detailed description of binding affinities and kinetics can be found in Paul, W.E., ed., Fundamental Immunology, 4th Ed., Lippincott-Raven, Philadelphia (1999), which focuses on antibody-immunogen interactions.

In one embodiment, the Fc variant protein has enhanced binding to one or more Fc ligand relative to a comparable molecule. In another embodiment, the Fc variant protein has an affinity for an Fc ligand that is at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold greater than that of a comparable molecule. In a specific embodiment, the Fc variant protein has enhanced binding to an Fc receptor. In another specific embodiment, the Fc variant protein has enhanced binding to the Fc receptor FcγRIIIA. In a further specific embodiment, the Fc variant protein has enhanced biding to the Fc receptor FcγRIIB. In still another specific embodiment, the Fc variant protein has enhanced binding to the Fc receptor FcRn. In yet another specific embodiment, the Fc variant protein has enhanced binding to C1q relative to a comparable molecule.

The ability of any particular Fc variant protein to mediate lysis of the target cell by ADCC can be assayed. To assess ADCC activity an Fc variant protein of interest is added to target cells in combination with immune effector cells, which may be activated by the antigen antibody complexes resulting in cytolysis of the target cell. Cytolysis is generally detected by the release of label (e.g. radioactive substrates, fluorescent dyes or natural intracellular proteins) from the lysed cells. Useful effector cells for such assays include peripheral blood mononuclear cells (PBMC) and Natural Killer (NK) cells. Specific examples of in vitro ADCC assays are described in Wisecarver et al., 1985 79:277-282; Bruggemann et al., 1987, J Exp Med 166:1351-1361; Wilkinson et al., 2001, J Immunol Methods 258:183-191; Patel et al., 1995 J Immunol Methods 184:29-38. ADCC activity of the Fc variant protein of interest may also be assessed in vivo, e.g., in an animal model such as that disclosed in Clynes et al., 1998, Proc. Natl. Acad. Sci. USA 95:652-656.

In one embodiment, an Fc variant protein has enhanced ADCC activity relative to a comparable molecule. In a specific embodiment, an Fc variant protein has ADCC activity that is at least 2 fold, or at least 3 fold, or at least 5 fold or at least 10 fold or at least 50 fold or at least 100 fold greater than that of a comparable molecule. In another specific embodiment, an Fc variant protein has enhanced binding to the Fc receptor FcγRIIIA and has enhanced ADCC activity relative to a comparable molecule. In other embodiments, the Fc variant protein has both enhanced ADCC activity and enhanced serum half life relative to a comparable molecule.

In one embodiment, an Fc variant protein has reduced ADCC activity relative to a comparable molecule. In a specific embodiment, an Fc variant protein has ADCC activity that is at least 2 fold, or at least 3 fold, or at least 5 fold or at least 10 fold or at least 50 fold or at least 100 fold lower than that of a comparable molecule. In another specific embodiment, an Fc variant protein has reduced binding to the Fc receptor FcγRIIIA and has reduced ADCC activity relative to a comparable molecule. In other embodiments, the Fc variant protein has both reduced ADCC activity and enhanced serum half life relative to a comparable molecule.

In one embodiment, an Fc variant protein has enhanced CDC activity relative to a comparable molecule. In a specific embodiment, an Fc variant protein has CDC activity that is at least 2 fold, or at least 3 fold, or at least 5 fold or at least 10 fold or at least 50 fold or at least 100 fold greater than that of a comparable molecule. In other embodiments, the Fc variant protein has both enhanced CDC activity and enhanced serum half life relative to a comparable molecule. In one embodiment, the Fc variant protein has reduced binding to one or more Fc ligand relative to a comparable molecule. In another embodiment, the Fc variant protein has an affinity for an Fc ligand that is at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold lower than that of a comparable molecule. In a specific embodiment, the Fc variant protein has reduced binding to an Fc receptor. In another specific embodiment, the Fc variant protein has reduced binding to the Fc receptor FcγRIIIA. In a further specific embodiment, an Fc variant described herein has an affinity for the Fc receptor FcγRIIIA that is at least about 5 fold lower than that of a comparable molecule, wherein said Fc variant has an affinity for the Fc receptor FcγRIIB that is within about 2 fold of that of a comparable molecule. In still another specific embodiment, the Fc variant protein has reduced binding to the Fc receptor FcRn. In yet another specific embodiment, the Fc variant protein has reduced binding to C1q relative to a comparable molecule.

In one embodiment, the present invention provides Fc variants, wherein the Fc region comprises a non naturally occurring amino acid residue at one or more positions selected from the group consisting of 234, 235, 236, 237, 238, 239, 240, 241, 243, 244, 245, 247, 251, 252, 254, 255, 256, 262, 263, 264, 265, 266, 267, 268, 269, 279, 280, 284, 292, 296, 297, 298, 299, 305, 313, 316, 325, 326, 327, 328, 329, 330, 331, 332, 333, 334, 339, 341, 343, 370, 373, 378, 392, 416, 419, 421, 440 and 443 as numbered by the EU index as set forth in Kabat. Optionally, the Fc region may comprise a non naturally occurring amino acid residue at additional and/or alternative positions known to one skilled in the art (see, e.g., U.S. Patents 5,624,821; 6,277,375; 6,737,056; PCT Patent Publications WO 01/58957; WO 02/06919; WO 04/016750; WO 04/029207; WO 04/035752; WO 04/074455; WO 04/099249; WO 04/063351; WO 05/070963; WO 05/040217, WO 05/092925 and WO 06/020114).

In a specific embodiment, the present invention provides an Fc variant, wherein the Fc region comprises at least one non naturally occurring amino acid residue selected from the group consisting of 234D, 234E, 234N, 234Q, 234T, 234H, 234Y, 234I, 234V, 234F, 235A, 235D, 235R, 235W, 235P, 235S, 235N, 235Q, 235T, 235H, 235Y, 235I, 235V, 235F, 236E, 239D, 239E, 239N, 239Q, 239F, 239T, 239H, 239Y, 240I, 240A, 240T, 240M, 241W, 241 L, 241Y, 241E, 241 R. 243W, 243L 243Y, 243R, 243Q, 244H, 245A, 247L, 247V, 247G, 251F, 252Y, 254T, 255L, 256E, 256M, 262I, 262A, 262T, 262E, 263I, 263A, 263T, 263M, 264L, 264I, 264W, 264T, 264R, 264F, 264M, 264Y, 264E, 265G, 265N, 265Q, 265Y, 265F, 265V, 265I, 265L, 265H, 265T, 266I, 266A, 266T, 266M, 267Q, 267L, 268E, 269H, 269Y, 269F, 269R, 270E, 280A, 284M, 292P, 292L, 296E, 296Q, 296D, 296N, 296S, 296T, 296L, 296I, 296H, 269G, 297S, 297D, 297E, 298H, 298I, 298T, 298F, 299I, 299L, 299A, 299S, 299V, 299H, 299F, 299E, 305I, 313F, 316D, 325Q, 325L, 325I, 325D, 325E, 325A, 325T, 325V, 325H, 327G, 327W, 327N, 327L, 328S, 328M, 328D, 328E, 328N, 328Q, 328F, 328I, 328V, 328T, 328H, 328A, 329F, 329H, 329Q, 330K, 330G, 330T, 330C, 330L, 330Y, 330V, 330I, 330F, 330R, 330H, 331G, 331A, 331L, 331M, 331F, 331W, 331K, 331Q, 331E, 331S, 331V, 331I, 331C, 331Y, 331H, 331R, 331N, 331D, 331T, 332D, 332S, 332W, 332F, 332E, 332N, 332Q, 332T, 332H, 332Y, 332A, 339T, 370E, 370N, 378D, 392T, 396L, 416G, 419H, 421K, 440Yand 434W as numbered by the EU index as set forth in Kabat. Optionally, the Fc region may comprise additional and/or alternative non naturally occurring amino acid residues known to one skilled in the art (see, e.g., U.S. Patents 5,624,821; 6,277,375; 6,737,056; PCT Patent Publications WO 01/58957; WO 02/06919; WO 04/016750; WO 04/029207; WO 04/035752 and WO 05/040217).

### Methods of Producing Antibodies

The cysteine engineered antibodies of the invention may be produced by any method known in the art for the synthesis of antibodies, in particular, by chemical synthesis or preferably, by recombinant expression techniques.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., *Antibodies: A Laboratory Manual*, (Cold Spring Harbor Laboratory Press, 2^{nd} ed. 1988); Hammerling, et al., in: *Monoclonal Antibodies and T-Cell Hybridomas* 563-681 (Elsevier, N.Y., 1981. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. Briefly, mice can be immunized with a target antigen (either the full length protein or a domain thereof, *e.g.,* the extracellular domain or the ligand binding domain) and once an immune response is detected, *e.g.,* antibodies specific for the target antigen are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. Hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Accordingly, monoclonal antibodies can be generated by culturing a hybridoma cell secreting an antibody of the invention wherein, preferably, the hybridoma is generated by fusing splenocytes isolated from a mouse immunized with a target antigen with myeloma cells and then screening the hybridomas resulting from the fusion for hybridoma clones that secrete an antibody able to bind to a specific target antigen.

Antibody fragments which recognize specific target antigen epitopes may be generated by any technique known to those of skill in the art. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain. Further, the antibodies of the present invention can also be generated using various phage display methods known in the art.

In phage display methods, functional antibody domains are displayed on the surface of phage particles which carry the polynucleotide sequences encoding them. In particular, DNA sequences encoding VH and VL domains are amplified from animal cDNA libraries (e.g., human or murine cDNA libraries of lymphoid tissues). The DNA encoding the VH and VL domains are recombined together with an scFv linker by PCR and cloned into a phagemid vector *(e.g.,* p CANTAB 6 or pComb 3 HSS). The vector is electroporated in *E. coli* and the *E. coli* is infected with helper phage. Phage used in these methods are typically filamentous phage including fd and M13 and the VH and VL domains are usually recombinantly fused to either the phage gene III or gene VIII. Phage expressing an antigen binding domain that binds to an epitope of interest can be selected or identified with antigen, *e.g*., using labeled antigen or antigen bound or captured to a solid surface or bead. Examples of phage display methods that can be used to make the antibodies of the present invention include those disclosed in Brinkman et al., 1995, J. Immunol. Methods 182:41-50; Ames et al., 1995, J. Immunol. Methods 184:177; Kettleborough et al., 1994, Eur. J. Immunol. 24:952-958; Persic et al., 1997, Gene 187:9; Burton et al., 1994, Advances in Immunology 57:191-280; International Application No. PCT/GB91/01134; International Publication Nos. WO 90/02809, WO 91/10737, WO 92/01047, WO 92/18619, WO 93/1 1236, WO 95/15982, WO 95/20401, and WO97/13844; and U.S. Patent Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, *e.g.,* as described below. Techniques to recombinantly produce Fab, Fab' and F(ab')2 fragments can also be employed using methods known in the art such as those disclosed in International Publication No. WO 92/22324; Mullinax et al., 1992, BioTechniques 12:864; Sawai et al., 1995, AJRI 34:26; and Better et al., 1988, Science 240:1041 ().

To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, *e.g.,* the human gamma 4 constant region, and the PCR amplified VL domains can be cloned into vectors expressing a VL constant region, *e.g.,* human kappa or lambda constant regions. Preferably, the vectors for expressing the VH or VL domains comprise an EF-1α promoter, a secretion signal, a cloning site for the variable domain, constant domains, and a selection marker such as neomycin. The VH and VL domains may also be cloned into one vector expressing the necessary constant regions. The heavy chain conversion vectors and light chain conversion vectors are then co-transfected into cell lines to generate stable or transient cell lines that express full-length antibodies, *e.g.,* IgG, using techniques known to those of skill in the art.

For some uses, including *in vivo* use of antibodies in humans and *in vitro* detection assays, it may be preferable to use human or chimeric antibodies. Completely human antibodies are particularly desirable for therapeutic treatment of human subjects. Human antibodies can be made by a variety of methods known in the art including phage display methods described above using antibody libraries derived from human immunoglobulin sequences. See also U.S. Patent Nos. 4,444,887 and 4,716,111; and International Publication Nos. WO 98/46645, WO 98/50433, WO 98/24893, WO 98/16654, WO 96/34096, WO 96/33735, and WO 91/10741;.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the J_{H} region prevents endogenous antibody production. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, *e.g.,* all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see, e.g.,* International Publication Nos. WO 98/24893, WO 96/34096, and WO 96/33735; and U.S. Patent Nos. 5,413,923, 5,625,126, 5,633,425, 5,569,825, 5,661,016, 5,545,806, 5,814,318, and 5,939,598. In addition, companies such as Medarex (Princeton, NJ) can be engaged to provide human antibodies directed against a selected antigen using technology similar to that described above.

A chimeric antibody is a molecule in which different portions of the antibody are derived from different immunoglobulin molecules such as antibodies having a variable region derived from a non-human antibody and a human immunoglobulin constant region. Methods for producing chimeric antibodies are known in the art. See *e.g*., Morrison, 1985, Science 229:1202; Oi et al., 1986, BioTechniques 4:214; Gillies et al., 1989, J. Immunol. Methods 125:191-202; and U.S. Patent Nos. 6,311,415, 5,807,715, 4,816,567, and 4,816,397. Chimeric antibodies comprising one or more CDRs from a non-human species and framework regions from a human immunoglobulin molecule can be produced using a variety of techniques known in the art including, for example, CDR-grafting (EP 239,400; International Publication No. WO 91/09967; and U.S. Patent Nos. 5,225,539, 5,530,101, and 5,585,089), veneering or resurfacing (EP 592,106; EP 519,596; Padlan, 1991, Molecular Immunology 28(4/5):489-498; Studnicka et al., 1994, Protein Engineering 7:805; and Roguska et al., 1994, PNAS 91:969), and chain shuffling (U.S. Patent No. 5,565,332).

Often, framework residues in the framework regions will be substituted with the corresponding residue from the CDR donor antibody to alter, preferably improve, antigen binding. These framework substitutions are identified by methods well known in the art, *e.g.,* by modeling of the interactions of the CDR and framework residues to identify framework residues important for antigen binding and sequence comparison to identify unusual framework residues at particular positions. (See, *e.g.,* U.S. Patent No. 5,585,089; and Riechmann et al., 1988, Nature 332:323.).

A humanized antibody is an antibody or its variant or fragment thereof which is capable of binding to a predetermined antigen and which comprises a framework region having substantially the amino acid sequence of a human immunoglobulin and a CDR having substantially the amino acid sequence of a non-human immunoglobulin. A humanized antibody comprises substantially all of at least one, and typically two, variable domains in which all or substantially all of the CDR regions correspond to those of a non-human immunoglobulin (*i.e.,* donor antibody) and all or substantially all of the framework regions are those of a human immunoglobulin consensus sequence. Preferably, a humanized antibody also comprises at least a portion of an immunoglobulin constant region (Fc), typically that of a human immunoglobulin. Ordinarily, the antibody will contain both the light chain as well as at least the variable domain of a heavy chain. The antibody also may include the CH1, hinge, CH2, CH3, and CH4 regions of the heavy chain. The humanized antibody can be selected from any class of immunoglobulins, including IgM, IgG, IgD, IgA and IgE, and any isotype, including IgG₁, IgG₂, IgG₃ and IgG₄. Usually the constant domain is a complement fixing constant domain where it is desired that the humanized antibody exhibit cytotoxic activity, and the class is typically IgG₁. Where such cytotoxic activity is not desirable, the constant domain may be of the IgG₂ class. The humanized antibody may comprise sequences from more than one class or isotype, and selecting particular constant domains to optimize desired effector functions is within the ordinary skill in the art. The framework and CDR regions of a humanized antibody need not correspond precisely to the parental sequences, *e.g*., the donor CDR or the consensus framework may be mutagenized by substitution, insertion or deletion of at least one residue so that the CDR or framework residue at that site does not correspond to either the consensus or the import antibody. Such mutations, however, will not be extensive. Usually, at least 75% of the humanized antibody residues will correspond to those of the parental framework region (FR) and CDR sequences, more often 90%, or even greater than 95%.

Humanized antibodies can be produced using variety of techniques known in the art, including but not limited to, CDR-grafting (European Patent No. EP 239,400; International Publication No. WO 91/09967; and U.S. Patent Nos. 5,225,539, 5,530,101, and 5,585,089), veneering or resurfacing (European Patent Nos. EP 592,106 and EP 519,596; Padlan, 1991, Molecular Immunology 28(4/5):489-498; Studnicka et al., 1994, Protein Engineering 7(6):805-814; and Roguska et al., 1994, PNAS 91:969-973), chain shuffling (U.S. Patent No. 5,565,332), and techniques disclosed in, *e.g.,* U.S. Patent Nos. 6,407,213, 5,766,886, 5,585,089, International Publication No. WO 9317105, Tan et al., 2002, J. Immunol. 169:1119-25, Caldas et al., 2000, Protein Eng. 13:353-60, Morea et al., 2000, Methods 20:267-79, Baca et al., 1997, J. Biol. Chem. 272:10678-84, Roguska et al., 1996, Protein Eng. 9:895-904, Couto et al., 1995, Cancer Res. 55 (23 Supp):5973s-5977s, Couto et al., 1995, Cancer Res. 55:1717-22, Sandhu, 1994, Gene 150:409-10, Pedersen et al., 1994, J. Mol. Biol. 235:959-73, Jones et al., 1986, Nature 321:522-525, Riechmann et al., 1988, Nature 332:323, and Presta, 1992, Curr. Op. Struct. Biol. 2:593-596. Often, framework residues in the framework regions will be substituted with the corresponding residue from the CDR donor antibody to alter, preferably improve, antigen binding. These framework substitutions are identified by methods well known in the art, *e.g.,* by modeling of the interactions of the CDR and framework residues to identify framework residues important for antigen binding and sequence comparison to identify unusual framework residues at particular positions. (*See, e.g.,* Queen et al., U.S. Patent No. 5,585,089; and Riechmann et al., 1988, Nature 332:323).

Further, the antibodies of the invention can, in turn, be utilized to generate anti-idiotype antibodies using techniques well known to those skilled in the art. (See, *e.g.,* Greenspan & Bona, 1989, FASEB J. 7:437-444; and Nissinoff, 1991, J. Immunol. 147:2429-2438). The invention provides methods employing the use of polynucleotides comprising a nucleotide sequence encoding an antibody of the invention or a fragment thereof.

In other embodiments, the invention comprises the expression of an isolated CH1 domain comprising cysteine engineered residues. Such isolated CH1 domains may be useful as scaffolds for display purposes, In other embodiments, the isolated CIII domains may be used in conjunction with Ckappa or Clambda subunits from an antibody light chain.

In yet other embodiments, antibodies of the invention may comprise a hinge region lacking at least one cysteine residue. In other embodiments, antibodies of the invention may comprise a hinge refion devoid of cysteine residues. In some embodiments, antibodies of the invention may comprise a hinge region in which all the cysteine residues are replace with cither serine or threonine. Such antibodies may exhibit increased conjugation efficiency and less disulfide scrambling.

Additionally, various publications describe methods for obtaining physiologically active molecules whose half-lives are modified either by introducing an FcRn-binding polypeptide into the molecules (WO 97/43316; U.S. Pat. No. 5,869,046; U.S. Pat. No. 5,747,035; WO 96/32478; WO 91/14438) or by fusing the molecules with antibodies whose FcRn-binding affinities are preserved but affinities for other Fc receptors have been greatly reduced (WO 99/43713) or fusing with FcRn binding domains of antibodies (WO 00/09560; U.S. Pat. No. 4,703,039). Specific techniques and methods of increasing half-life of physiologically active molecules can also be found in U.S. Patent No. 7,083.784 granted Aug 1, 2006 entitled "Antibodies with Increased Half-lives". Specifically, it is contemplated that the antibodies of the invention comprise an Fc region comprising ammo acid residue mutations (as numbered by the hU index in Kabat): M252Y/S2541/T256E or H433K/N434F/Y436H.

### Polynucleotides Encoding an Antibody

The polynucleotides may be obtained, and the nucleotide sequence of the polynucleotides determined, by any method known in the art. Since the amino acid sequences of the antibodies are known, nucleotide sequences encoding these antibodies can be determined using methods well known in the art, *i.e.,* nucleotide codons known to encode particular amino acids are assembled in such a way to generate a nucleic acid that encodes the antibody or fragment thereof of the invention. Such a polynucleotide encoding the antibody may be assembled from chemically synthesized oligonucleotides (e.g., as described in Kutmeier et al., 1994, BioTechniques 17:242), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from nucleic acid from a suitable source. If a clone containing a nucleic acid encoding a particular antibody is not available, but the sequence of the antibody is known, a nucleic acid encoding the immunoglobulin may be chemically synthesized or obtained from a suitable source (*e.g.,* an antibody cDNA library, or a cDNA library generated from, or nucleic acid, preferably poly A+ RNA, isolated from, any tissue or cells expressing the antibody by PCR amplification using synthetic primers hybridizable to the 3' and 5' ends of the sequence or by cloning using an oligonucleotide probe specific for the particular gene sequence to identify, *e.g.,* a cDNA clone from a cDNA library that encodes the antibody. Amplified nucleic acids generated by PCR may then be cloned into replicable cloning vectors using any method well known in the art.

Once the nucleotide sequence of the antibody is determined, the nucleotide sequence of the antibody may be manipulated using methods well known in the art for the manipulation of nucleotide sequences, *e.g.*, recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY), to generate antibodies having a different amino acid sequence, for example to create amino acid substitutions, deletions, and/or insertions.

In a specific embodiment, one or more of the CDRs is inserted within framework regions using routine recombinant DNA techniques. The framework regions may be naturally occurring or consensus framework regions, and preferably human framework regions (see, *e.g.,* Chothia et al., 1998, J. Mol. Biol. 278: 457-479 for a listing of human framework regions). Preferably, the polynucleotide generated by the combination of the framework regions and CDRs encodes an antibody that specifically binds to EphA2 or EphA4. Preferably, as discussed *supra,* one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibodies lacking one or more intrachain disulfide bonds. Other alterations to the polynucleotide are encompassed by the present invention and within the skill of the art.

### Recombinant Expression of an Antibody

Recombinant expression of an antibody of the invention, derivative, analog or fragment thereof, requires construction of an expression vector containing a polynucleotide that encodes the antibody. Once a polynucleotide encoding an antibody or a heavy or light chain of an antibody, or portion thereof, of the invention has been obtained, the vector for the production of the antibody may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination.

The invention, thus, provides replicable vectors comprising a nucleotide sequence encoding an antibody of the invention, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody (see, *e.g.*, International Publication Nos. WO 86/05807 and WO 89/01036; and U.S. Patent No. 5,122,464) and the variable domain of the antibody may be cloned into such a vector for expression of the entire heavy, the entire light chain, or both the entire heavy and light chains.

The expression vector is transferred to a host cell by conventional techniques and the transfected cells are then cultured by conventional techniques to produce an antibody of the invention. Thus, the invention includes host cells containing a polynucleotide encoding an antibody of the invention or fragments thereof, or a heavy or light chain thereof, or portion thereof, or a single chain antibody of the invention, operably linked to a heterologous promoter. In certain embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibodies of the invention (see, *e.g.,* U.S. Patent No. 5,807,715). Such host-expression systems represent vehicles by which the coding sequences of interest may be produced and subsequently purified, but also represent cells which may, when transformed or transfected with the appropriate nucleotide coding sequences, express an antibody of the invention *in situ.* These include but are not limited to microorganisms such as bacteria (*e.g., E. coli* and *B. subtilis*) transformed with recombinant bacteriophage DNA, plasmid DNA or cosmid DNA expression vectors containing antibody coding sequences; yeast (*e.g., Saccharomyces Pichia*) transformed with recombinant yeast expression vectors containing antibody coding sequences; insect cell systems infected with recombinant virus expression vectors *(e.g.,* baculovirus) containing antibody coding sequences; plant cell systems infected with recombinant virus expression vectors (*e.g.,* cauliflower mosaic virus, CaMV; tobacco mosaic virus, TMV) or transformed with recombinant plasmid expression vectors *(e.g.,* Ti plasmid) containing antibody coding sequences; or mammalian cell systems (*e.g.,* COS, CHO, BHK, 293, NS0, and 3T3 cells) harboring recombinant expression constructs containing promoters derived from the genome of mammalian cells (*e.g.,* metallothionein promoter) or from mammalian viruses (*e.g.,* the adenovirus late promoter; the vaccinia virus 7.5K promoter). Preferably, bacterial cells such as *Escherichia coli,* and more preferably, eukaryotic cells, especially for the expression of whole recombinant antibody, are used for the expression of a recombinant antibody.

For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, BioTechnology 8:2).

In bacterial systems, a number of expression vectors may be advantageously selected depending upon the use intended for the antibody being expressed. For example, when a large quantity of such a protein is to be produced, for the generation of pharmaceutical compositions of an antibody, vectors which direct the expression of high levels of fusion protein products that are readily purified may be desirable. Such vectors include, but are not limited to, the *E. coli* expression vector pUR278 (Ruther et al., 1983, EMBO 12:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. PGEX vectors may also be used to express foreign polypeptides as fusion proteins with glutathione 5-transferase (GST). In general, such fusion proteins are soluble and can easily be purified from lysed cells by adsorption and binding to matrix glutathione-agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in *Spodoptera frugiperda* cells. The antibody coding sequence may be cloned individually into non-essential regions of the virus and placed under control of an AcNPV promoter.

In mammalian host cells, a number of viral-based expression systems may be utilized. In cases where an adenovirus is used as an expression vector, the antibody coding sequence of interest may be ligated to an adenovirus transcription/translation control complex, *e.g.,* the late promoter and tripartite leader sequence. This chimeric gene may then be inserted in the adenovirus genome by *in vitro* or *in vivo* recombination. Insertion in a non-essential region of the viral genome *(e.g.,* region El or E3) will result in a recombinant virus that is viable and capable of expressing the antibody in infected hosts *(e.g.,* see Logan & Shenk, 1984, PNAS 8 1:6355-6359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, *e.g.,* Bittner et al., 1987, Methods in Enzymol. 153:516-544).

In addition, a host cell strain may be chosen which modulates the expression of the inserted sequences, or modifies and processes the gene product in the specific fashion desired. Such modifications (*e.g.,* glycosylation) and processing *(e.g.,* cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERO, BHK, HeLa, COS, MDCK, 293, 3T3, W138, BT483, Hs578T, HTB2, BT2O, NS1 and T47D, NS0 (a murine myeloma cell line that does not endogenously produce any immunoglobulin chains), CRL7O30 and HsS78Bst cells.

For long-term, high-yield production of recombinant proteins, stable expression is preferred. For example, cell lines which stably express the antibody may be engineered. Rather than using expression vectors which contain viral origins of replication, host cells can be transformed with DNA controlled by appropriate expression control elements (*e.g.,* promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody. Such engineered cell lines may be particularly useful in screening and evaluation of compositions that interact directly or indirectly with the antibody.

A number of selection systems may be used, including but not limited to, the herpes simplex virus thymidine kinase (Wigler et al., 1977, Cell 11:223), glutamine synthetase, hypoxanthine guanine phosphoribosyltransferase (Szybalska & Szybalski, 1992, Proc. Natl. Acad. Sci. USA 48:202), and adenine phosphoribosyltransferase (Lowy et al., 1980, Cell 22:8-17) genes can be employed in tk-, gs-, hgprt- or aprt- cells, respectively. Also, antimetabolite resistance can be used as the basis of selection for the following genes: *dhfr,* which confers resistance to methotrexate (Wigler et al., 1980, PNAS 77:357; O'Hare et al., 1981, PNAS 78:1527); *gpt,* which confers resistance to mycophenolic acid (Mulligan & Berg, 1981, PNAS 78:2072); neo, which confers resistance to the aminoglycoside G-418 (Wu and Wu, 1991, Biotherapy 3:87; Tolstoshev, 1993, Ann. Rev. Pharmacol. Toxicol. 32:573; Mulligan, 1993, Science 260:926; and Morgan and Anderson, 1993, Ann. Rev. Biochem. 62: 191; May, 1993, TIB TECH 11:155-); and *hygro*, which confers resistance to hygromycin (Santerre et al., 1984, Gene 30:147). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., 1981, J. Mol. Biol. 150:1,.

The expression levels of an antibody can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, PNAS 77:2197). The coding sequences for the heavy and light chains may comprise cDNA or genomic DNA.

Once a cysteine engineered antibody of the invention has been produced by recombinant expression, it may be purified by any method known in the art for purification of an immunoglobulin molecule, for example, by chromatography (e.g., ion exchange, affinity, particularly by affinity for the specific antigen after Protein A, and sizing column chromatography), centrifugation, differential solubility, or by any other standard technique for the purification of proteins. Further, the antibodies of the present invention or fragments thereof may be fused to heterologous polypeptide sequences described herein or otherwise known in the art to facilitate purification.

### Scalable Production of Cysteine engineered antibodies

In an effort to obtain large quantities of the cysteine engineered antibodies of the invention, they may be produced by a scalable process (hereinafter referred to as "scalable process of the invention"). In some embodiments, cysteine engineered antibodies may be produced by a scalable process of the invention in the research laboratory that may be scaled up to produce the proteins of the invention in analytical scale bioreactors (for example, but not limited to 5 L, 10L, 15L, 3OL, or 50L bioreactors) while maintaining the functional activity of the proteins. For instance, in one embodiment, proteins produced by scalable processes of the invention exhibit low to undetectable levels of aggregation as measured by HPSEC or rCGE, that is, no more than 5%, no more than 4%, no more than 3%, no more than 2%, no more than 1%, or no more than 0.5% aggregate by weight protein, and/or low to undetectable levels of fragmentation, that is, 80% or higher, 85% or higher, 90% or higher, 95% or higher, 98% or higher, or 99% or higher, or 99.5% or higher of the total peak area in the peak(s) representing intact cysteine engineered antibodies.

In other embodiments, the cysteine engineered antibodies may be produced by a scalable process of the invention in the research laboratory that may be scaled up to produce the proteins of the invention in production scale bioreactors (for example, but not limited to 75L5 10OL, 150L, 3001., or 500L). In some embodiments, the scalable process of the invention results in little or no reduction in production efficiency as compared to the production process performed in the research laboratory. In other embodiments, the scalable process of the invention produces cysteine engineered antibodies at production efficiency of about 10 mg/L, about 20 m/L, about 30 mg/L, about 50 mg/L, about 75 mg/L, about 100 mg/ L, about 125 mg/L, about 150 mg/L, about 175 mg/L, about 200 mg/L, about 250 mg/L, about 300 mg/L or higher.

In other embodiments, the scalable process of the invention produces cysteine engineered antibodies at production efficiency of at least about 10 mg/L, at least about 20 mg/L, at least about 30 mg/L, at least about. 50 mg/L, at least about 75 mg/L, at least about 100 mg/L, at least about 125 mg/L, at least about 150 mg/L, at least about 175 mg/L. at least about 200 mg-'L, at least about 250 mg/L, at least about 300 mg/L. or higher.

In other embodiments, the scalable process of the invention produces cysteine engineered antibodies at production efficiency from about 10 mg/L to about 300 mg/L, from about 10 mg/L to about 250 mg/L, from about 10 mg/L to about 200 mg/L, from about 10 mg/L to about 175 mg/L, from about 10 mg/L to about 150 rag/L, from about 10 mg/L to about 100 mg/L, from about 20 mg/L to about 300 mg/L, from about 20 mg/L to about 250 mg/L, from about 20 mg/L to about 200 mg/L, from 20 mg/L to about 175 mg/L, from about 20 mg/L to about 150 mg/L, from about 20 mg/L to about 125 mg/L, from about 20 mg/L to about 100 mg/L, from about 30 mg/L to about 300 mg/L, from about 30 mg/L to about 250 mg/L, from about 30 mg/L to about 200 mg/L, from about 30 mg/L to about 175 mg/L, from about 30 mg/L to about 150 mg/L, from about 30 mg/L to about 125 mg/L, from about 30 mg/L to about 100 mg/L, from about 50 mg/L to about 300 mg/L, from about 50 mg/L- to about 250 mg/L, from about 50 mg/L to about 200 mg/L, from 50 mg/L to about 175 mg/L, from about 50 mg/L to about 150 mg/L, from about 50 mg/L to about 125 mg/L, front about 50 mg/L to about 100 mg/L.

To ensure the stability of the antibodies of the invention, suitable assays have been developed. In one embodiment, the stability of proteins of the invention is characterized by known techniques in the art. In other embodiments, the stability of the proteins of the invention can be assessed by aggregation and/or fragmentation rate or profile. To determine the level of aggregation or fragmentation, many techniques may be used. In one embodiment, the aggregation and/or fragmentation profile may be assessed by the use of analytical ultracentrifugation (AUC), size-exclusion chromatography (SEC), high-performance size-exclusion chromatography (HPSEC), melting temperature (Tₘ), polyacrylamide gel electrophoresis (PAGE), capillary gel electrophoresis (CGE), light scattering (SLS), Fourier Transform Infrared Spectroscopy (FTIR), circular dichroism (CD), urea-induced protein unfolding techniques, intrinsic tryptophan fluorescence, differential scanning calorimetry, or 1-anilino-8-naphthalenesulfonic acid (ANS) protein binding techniques. In another embodiment, the stability of proteins of the invention is characterized by polyacrylamide gel electrophoresis (PAGE) analysis. In another embodiment, the stability of the proteins of the invention is characterized by size exclusion chromatography (SEC) profile analysis.

### Antibody Conjugates

The present invention encompasses the use of cysteine engineered antibodies recombinantly fused or chemically conjugated (including both covalent and non-covalent conjugations) to a heterologous agent to generate a fusion protein as targeting moieties (hereinafter referred to as "antibody conjugates"). The heterologous agent may be a polypeptide (or portion thereof, preferably to a polypeptide of at least 10, at least 20, at least 30, at least 40, at least 50, at least 60, at least 70, at least 80, at least 90 or at least 100 amino acids), nucleic acid, small molecule (less than 1000 daltons), or inorganic or organic compound. The fusion does not necessarily need to be direct, but may occur through linker sequences. Antibodies fused or conjugated to heterologous agents may be used *in vivo* to detect, treat, manage, or monitor the progression of a disorder using methods known in the art. See *e.g.,* International Publication WO 93/21232; EP 439,095; Naramura et al., 1994, Immunol. Lett. 39:91-99; U.S. Patent 5,474,981; Gillies et al., 1992, PNAS 89:1428-1432; and Fell et al., 1991, J. Immunol. 146:2446-2452,. In some embodiments, the disorder to be detected, treated, managed, or monitored is an autoimmune, inflammatory, infectious disease or cancer related disorder. Methods for fusing or conjugating polypeptides to antibody portions are known in the art. See, *e.g.,* U.S. Patent Nos. 5,336,603, 5,622,929, 5,359,046, 5,349,053, 5,447,851, and 5,112,946; EP 307,434; EP 367,166; International Publication Nos. WO 96/04388 and WO 91/06570; Ashkenazi et al., 1991, PNAS 88: 10535-10539; Zheng et al., 1995, J. Immunol. 154:5590-5600; and Vil et al., 1992, PNAS 89:11337- 11341.

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of cysteine engineered antibodies of the invention (e.g., antibodies with higher affinities and lower dissociation rates). See, generally, U.S. Patent Nos. 5,605,793; 5,811,238; 5,830,721; 5,834,252; and 5,837,458, and Patten et al., 1997, Curr. Opinion Biotechnol. 8:724-33; Harayama, 1998, Trends Biotechnol. 16:76; Hansson, et al., 1999, J. Mol. Biol. 287:265; and Lorenzo and Blasco, 1998, BioTechniques 24:308. Antibodies or fragments thereof, or the encoded antibodies or fragments thereof, may be altered by being subjected to random mutagenesis by error-prone PCR, random nucleotide insertion or other methods prior to recombination. One or more portions of a polynucleotide encoding an antibody or antibody fragment may be recombined with one or more components, motifs, sections, parts, domains, fragments, etc. of one or more heterologous agents.

In one embodiment, cysteine engineered antibodies of the present invention or fragments or variants thereof are conjugated to a marker sequence, such as a peptide, to facilitate purification. In certain embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., 1989, PNAS 86:821, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., 1984, Cell 37:767) and the "flag" tag.

In other embodiments, antibodies of the present invention thereof are conjugated to a diagnostic or detectable agent. Such antibodies can be useful for monitoring or prognosing the development or progression of a disorder (such as, but not limited to cancer) as part of a clinical testing procedure, such as determining the efficacy of a particular therapy.

Such diagnosis and detection can accomplished by coupling the antibody to detectable substances including, but not limited to various enzymes, such as but not limited to horseradish peroxidase, alkaline phosphatase, beta-galactosidase, or acetylcholinesterase; prosthetic groups, such as but not limited to streptavidin/biotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as but not limited to, bioluminescent materials, such as but not limited to, luciferase, luciferin, and aequorin; radioactive materials, such as but not limited to, bismuth (²¹³Bi), carbon (¹⁴C), chromium (⁵¹Cr), cobalt (¹⁷Co), fluorine (¹⁸F), gadolinium (¹⁵³Gd, ¹⁵⁹Gd), gallium (⁶⁸Ga, ⁶⁷Ga), germanium (⁶⁸Ge), holmium (¹⁶⁶Ho), indium (¹¹⁵In, ¹¹³In, ¹¹²In, ¹¹¹In), iodine (¹³¹I, ¹²⁵I, ¹²³I, ¹²¹I), lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (¹⁴⁷Sc), selenium (⁷⁵Se), strontium (⁸⁵Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (¹⁶⁹Yb, ¹⁷⁵Yb), yttrium (⁹⁰Y), zinc (⁶⁵Zn); positron emitting metals using various positron emission tomographies, and nonradioactive paramagnetic metal ions.

In other embodiments, cysteine engineered antibodies of the present invention are conjugated to a therapeutic agent such as a cytotoxin, *e.g.*, a cytostatic or cytocidal agent, a therapeutic agent or a radioactive metal ion, *e.g*., alpha-emitters. A cytotoxin or cytotoxic agent includes any agent that is detrimental to cells. Examples include paclitaxel, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, epirubicin, and cyclophosphamide and analogs or homologs thereof. Therapeutic agents include, but are not limited to, antimetabolites (*e.g.,* methotrexate, 6-mercaptopurine, 6-thioguanine, cytarabine, 5-fluorouracil decarbazine), alkylating agents (*e.g.,* mechlorethamine, thioepa chlorambucil, melphalan, carmustine (BCNU) and lomustine (CCNU), cyclothosphamide, busulfan, dibromomannitol, streptozotocin, mitomycin C, and cisdichlorodiamine platinum (II) (DDP) cisplatin), anthracyclines (*e.g.,* daunorubicin (formerly daunomycin) and doxorubicin), antibiotics *(e.g.,* dactinomycin (formerly actinomycin), bleomycin, mithramycin, and anthramycin (AMC)), and anti-mitotic agents (*e.g.,* vincristine and vinblastine).

In one embodiment, the cytotoxic agent is selected from the group consisting of an enediyne, a lexitropsin, a duocarmycin, a taxane, a puromycin, a dolastatin, a maytansinoid, and a vinca alkaloid. In other embodiments, the cytotoxic agent is paclitaxel, docetaxel, CC-1065, SN-38, topotecan, morpholino-doxorubicin, rhizoxin, cyanomorpholino-doxorubicin, dolastatin-10, echinomycin, combretastatin, calicheamicin, maytansine, DM-1, an auristatin or other dolastatin derivatives, such as auristatin E or auristatin F, AEB, AEVB, AEFP, MMAE (monomethylauristatin E), MMAF (monomethylauristatin F), eleutherobin or netropsin. The synthesis and structure of auristatin E, also known in the art as dolastatin-10, and its derivatives are described in U.S. Patent Application Publ. Nos. 2003/0083263 A1 and 2005/0009751 A1; in the International Patent Application No.: PCT/US02/13435, in U.S. Pat. Nos. 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414.

In other embodiments, the cytotoxic agent of an antibody conjugate of the invention is an anti-tubulin agent. Anti-tubulin agents are a well established class of cancer therapy compounds. Examples of anti-tubulin agents include, but are not limited to, taxanes (e.g., Taxol® (paclitaxel), docetaxel), T67 (Tularik), vincas, and auristatins (e.g., auristatin E, AEB, AEVB, MMAE, MMAF, AEFP). Antitubulin agents included in this class are also: vinca alkaloids, including vincristine and vinblastine, vindesine and vinorelbine; taxanes such as paclitaxel and docetaxel and baccatin derivatives, epithilone A and B, nocodazole, 5-Fluorouracil and colcimid, estramustine, cryptophysins, cemadotin, maytansinoids, combretastatins, dolastatins, discodermolide and eleutherobin In more specific embodiments, the cytotoxic agent is selected from the group consisting of a vinca alkaloid, a podophyllotoxin, a taxane, a baccatin derivative, a cryptophysin, a maytansinoid, a combretastatin, and a dolastatin. In more specific embodiments, the cytotoxic agent is vincristine, vinblastine, vindesine, vinorelbine, VP-16, camptothecin, paclitaxel, docetaxel, epithilone A, epithilone B, nocodazole, coichicine, colcimid, estramustine, cemadotin, discodermolide, maytansine, DM-1, an auristatin or other dolastatin derivatives, such as auristatin E or auristatin F, AEB, AEVB, AEFP, MMAE (monomethylauristatin E), MMAF (monomethylauristatin F), eleutherobin or netropsin.

In a specific embodiment, the drug is a maytansinoid, a group of anti-tubulin agents. In a more specific embodiment, the drug is maytansine. Further, in a specific embodiment, the cytotoxic or cytostatic agent is DM-1 (ImmunoGen, Inc.; see also Chari et al. 1992, Cancer Res 52:127-131). Maytansine, a natural product, inhibits tubulin polymerization resulting in a mitotic block and cell death. Thus, the mechanism of action of maytansine appears to be similar to that of vincristine and vinblastine. Maytansine, however, is about 200 to 1,000-fold more cytotoxic in vitro than these vinca alkaloids. In another specific embodiment, the drug is an AEFP.

In some embodiments, the antibodies may be conjugated to other small molecule or protein toxins, such as, but not limited to abrin, brucine, cicutoxin, diphtheria toxin, botulism toxin, shiga toxin, endotoxin, tetanus toxin, pertussis toxin, anthrax toxin, cholera toxin falcarinol, alfa toxin, geldanamycin, gelonin, lotaustralin, ricin, strychnine, and tetradotoxin.

Further examples of toxins, spacers, linkers, stretchers and the like, and their structures can be found in U.S. Patent Application Publication Nos. 2006/0074008 A1, 2005/0238649 A1, 2005/0123536 A1, 2005/0180972 A1, 2005/0113308 A1, 2004/0157782 A1, U.S. Patent No. 6,884,869 B2, U.S. Patent No. 5,635,483.

As discussed herein, the compounds used for conjugation to the antibody conjugates of the present invention can include conventional chemotherapeutics, such as doxorubicin, paclitaxel, carboplatin, melphalan, vinca alkaloids, methotrexate, mitomycin C, etoposide, and others. In addition, potent agents such CC-1065 analogues, calichiamicin, maytansine, analogues of dolastatin 10, rhizoxin, and palytoxin can be linked to the antibodies using the conditionally stable linkers to form potent immunoconjugates.

In certain embodiments, the cytotoxic or cytostatic agent is a dolastatin. In more specific embodiments, the dolastatin is of the auristatin class. In a specific embodiment of the invention, the cytotoxic or cytostatic agent is MMAE. In another specific embodiment of the invention, the cytotoxic or cytostatic agent is AEFP. In another specific embodiment of the invention, the cytotoxic or cytostatic agent is MMAF.

In other embodiments, antibodies of the present invention are conjugated to a therapeutic agent or drug moiety that modifies a given biological response. Therapeutic agents or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, *e.g.,* TNF-α, TNF-β, AIM I (see, International Publication No. WO 97/33899), AIM II (see, International Publication No. WO 97/34911), Fas Ligand (Takahashi et al., 1994, J. Immitnol., 6:1567), and VEGf (see, International Publication No. WO 99/23105), a thrombotic agent or an anti-angiogenic agent, *e.g.,* angiostatin or endostatin; or, a biological response modifier such as, for example, a lymphokine (*e*.*g*., interleukin-1 ("IL-1"), interleukin-2 ("IL-2"), interleukin-4 ("IL-4"), interleukin-6 ("IL-6"), interleukin-7 ("IL-7"), interleukin-9 ("IL-9"), interleukin-15 ("IL-15"), interleukin-12 ("IL-12"), granulocyte macrophage colony stimulating factor ("GM-CSF"), and granulocyte colony stimulating factor ("G-CSF")), or a growth factor *(e.g.,* growth hormone ("GH")).

In other embodiments, antibodies of the present invention are conjugated to a polypeptide that comprises poly arginine or poly-lysine residues. In some embodiments, said polypeptide comprises 5, 6, 7, 8, 9, 10, 11, 12, 13, 14, 15, or more amino acid residues. In some embodiments, the poly-arginine polypeptide may comprise at least 5, 6, 7, 8, 9, 10, 11, 12, 13, 14, 15, or more arginine residues. In other embodiments, the polylysine polypeptide polypeptide may comprise at least 5, 6, 7, 8, 9, 10, 11, 12, 13, 14, 15, or more lysine residues. In other embodiments, the polypeptide may comprise any combination of arginine and lysine residues.

In other embodiments, antibodies of the present invention are conjugated to a therapeutic agent such as a radioactive materials or macrocyclic chelators useful for conjugating radiometal ions (see above for examples of radioactive materials). In certain embodiments, the macrocyclic chelator is 1,4,7,10-tetraazacyclododecane-N,N',N",N"-tetraacetic acid (DOTA) which can be attached to the antibody via a linker molecule. Such linker molecules, further discussed herein below, are commonly known in the art and described in Denardo et al., 1998, Clin Cancer Res. 4:2483-90; Peterson et al., 1999, Bioconjug. Chem. 10:553; and Zimmerman et al., 1999, Nucl. Med. Biol. 26:943-.

In other embodiments, antibodies of the present invention are conjugated to a nucleic acid. The nucleic acid may be selected from the group consisting of DNA, RNA, short interfering RNA (siRNA), microRNA, hairpin or nucleic acid mimetics such as peptide nucleic acid. In some embodiments the conjugated nucleic acid is at least 10, at least 20, at least 30, at least 40, at least 50 , at least 60 at least 100, at least 200, at least 500, at least 1000, at least 5000 or more base pairs. In some embodiments, the conjugated nucleic acid is single stranded. In alternative embodiments, the conjugated nucleic acid is double stranded.

In some embodiments, the conjugated nucleic acid encodes an open reading frame. In some embodiments, the open reading frame encoded by the conjugated nucleic acid corresponds to a apoptosis inducing protein, a viral protein, an enzyme, or a tumor suppressor protein. Techniques for delivery of such nucleic acids to cells may be found at Song et al. Nature Biotechnology, 2005, Vol23:6 p709-717 and also US Patent No. 6,333,396.

Techniques for conjugating therapeutic moieties to antibodies are well known. Moieties can be conjugated to antibodies by any method known in the art, including, but not limited to aldehyde/Schiff linkage, sulphydryl linkage, acid-labile linkage, cis-aconityl linkage, hydrazone linkage, enzymatically degradable linkage (see generally Garnett, 2002, Adv. Drug Deliv. Rev. 53:171-216). Additional techniques for conjugating therapeutic moieties to antibodies are well known, see, *e.g.,* Arnon et al., "Monoclonal Antibodies For Immunotargeting Of Drugs In Cancer Therapy," in Monoclonal Antibodies And Cancer Therapy, Reisfeld et al. (eds.), pp. 243-56 (Alan R. Liss, Inc. 1985); Hellstrom et al., "Antibodies For Drug Delivery," in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review," in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy," in Monoclonal Antibodies For Cancer Detection And Therapy, Baldwin et al. (eds.), pp. 303-16 (Academic Press 1985), and Thorpe et al., 1982, Immunol. Rev. 62:119-58. Methods for fusing or conjugating antibodies to polypeptide moieties are known in the art. See, *e.g.,* U.S. Patent Nos. 5,336,603, 5,622,929, 5,359,046, 5,349,053, 5,447,851, and 5,112,946; EP 307,434; EP 367,166; International Publication Nos. WO 96/04388 and WO 91/06570; Ashkenazi et al., 1991, PNAS 88: 10535-10539; Zheng et al., 1995, J. Immunol. 154:5590-5600; and Vil et al., 1992, PNAS 89:11337- 11341. The fusion of an antibody to a moiety does not necessarily need to be direct, but may occur through linker sequences. Such linker molecules are commonly known in the art and described in Denardo et al., 1998, Clin Cancer Res. 4:2483-90; Peterson et al., 1999, Bioconjug. Chem. 10:553; Zimmerman et al., 1999, Nucl. Med. Biol. 26:943-50; Garnett, 2002, Adv. Drug Deliv. Rev. 53:171-216.

Two approaches may be taken to minimize drug activity outside the cells that are targeted by the antibody conjugates of the invention: first, an antibody that binds to cell membrane receptor but not soluble receptor may be used, so that the drug, including drug produced by the actions of the prodrug converting enzyme, is concentrated at the cell surface of the activated lymphocyte. Another approach for minimizing the activity of drugs bound to the antibodies of the invention is to conjugate the drugs in a manner that would reduce their activity unless they are hydrolyzed or cleaved off the antibody. Such methods would employ attaching the drug to the antibodies with linkers that are sensitive to the environment at the cell surface of the activated lymphocyte (e.g., the activity of a protease that is present at the cell surface of the activated lymphocyte) or to the environment inside the activated lymphocyte the conjugate encounters when it is taken up by the activated lymphocyte (e.g., in the endosomal or, for example by virtue of pH sensitivity or protease sensitivity, in the lysosomal environment). Examples of linkers that can be used in the present invention are disclosed in U.S. Patent Application Publication Nos. 2005/0123536 A1, 2005/0180972 A1, 2005/0113308 A1, 2004/0157782 A1, and U.S. Patent No. 6,884,869 B2.

In one embodiment, the linker is an acid-labile hydrazone or hydrazide group that is hydrolyzed in the lysosome (see, e.g., U.S. Pat. No. 5,622,929). In alternative embodiments, drugs can be appended to antibodies through other acid-labile linkers, such as cis-aconitic amides, orthoesters, acetals and ketals (Dubowchik and Walker, 1999, Pharm. Therapeutics 83:67-123; Neville et al., 1989, Biol. Chem. 264:14653-14661). Such linkers are relatively stable under neutral pH conditions, such as those in the blood, but are unstable at below pH 5, the approximate pH of the lysosome.

In other embodiments, drugs are attached to the antibodies of the invention using peptide spacers that are cleaved by intracellular proteases. Target enzymes include cathepsins B and D and plasmin, all of which are known to hydrolyze dipeptide drug derivatives resulting in the release of active drug inside target cells (Dubowchik and Walker, 1999, Pharm. Therapeutics 83:67-123). The advantage of using intracellular proteolytic drug release is that the drug is highly attenuated when conjugated and the serum stabilities of the conjugates can be extraordinarily high.

In yet other embodiments, the linker is a malonate linker (Johnson et al., 1995. Anticancer Res. 15:1387-93), a maleimidobeiizoyl linker (Lau et al., 1995, Bioorg-Med-Chem. 3(10):1299-1304), or a 3'-N-amide analog (Lau et al, 1995, Bioorg-Med-Chem. 3(103:1305-12).

As discussed above, antibody conjugates are generally made by conjugating a compound or a drug to an antibody through a linker. Any linker that is known in the art may be used in the conjugates of the present invention, e.g., bifunctional agents (such as dialdehydes or imidoesters) or branched hydrazone linkers (see, e.g., U.S. Pat. No. 5,824,805,).

In certain, non-limiting, embodiments of the invention, the linker region between the conjugate moiety and the antibody moiety is cleavable under certain conditions, wherein cleavage or hydrolysis of the linker releases the drug moiety from the antibody moiety. In some embodiments, the linker is sensitive to cleavage or hydrolysis under intracellular conditions.

In one embodiment, the linker region between the conjugate moiety and the antibody moiety is cleavable if the pH changes by a certain value or exceeds a certain value. In another embodiment of the invention, the linker is cleavable in the milieu of the lysosome, e.g., under acidic conditions (i.e., a pH of around 5-5.5 or less). In other embodiments, the linker is a peptidyl linker that is cleaved by a peptidase or protease enzyme, including but not limited to a lysosomal protease enzyme, a membrane-associated protease, an intracellular protease, or an endosomal protease. Typically, the linker is at least two amino acids long, more typically at least three amino acids long. For example, a peptidyl linker that is cleavable by cathepsin-B (e.g., a Gly-Phe-Leu-Gly linker), a thiol-dependent protease that is highly expressed in cancerous tissue, can be used. Other such linkers are described, e.g., in U.S. Pat. No. 6,214,345.

In other, non-mutually exclusive embodiments of the invention, the linker by which the antibody and compound of an antibody conjugate of the invention are conjugated promotes cellular internalization. In certain embodiments, the linker-drug moiety promotes cellular internalization. In certain embodiments, the linker is chosen such that the structure of the entire antibody conjugate promotes cellular internalization. In one embodiment, the linker is a thioether linker (see, e.g., U.S. Pat. No. 5,622,929 to Willner et al., which is incorporated by reference herein in its entirety). In another embodiment, the linker is a hydrazone linker (see, e.g., U.S. Pat. Nos. 5,122,368 to Greenfield et al. and 5,824,805 to King et al).

In yet other embodiments, the linker is a disulfide linker. A variety of disulfide linkers are known in the art, including but not limited to those that can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alpha-methyl-alpha-(2-pyridyl-dithio)tol-uene). SPDB and SMPT (see, e.g., Thorpe et al., 1987, Cancer Res., 47:5924-5931; Wawrzynczak et al., 1987, In Immunoconjugates: Antibody Conjugates in Radioimagery and Therapy of Cancer, ed. C. W. Vogel, Oxford U. Press, pp. 28-55; see also U.S. Pat. No. 4,880,935 to Thorpe et al.).

A variety of linkers that can be used with the compositions and methods of the present invention are described in U.S. Patent Application Publication No. US 2004/0018194 A1.

In yet other embodiments of the present invention, the linker unit of an antibody conjugate links the cytotoxic or cytostatic agent (drug unit; -D) and the antibody unit (-A). In certain embodiments, the linker unit has the general formula:
i. -Ta-Ww-Yy- wherein:
ii. -T- is a stretcher unit;
iii. a is 0 or 1;
iv. each -W- is independently an amino acid unit;
v. w is independently an integer ranging from 2 to 12;
vi. -Y- is a spacer unit; and
vii. y is 0, 1 or 2.

The stretcher unit (-T-), when present, links the antibody unit to an amino acid unit (--W--). Useful functional groups that can be present on an antibody, either naturally or via chemical manipulation include, but are not limited to, sulfhydryl, amino, hydroxyl, the anomeric hydroxyl group of a carbohydrate, and carboxyl. Cysteine engineered antibodies of the invention present at least one free sulfhydryl groups for conjugation. Other methods of introducing free sulfhydryl groups involve the reduction of the intramolecular disulfide bonds of an antibody. Alternatively, sulfhydryl groups can be generated by reaction of an amino group of a lysine moiety of an antibody with 2-iminothiolane (Traut's reagent) or other sulfhydryl generating reagents.

The amino acid unit (--W--) links the stretcher unit (-T-) to the Spacer unit (--Y--) if the Spacer unit is present, and links the stretcher unit to the cytotoxic or cytostatic agent (Drug unit; D) if the spacer unit is absent.

In some embodiments, -W_{w}- is a dipeptide, tripeptide, tetrapeptide, pentapeptide, hexapeptide, heptapeptide, octapeptide, nonapeptide, decapeptide, undecapeptide or dodecapeptide unit. The amino acid unit of the linker unit can be enzymatically cleaved by an enzyme including, but not limited to, a tumor-associated protease to liberate the drug unit (-D) which is protonated in vivo upon release to provide a cytotoxic drug (D).

In a one embodiment, the amino acid unit is a phenylalanine-lysine dipeptide (phe-lys or FK linker). In another embodiment, the amino acid unit is a valine-citrulline dipeptide (val-cit or VC linker).

The spacer unit (--Y--), when present, links an amino acid unit to the drug unit. Spacer units are of two general types: self-immolative and non self-immolative. A non self-immolative spacer unit is one in which part or all of the spacer unit remains bound to the drug unit after enzymatic cleavage of an amino acid unit from the antibody-linker-drug conjugate or the drug-linker compound. Examples of a non self-immolative spacer unit include, but are not limited to a (glycine-glycine) spacer unit and a glycine spacer unit. When an antibody-linker-drug conjugate of the invention containing a glycine-glycine spacer unit or a glycine spacer unit undergoes enzymatic cleavage via a tumor-cell associated-protease, a cancer-cell-associated protease or a lymphocyte-associated protease, a glycine-glycine-drug moiety or a glycine-drug moiety is cleaved from A-T-W_{w}--. To liberate the drug, an independent hydrolysis reaction should take place within the target cell to cleave the glycine-drug unit bond.

Other examples of self-immolative spacers include, but are not limited to, aromatic compounds that are electronically equivalent to the PAB group such a 2-aminoimidazol-5-methanol derivatives (see Hay et al., Bioorg. Med. Chem. Lett., 1999, 9, 2237 for examples) and ortho or para-aminobenzylacetals. Spacers can be used that undergo facile cyclization upon amide bond hydrolysis, such as substituted and unsubstituted 4-aminobutyric acid amides (Rodrigues et al., Chemistry, Biology, 1995, 2, 223), appropriately substituted ring systems (Storm, et al., J. Amer. Chem. Soc., 1972, 94, 5815) and 2-aminophenylpropionic acid amides (Amsberry, et al., J. Org. Chem., 1990, 55, 5867). Elimination of amine-containing drugs that are substituted at the α-position of glycine (Kingsbury, et al., J. Med. Chem., 1984, 27, 1447) are also examples of self-immolative spacer strategies that can be applied to the antibody-linker-drug conjugates of the invention.

### Methods of conjugating a heterologus molecule to an antibody

Heterologus molecules, such as those described herein may be efficiently conjugated to antibodies of the invention through the free thiol groups the engineered cysteine residues provide. In one aspect, the invention provides methods for efficiently conjugating heterologus molecules to cysteine engineered antibodies. In one embodiment, the conjugation of a heterologus molecule may occur at a free thiol group provided by at least one engineered cysteine residue selected from the positions 131, 132, 133, 134, 135, 136, 137, 138, and 139 of the CH1 domain of an antibody. In other embodiments, the method of the invention comprises the efficient conjugation of a heterologus molecule at a free thiol group provided by at least one, at least two, at least three, at least four, at least five, at least six, at least seven, or at least eight engineered cysteine residues selected from the positions 131, 132, 133, 134, 135, 136, 137, 138, and 139 of the CH1 domain of an antibody.

The engineering of non-naturally occurring cysteine residues into antibodies may alter the disulfide pairing of the heavy and light chains such that a naturally occurring cysteine residue which was part of a disulfide bond is liberated and presents a free thiol group capable of conjugation. In another embodiment, the method comprises the efficient conjugation of a heterologus molecule to a cysteine engineered antibody at a free thiol group not provided by at least one engineered cysteine residue selected from the positions 131, 132, 133, 134, 135, 136, 137, 138, and 139 of the CH1 domain of an antibody.

The presence of free thiol groups in antibodies may be determined by various art accepted techniques, such as those described in Example 1. The efficiency of conjugation of a heterologus molecule to an antibody may be determined by assessing the presence of free thiols remaining after the conjugation reaction. In one embodiment, the invention provides a method of efficiently conjugating a heterologus molecule to a cysteine engineered antibody. In one embodiment, the conjugation efficiency is at least 5%, at least 10%, at least 15%, at least 20%, at least 25%, at least 30%, at least 35%, at least 40%, at least 45%, at least 50%, at least 55%, at least 60%, at least 70%, at least 75%, at least 80%, at least 85%, at least 90%, at least 95% , at least 98% or more as measured by the level of free thiol groups remaining after the conjugation reaction.

In another embodiment, the invention provides a method of conjugating a heterologus molecule to an antibody wherein the antibody comprises at least one amino acid substitution, such that 2 or more free thiol groups are formed. In another embodiment, the method comprises an antibody wherein the antibody comprises at least one amino acid substitution , such that at least 2, at least 4, at least 6, at least 8, at least 10, at least 12, at least 14, at least 16 or more free thiol groups are formed.

Antibodies of the invention capable of conjugation may contain free cysteine residues that comprise sulfhydryl groups that are blocked or capped. Such caps include proteins, peptides, ions and other materials that interact with the sulfhydryl group and prevent or inhibit conjugate formation, In some embodiments, antibodies of the invention may require uncapping prior to a conjugation reaction. In specific embodiments, antibodies of the invention are uncapped and display a free sulfhydryl group capable of conjugation, In other specific embodiments, antibodies of the invention are subjected to an uncapping reaction that does not disturb or rearrange the naturally occurring disulfide bonds, in other embodiments, antibodies of the invention are subjected to an uncapping reaction as presented in Examples 9 or 10,

In some embodiments, antibodies of the invention may be subjected to conjugation reactions wherein the antibody to be conjugated is present at a concentration of at least 1 mg/ml, at least 2 mg/ml, at least 3 mg/ml, at least 4 mg.ml, at least 5 mg/ml or higher.

### Methods of using antibody conjugates

It is contemplated that the antibody conjugates of the present invention may be used to treat various diseases or disorders, e.g. chacterized by the overexpression of a tumor antigen. Exemplary conditions or hyperproliferative disorders include benign or malignant tumors, leukemia and lymphoid malignancies. Others include neuronal, glial, astrocytal, hypothalamic, glandular, macrophagal, epithelial, endothelial, and stromal malignancies. Other cancers or hyperproliferative disorders include: ancers of the head, neck, eye, mouth, throat, esophagus, chest, skin, bone, lung, colon, rectum, colorectal, stomach, spleen, kidney, skeletal muscle, subcutaneous tissue, metastatic melanoma, endometrial, prostate, breast, ovaries, testicles, thyroid, blood, lymph nodes, kidney, liver, pancreas, brain, or central nervous system. Examples of cancers that can be prevented, managed, treated or ameliorated in accordance with the methods of the invention include, but are not limited to, cancer of the head, neck, eye, mouth, throat, esophagus, chest, bone, lung, colon, rectum, stomach, prostate, breast, ovaries, kidney, liver, pancreas, and brain. Additional cancers include, but are not limited to, the following: leukemias such as but not limited to, acute leukemia, acute lymphocytic leukemia, acute myelocytic leukemias such as myeloblastic, promyelocytic, myelomonocytic, monocytic, erythroleukemia leukemias and myelodysplastic syndrome, chronic leukemias such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple mycloma, nonsecretory myeloma, osteosclerotic myeloma, plasma cell leukemia, solitary plasmacytoma and extramedullary plasmacytoma; Waldenstrom's macroglobulinemia; monoclonal gammopathy of undetermined significance; benign monoclonal gammopathy; heavy chain disease; bone cancer and connective tissue sarcomas such as but not limited to bone sarcoma, myeloma bone disease, multiple myeloma, cholesteatoma-induced bone osteosarcoma, Paget's disease of bone, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, and synovial sarcoma; brain tumors such as but not limited to, glioma, astrocytoma, brain stem glioma, ependymoma, oligodendroglioma, non-glial tumor, acoustic neurinoma, craniopharyngioma, medulloblastoma, meningioma, pineocytoma, pineoblastoma, and primary brain lymphoma; breast cancer including but not limited to adenocarcinoma, lobular (small cell) carcinoma, intraductal carcinoma, medullary breast cancer, mucinous breast cancer, tubular breast cancer, papillary breast cancer, Paget's disease (including juvenile Paget's disease) and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cyctic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma, gallbladder cancers such as adenocarcinoma; cholangiocarcinomas such as but not limited to pappillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell cancer, adenocarcinoma, hypernephroma, fibrosarcoma, transitional cell cancer (renal pelvis and/or ureter); Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesotheliorna, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, see Fishman et al., 1985, Medicine, 2d Ed., J. B. Lippincott Co., Philadelphia and Murphy et al., 1997, Informed Decisions: The Complete Book of Cancer Diagnosis, Treatment, and Recovery, Viking Penguin, Penguin Books U.S.A., inc., United States of America). It is also contemplated that cancers caused by aberrations in apoptosis can also be treated by the methods and compositions of the invention. Such cancers may include, but not be limited to, follicular lymphomas, carcinomas with p53 mutations, hormone dependent tumors of the breast, prostate and ovary, and precancerous lesions such as familial adenomatous polyposis, and myelodysplastic syndromes.

The proteins of the invention and compositions comprising the same are useful for many purposes, for example, as therapeutics against a wide range of chronic and acute diseases and disorders including, but not limited to, autoimmune and/or inflammatory disorders, which include Sjogren's syndrome, rheumatoid arthritis, lupus psoriasis, atherosclerosis, diabetic and other retinopathies, retrolental fibroplasia, age-related macular degeneration, neovascular glaucoma, hemangiomas, thyroid hyperplasias (including Grave's disease), corneal and other tissue transplantation, and chronic inflammation, sepsis, rheumatoid arthritis, peritonitis, Crohn's disease, reperfusion injury, septicemia, endotoxic shock, cystic fibrosis, endocarditis, psoriasis, arthritis (*e.g.*, psoriatic arthritis), anaphylactic shock, organ ischemia, reperfusion injury, spinal cord injury and allograft rejection. Other Examples of autoimmune and/or inflammatory disorders include, but are not limited to, alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, Sjogren's syndrome, psoriasis, atherosclerosis, diabetic and other retinopathies, retrolental fibroplasia, age-related macular degeneration, neovascular glaucoma, hemangiomas, thyroid hyperplasias (including Grave's disease), corneal and other tissue transplantation, and chronic inflammation, sepsis, rheumatoid arthritis, peritonitis, Crohn's disease, reperfusion injury, septicemia, endotoxic shock, cystic fibrosis, endocarditis, psoriasis, arthritis (*e.g.*, psoriatic arthritis), anaphylactic shock, organ ischemia, reperfusion injury, spinal cord injury and allograft rejection. autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erythematosus, Meniere's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, Sjogren's syndrome, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, and Wegener's granulomatosis. Examples of inflammatory disorders include, but are not limited to, asthma, encephilitis, inflammatory bowel disease, chronic obstructive pulmonary disease (COPD), allergic disorders, septic shock, pulmonary fibrosis, undifferentitated spondyloarthropathy, undifferentiated arthropathy, arthritis, inflammatory osteolysis, and chronic inflammation resulting from chronic viral or bacteria infections. The compositions and methods of the invention can be used with one or more conventional therapies that are used to prevent, manage or treat the above diseases.

The invention also provides methods of using antibodies and/or antibody conjugates to inactivate various infectious agents such as viruses, fungi, eukaryotic microbes, and bacteria. In some embodiments the antibodies or antibody conjugates of the invention may be used to inactivate RSV, hMPV, PIV, or influenza viruses. In other embodiments, the antibodies and/or antibody conjugates of the invention may be used to inactivate fungal pathogens, such as, but not limited to members of *Naegleria*, *Aspergillus*, *Blastomyces, Histoplasma*, *Candida* or *Tinea* genera. In other embodiments, the antibodies and/or antibody conjugates of the invention may be used to inactivate eukaryotic microbes, such as, but not limited to members of *Giardia*, *Toxoplasma*, *Plasmodium*, *Trypanosoma*, and *Entamoeba* genera. In other embodiments, the antibodies and/or antibody conjugates of the invention may be used to inactivate bacterial pathogens, such as but not limited to members of *Staphylococcus*, *Streptococcus*, *Pseudomonas*, *Clostridium*, *Borrelia, Vibro* and *Neiserria* genera.

The antibodies and/or antibody conjugates of the invention and compositions comprising the same are useful for many purposes, for example, as therapeutics against a wide range of chronic and acute diseases and disorders including, but not limited to, infectious disease, including viral, bacterial and fungal diseases. Examples of viral pathogens include but are not limited to: adenovirdiae (*e.g*., mastadenovirus and aviadenovirus), herpesviridae (*e.g.*, herpes simplex virus 1, herpes simplex virus 2, herpes simplex virus 5, and herpes simplex virus 6), leviviridae (*e.g*., levivirus, enterobacteria phase MS2, allolevirus), poxviridae (*e.g*., chordopoxvirinae, parapoxvirus, avipoxvirus, capripoxvirus, leporiipoxvirus, suipoxvirus, molluscipoxvirus, and entomopoxvirinae), papovaviridae (*e.g.,* polyomavirus and papillomavirus), paramyxoviridae *(e.g.,* paramyxovirus, parainfluenza virus 1, mobillivirus *(e.g.,* measles virus), rubulavirus *(e.g.,* mumps virus), pneumonovirinae (*e.g.*, pneumovirus, human respiratory synctial virus), and metapneumovirus (*e.g.*, avian pneumovirus and human metapneumovirus)), picornaviridae *(e.g.,* enterovirus, rhinovirus, hepatovirus (*e.g.,* human hepatitis A virus), cardiovirus, and apthovirus), reoviridae (*e.g.*, orthoreovirus, orbivirus, rotavirus, cypovirus, fijivirus, phytoreovirus, and oryzavirus), retroviridae (*e.g.*, mammalian type B retroviruses, mammalian type C retroviruses, avian type C retroviruses, type D retrovirus group, BLV-HTLV retroviruses, lentivirus (*e.g.* human immunodeficiency virus 1 and human immunodeficiency virus 2), spumavirus), flaviviridae (*e.g*., hepatitis C virus), hepadnaviridae *(e.g.,* hepatitis B virus), togaviridae (*e.g.,* alphavirus (*e.g.,* sindbis virus) and rubivirus *(e.g.,* rubella virus)), rhabdoviridae (*e.g.,* vesiculovirus, lyssavirus, ephemerovirus, cytorhabdovirus, and necleorhabdovirus), arenaviridae (*e.g*., arenavirus, lymphocytic choriomeningitis virus, Ippy virus, and lassa virus), and coronaviridae (*e.g*., coronavirus and torovirus). Examples of bacterial pathogens include but are not limited to: but not limited to, the *Aquaspirillum* family, *Azospirillum* family, *Azotobacteraceae* family, *Bacteroidaceae* family, *Bartonella* species, *Bdellovibrio* family, *Campylobacter* species, *Chlamydia* species *(e.g., Chlamydia pneumoniae*), clostridium, *Enterobacteriaceae* family (*e.g., Citrobacter* species, Edwardsiella, *Enterobacter aerogenes*, *Erwinia* species, *Escherichia coli,* Hafnia species, *Klebsiella* species, Morganella species, *Proteus vulgaris*, Providencia, *Salmonella* species, *Serratia marcescens,* and *Shigella flexneri*), *Gardinella* family, *Haemophilus influenzae*, *Halobacteriaceae* family, *Helicobacter* family, *Legionallaceae* family, *Listeria* species, *Methylococcaceae* family, mycobacteria (*e*.*g*., *Mycobacterium tuberculosis*), *Neisseriaceae* family, *Oceanospirillum* family, *Pasteurellaceae* family, *Pneumococcus* species, *Pseudomonas* species, *Rhizobiaceae* family, *Spirillum* family, *Spirosomaceae* family, *Staphylococcuss (e.g.,* methicillin resistant *Staphylococcus aureus* and *Staphylococcus pyrogenes*), *Streptococcus* (*e*.*g*., *Streptococcus enteritidis, Streptococcus fasciae,* and *Streptococcus pneumoniae*), *Vampirovibr Helicobacter* family, and *Vampirovibrio* family. Examples of fungal pathogens include, but are not limited to: *Absidia* species (*e.g., Absidia corymbifera and Absidia ramosa*), *Aspergillus* species, (*e.g., Aspergillus flavus, Aspergillus fumigatus, Aspergillus nidulans, Aspergillus niger,* and *Aspergillus terreus*), *Basidiobolus ranarum, Blastomyces dermatitidis,Candida* species (*e.g., Candida albicans, Candida glabrata, Candida kerr, Candida krusei, Candida parapsilosis, Candida pseudotropicalis, Candida quillermondii, Candida rugosa, Candida stellatoidea,* and *Candida tropicalis), Coccidioides immitis, Conidiobolus* species, *Cryptococcus neoforms, Cunninghamella* species, dermatophytes, *Histoplasma capsulatum, Microsporum gypseum*, *Mucor pusillus, Paracoccidioides brasiliensis, Pseudallescheria boydii, Rhinosporidium seeberi, Pneumocystis carinii, Rhizopus species* (*e.g., Rhizopus arrhizus, Rhizopus oryzae, and Rhizopus microsporus*), *Saccharomyces* species, *Sporothrix schenckii,* zygomycetes, and classes such as *Zygomycetes, Ascomycetes,* the *Basidiomycetes, Deuteromycetes,* and *Oomycetes.*

The invention also provides methods of using antibodies to deplete a cell population. In one embodiment, methods of the invention are useful in the depletion of the following cell types: eosinophil, basophil, neutrophil, T cell, B cell, mast cell, monocytes, endothelial cell and tumor cell.

The antibodies of the invention and conjugates thereof may also be useful in the diagnosis and detection of diseases of symptoms thereof. In another embodiment, the compositions of the invention may be useful in the monitoring of disease progression. In another embodiment, the compositions of the invention may be useful in the monitoring of treatment regimens. In another embodiment, the compositions of the invention are useful for diagnosis in an *ex vivo* application, such as a diagnostic kit.

The compositions of the invention may be useful in the visualization of target antigens. In some embodiments, the target antigens are cell surface receptors that internalize. In other embodiments, the target antigen is an intracellular antigen. In other embodiments the target is an intranuclear antigen.

In one embodiment, the antibodies or antibody-drug conjugates of the invention once bound, internalize into cells wherein internalization is at least about 10%, at least about 20%, at least about 30%, at least about 40%, at least about 50%, at least about 60%, at least about 70%, at least about 80%, or at least about 90%, at least about 100%, at least about 110%, at least about 130%, at least about 140%, at least about 150%, at least about 160%, or at least about 170% more than control antibodies as described herein.

In another embodiment, the antibodies of the invention once bound, internalize into cells wherein internalization is 1-10%, 10-20%, 20 - 30%, 30- 40%, 40- 50%, 50-60%, 60-70%, 70-80%, 80-90%, 90-100%, 100-110%, 110-120%, 120-130%, 130-140%, 140-150%, 150-160%, 160-170% more than control antibodies as described herein.

In another embodiment, the antibodies of the invention once bound, internalize into cells wherein internalization is 1-10%, 10-20%, 20 - 30%, 30- 40%,40- 50%, 50-60%, 60-70%, 70-80%, 80-90%, 90-100%, 100-110%, 110-120%, 120-130%, 130-140%, 140-150%, 150-160%, 160-170% more than control antibodies as determined by the internalization assay using a secondary antibody.

### Pharmaceutical Compositions

In another aspect, the present invention provides a composition, for example, but not limited to, a pharmaceutical composition, containing one or a combination of antibodies, or antibody conjugates of the present invention, formulated together with a pharmaceutically acceptable carrier. Such compositions may include one or a combination of, for example, but not limited to two or more different antibodies of the invention. For example, a pharmaceutical composition of the invention may comprise a combination of antibodies that bind to different epitopes on the target antigen or that have complementary activities.

Pharmaceutical compositions of the invention also can be administered in combination therapy, such as, combined with other agents. For example, the combination therapy can include an antibody of the present invention combined with at least one other therapy wherein the therapy may be surgery, immunotherapy, chemotherapy, radiation treatment, or drug therapy.

The pharmaceutical compounds of the invention may include one or more pharmaceutically acceptable salts. Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition of the invention also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of suitable aqueous and non-aqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutical compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be suitable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

In one embodiment the compositions of the invention are pyrogen-free formulations which are substantially free of endotoxins and/or related pyrogenic substances. Endotoxins include toxins that are confined inside a microorganism and are released when the microorganisms are broken down or die. Pyrogenic substances also include fever-inducing, thermostable substances (glycoproteins) from the outer membrane of bacteria and other microorganisms. Both of these substances can cause fever, hypotension and shock if administered to humans. Due to the potential harmful effects, it is advantageous to remove even low amounts of endotoxins from intravenously administered pharmaceutical drug solutions. The Food & Drug Administration ("FDA") has set an upper limit of 5 endotoxin units (EU) per dose per kilogram body weight in a single one hour period for intravenous drug applications (The United States Pharmacopeial Convention, Pharmacopeial Forum 26 (1):223 (2000)). When therapeutic proteins are administered in amounts of several hundred or thousand milligrams per kilogram body weight it is advantageous to remove even trace amounts of endotoxin. In one embodiment, endotoxin and pyrogen levels in the composition are less then 10 EU/mg, or less then 5 EU/mg, or less then 1 EU/mg, or less then 0.1 EU/mg, or less then 0.01 EU/mg, or less then 0.001 EU/mg. In another embodiment, endotoxin and pyrogen levels in the composition are less then about 10 EU/mg, or less then about 5 EU/mg, or less then about 1 EU/mg, or less then about 0.1 EU/mg, or less then about 0.01 EU/mg, or less then about 0.001 EU/mg.

In one embodiment, the invention comprises administering a composition wherein said administration is oral, parenteral, intramuscular, intranasal, vaginal, rectal, lingual, sublingual, buccal, intrabuccal, intravenous, cutaneous, subcutaneous or transdermal.

In another embodiment the invention further comprises administering a composition in combination with other therapies, such as surgery, chemotherapy, hormonal therapy, biological therapy, immunotherapy or radiation therapy.

### Dosing/Administration

To prepare pharmaceutical or sterile compositions including an antibody or antibody conjugate of the invention, the antibody/antibody conjugate is mixed with a pharmaceutically acceptable carrier or excipient. Formulations of therapeutic and diagnostic agents can be prepared by mixing with physiologically acceptable carriers, excipients, or stabilizers in the form of, e.g., lyophilized powders, slurries, aqueous solutions, lotions, or suspensions (see, e.g., Hardman, et al. (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y.).

Selecting an administration regimen for a therapeutic depends on several factors, including the serum or tissue turnover rate of the entity, the level of symptoms, the immunogenicity of the entity, and the accessibility of the target cells in the biological matrix. In certain embodiments, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate doses of antibodies, cytokines, and small molecules are available (see, e.g., Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, N.Y.; Bach (ed.) (1993) Monoclonal Antibodies and Peptide Therapy in Autoimmune Diseases, Marcel Dekker, New York, N.Y.; Baert, et al. (2003) New Engl. J. Med. 348:601-608; Milgrom, et al. (1999) New Engl. J. Med. 341:1966-1973; Slamon, et al. (2001) New Engl. J. Med. 344:783-792; Beniaminovitz, et al. (2000) New Engl. J. Med. 342:613-619; Ghosh, et al. (2003) New Engl. J. Med. 348:24-32; Lipsky, et al. (2000) New Engl. J. Med. 343:1594-1602).

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

Compositions comprising antibodies or antibody conjugates of the invention can be provided by continuous infusion, or by doses at intervals of, e.g., one day, one week, or 1-7 times per week. Doses may be provided intravenously, subcutaneously, topically, orally, nasally, rectally, intramuscular, intracerebrally, or by inhalation. A specific dose protocol is one involving the maximal dose or dose frequency that avoids significant undesirable side effects. A total weekly dose may be at least 0.05 µg/kg body weight, at least 0.2 µg/kg, at least 0.5 µg/kg, at least 1 µg/kg, at least 10 µg/kg, at least 100 µg/kg, at least 0.2 mg/kg, at least 1.0 mg/kg, at least 2.0 mg/kg, at least 10 mg/kg, at least 25 mg/kg, or at least 50 mg/kg (see, e.g., Yang, et al. (2003) New Engl. J. Med. 349:427-434; Herold, et al. (2002) New Engl. J. Med. 346:1692-1698; Liu, et al. (1999) J. Neurol. Neurosurg. Psych. 67:451-456; Portielji, et al. (20003) Cancer Immunol. Immunother. 52:133-144).. The dose may be at least 15 µg, at least 20 µg, at least 25 µg, at least 30 µg, at least 35 µg, at least 40 µg, at least 45 µg, at least 50 µg, at least 55 µg, at least 60 µg, at least 65 µg, at least 70 µg, at least 75 µg, at least 80 µg, at least 85 µg, at least 90 µg, at least 95 µg, or at least 100 µg. The doses administered to a subject may number at least 1, 2, 3, 4, 5, 6, 7, 8, 9, 10, 11, or 12, or more.

For antibodies or antibody conjugates of the invention, the dosage administered to a patient may be 0.0001 mg/kg to 100 mg/kg of the patient's body weight. The dosage may be between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight.

The dosage of the antibodies or antibody conjugates of the invention may be calculated using the patient's weight in kilograms (kg) multiplied by the dose to be administered in mg/kg. The dosage of the antibodies of the invention may be 150 µg/kg or less, 125 µg/kg or less, 100 µg/kg or less, 95 µg/kg or less, 90 µg/kg or less, 85 µg/kg or less, 80 µg/kg or less, 75 µg/kg or less, 70 µg/kg or less, 65 µg/kg or less, 60 µg/kg or less, 55 µg/kg or less, 50 µg/kg or less, 45 µg/kg or less, 40 µg/kg or less, 35 µg/kg or less, 30 µg/kg or less, 25 µg/kg or less, 20 µg/kg or less, 15 µg/kg or less, 10 µg/kg or less, 5 µg/kg or less, 2.5 µg/kg or less, 2 µg/kg or less, 1.5 µg/kg or less, 1 µg/kg or less, 0.5 µg/kg or less, or 0.5 µg/kg or less of a patient's body weight.

Unit dose of the antibodies or antibody conjugates of the invention may be 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 m g, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

The dosage of the antibodies or antibody conjugates of the invention may achieve a serum titer of at least 0.1 µg/ml, at least 0.5 µg/ml, at least 1 µg/ml, at least 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in a subject. Alternatively, the dosage of the antibodies of the invention may achieve a serum titer of at least 0.1 µLg/ml, at least 0.5 µg/ml, at least 1 µg/ml, at least, 2 µg/ml, at least 5 µg/ml, at least 6 µg/ml, at least 10 µg/ml, at least 15 µg/ml, at least 20 µg/ml, at least 25 µg/ml, at least 50 µg/ml, at least 100 µg/ml, at least 125 µg/ml, at least 150 µg/ml, at least 175 µg/ml, at least 200 µg/ml, at least 225 µg/ml, at least 250 µg/ml, at least 275 µg/ml, at least 300 µg/ml, at least 325 µg/ml, at least 350 µg/ml, at least 375 µg/ml, or at least 400 µg/ml in the subject.

Doses of antibodies or antibody conjugates of the invention may be repeated and the administrations may be separated by at least 1 day, 2 days, 3 days, 5 days, 10 days, 15 days, 30 days, 45 days, 2 months, 75 days, 3 months, or at least 6 months.

An effective amount for a particular patient may vary depending on factors such as the condition being treated, the overall health of the patient, the method route and dose of administration and the severity of side affects (see, e.g., Maynard, et al. (1996) A Handbook of SOPs for Good Clinical Practice, Interpharm Press, Boca Raton, Fla.; Dent (2001) Good Laboratory and Good Clinical Practice, Urch Publ., London, UK).

The route of administration may be by, e.g., topical or cutaneous application, injection or infusion by intravenous, intraperitoneal, intracerebral, intramuscular, intraocular, intraarterial, intracerebrospinal, intralesional, or by sustained release systems or an implant (see, e.g., Sidman et al. (1983) Biopolymers 22:547-556; Langer, et al. (1981) J. Biomed. Mater. Res. 15:167-277; Langer (1982) Chem. Tech. 12:98-105; Epstein, et al. (1985) Proc. Natl. Acad. Sci. USA 82:3688-3692; Hwang, et al. (1980) Proc. Natl. Acad. Sci. USA 77:4030-4034; U.S. Pat. Nos. 6,350466 and 6,316,024). Where necessary, the composition may also include a solubilizing agent and a local anesthetic such as lidocaine to ease pain at the site of the injection. In addition, pulmonary administration can also be employed, *e.g.,* by use of an inhaler or nebulizer, and formulation with an aerosolizing agent. See, *e.g.,* U.S. Pat. Nos. 6,019,968, 5,985, 320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903. In one embodiment, an antibody, combination therapy, or a composition of the invention is administered using Alkermes AIR™ pulmonary drug delivery technology (Alkermes, Inc., Cambridge, Mass.).

A composition of the present invention may also be administered via one or more routes of administration using one or more of a variety of methods known in the art. As will be appreciated by the skilled artisan, the route and/or mode of administration will vary depending upon the desired results. Selected routes of administration for antibodies of the invention include intravenous, intramuscular, intradermal, intraperitoneal, subcutaneous, spinal or other parenteral routes of administration, for example by injection or infusion. Parenteral administration may represent modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, a composition of the invention can be administered via a non-parenteral route, such as a topical, epidermal or mucosal route of administration, for example, intranasally, orally, vaginally, rectally, sublingually or topically.

If the antibodies of the invention or conjugates thereof are administered in a controlled release or sustained release system, a pump may be used to achieve controlled or sustained release (see Langer, supra; Sefton, 1987, CRC Crit. Ref. Biomed. Eng. 14:20*;* Buchwald et al., 1980, Surgery 88:507; Saudek et al., 1989, N. Engl. J. Med. 321:574). Polymeric materials can be used to achieve controlled or sustained release of the therapies of the invention (see *e.g.,* Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Pat. No. 5,679,377; U.S. Pat. No. 5,916,597; U.S. Pat. No. 5,912,015; U.S. Pat. No. 5,989,463; U.S. Pat. No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In one embodiment, the polymer used in a sustained release formulation is inert, free of leachable impurities, stable on storage, sterile, and biodegradable. A controlled or sustained release system can be placed in proximity of the prophylactic or therapeutic target, thus requiring only a fraction of the systemic dose (see, *e.g.,* Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more antibodies of the invention or conjugates thereof. See, *e*.*g*., U.S. Pat. No. 4,526,938, PCT publication WO 91/05548, PCT publication WO 96/20698, Ning et al., 1996, "Intratumoral Radioimmunotheraphy of a Human Colon Cancer Xenograft Using a Sustained-Release Gel," Radiotherapy & Oncology 39:179-189, Song et al., 1995, "Antibody Mediated Lung Targeting of Long-Circulating Emulsions," PDA Journal of Pharmaceutical Science & Technology 50:372-397, Cleek et al., 1997, "Biodegradable Polymeric Carriers for a bFGF Antibody for Cardiovascular Application," Pro. Int'l. Symp. Control. Rel. Bioact. Mater. 24:853-854, and Lam et al., 1997, "Microencapsulation of Recombinant Humanized Monoclonal Antibody for Local Delivery," Proc. Int'l. Symp. Control Rel. Bioact. Mater. 24:759-760.

If the antibody or antibody conjugate of the invention is administered topically, it can be formulated in the form of an ointment, cream, transdermal patch, lotion, gel, shampoo, spray, aerosol, solution, emulsion, or other form well-known to one of skill in the art. See, *e.g.*, Remington's Pharmaceutical Sciences and Introduction to Pharmaceutical Dosage Forms, 19th ed., Mack Pub. Co., Easton, Pa. (1995). For non-sprayable topical dosage forms, viscous to semi-solid or solid forms comprising a carrier or one or more excipients compatible with topical application and having a dynamic viscosity, in some instances, greater than water are typically employed. Suitable formulations include, without limitation, solutions, suspensions, emulsions, creams, ointments, powders, liniments, salves, and the like, which are, if desired, sterilized or mixed with auxiliary agents *(e.g.,* preservatives, stabilizers, wetting agents, buffers, or salts) for influencing various properties, such as, for example, osmotic pressure. Other suitable topical dosage forms include sprayable aerosol preparations wherein the active ingredient, in some instances, in combination with a solid or liquid inert carrier, is packaged in a mixture with a pressurized volatile *(e.g.,* a gaseous propellant, such as freon) or in a squeeze bottle. Moisturizers or humectants can also be added to pharmaceutical compositions and dosage forms if desired. Examples of such additional ingredients are well-known in the art.

If the compositions comprising antibodies or antibody conjugates are administered intranasally, it can be formulated in an aerosol form, spray, mist or in the form of drops. In particular, prophylactic or therapeutic agents for use according to the present invention can be conveniently delivered in the form of an aerosol spray presentation from pressurized packs or a nebuliser, with the use of a suitable propellant *(e.g.,* dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, *e.g.,* gelatin) for use in an inhaler or insufflator may be formulated containing a powder mix of the compound and a suitable powder base such as lactose or starch.

Methods for co-administration or treatment with a second therapeutic agent, e.g., a cytokine, steroid, chemotherapeutic agent, antibiotic, or radiation, are well known in the art (see, e.g., Hardman, et al. (eds.) (2001) Goodman and Gilman's The Pharmacological Basis of Therapeutics, 10.sup.th ed., McGraw-Hill, New York, N.Y.; Poole and Peterson (eds.) (2001) Pharmacotherapeutics for Advanced Practice:A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). An effective amount of therapeutic may decrease the symptoms by at least 10%; by at least 20%; at least about 30%; at least 40%, or at least 50%.

Additional therapies (*e.g.*, prophylactic or therapeutic agents), which can be administered in combination with the antibodies of the invention or conjugates thereof, may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the antibodies of the invention. The two or more therapies may be administered within one same patient visit.

The antibodies or antibody conjugates of the invention and the other therapies may be cyclically administered. Cycling therapy involves the administration of a first therapy *(e.g.,* a first prophylactic or therapeutic agent) for a period of time, followed by the administration of a second therapy *(e.g.,* a second prophylactic or therapeutic agent) for a period of time, optionally, followed by the administration of a third therapy *(e.g.,* prophylactic or therapeutic agent) for a period of time and so forth, and repeating this sequential administration, *i.e.,* the cycle in order to reduce the development of resistance to one of the therapies, to avoid or reduce the side effects of one of the therapies, and/or to improve the efficacy of the therapies.

In certain embodiments, the antibodies and antibody conjugates of the invention can be formulated to ensure proper distribution *in vivo.* For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, *e.g.,* U.S. Patents 4,522,811; 5,374,548; and 5,399,331. The liposomes may comprise one or more moieties which are selectively transported into specific cells or organs, thus enhance targeted drug delivery (*see, e.g.,* V.V. Ranade (1989) J. Clin. Pharmacol. 29:685). Exemplary targeting moieties include folate or biotin (see, *e.g.,* U.S. Patent 5,416,016 to Low *et a*l.); mannosides (Umezawa et al., (1988) Biochem. Biophys. Res. Commun. 153:1038); antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134); p120 (Schreier et al. (1994) J. Biol. Chem. 269:9090); see also K. Keinanen; M.L. Laukkanen (1994) FEBS Lett. 346:123; J.J. Killion; I.J. Fidler (1994) Immunomethods 4:273.

The invention provides protocols for the administration of pharmaceutical composition comprising antibodies or antibody conjugates of the invention alone or in combination with other therapies to a subject in need thereof. The therapies (e.g., prophylactic or therapeutic agents) of the combination therapies of the present invention can be administered concomitantly or sequentially to a subject. The therapy (e.g., prophylactic or therapeutic agents) of the combination therapies of the present invention can also be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time and repeating this sequential administration, i.e., the cycle, in order to reduce the development of resistance to one of the therapies (e.g., agents) to avoid or reduce the side effects of one of the therapies (e.g., agents), and/or to improve, the efficacy of the therapies.

The therapies (e.g., prophylactic or therapeutic agents) of the combination therapies of the invention can be administered to a subject concurrently. The term "concurrently" is not limited to the administration of therapies (e.g., prophylactic or therapeutic agents) at exactly the same time, but rather it is meant that a pharmaceutical composition comprising antibodies or antibody conjugates of the invention are administered to a subject in a sequence and within a time interval such that the antibodies of the invention or conjugates thereof can act together with the other therapy(ies) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (e.g., prophylactic or therapeutic agents) are administered to a subject less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In other embodiments, two or more therapies (e.g., prophylactic or therapeutic agents) are administered to a within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

### Specific embodiments

1. A cysteine engineered antibody, wherein the cysteine engineered antibody comprises a substitution of one or more amino acids to a cysteine residue in the 131-139 region of the heavy chain of an antibody as defined by the EU Index numbering system, wherein the cysteine engineered antibody comprises at least one free thiol group.
2. The cysteine engineered antibody of embodiment 1, wherein said antibody comprises 2 or more free thiol groups.
3. The cysteine engineered antibody of embodiment 1, wherein said antibody comprises 4 or more free thiol groups.
4. The cysteine engineered antibody of embodiment 1, wherein said antibody comprises 6 or more free thiol groups.
5. The cysteine engineered antibody of embodiment 1, wherein said antibody comprises 8 or more free thiol groups.
6. The cysteine engineered antibody of embodiment 1, wherein said antibody comprises 10 or more free thiol groups.
7. The cysteine engineered antibody of embodiment 1, wherein said antibody comprises 12 or more free thiol groups.
8. The cysteine engineered antibody of embodiment 1, wherein said antibody comprises 14 or more free thiol groups.
9. The cysteine engineered antibody of embodiment 1, wherein said antibody comprises 16 or more free thiol groups.
10. The cysteine engineered antibody of embodiment 1, wherein the substituted amino acids are selected from the group consisting of: 131, 132, 134, 135, 136, and 139 of the antibody heavy chain, according to the EU Index numbering system.
11. The cysteine engineered antibody of any of embodiments 1-10, wherein said antibody is an antibody fragment in an Fab or Fab₂ format.
12. The cysteine engineered antibody of any of embodiments 1-11, wherein the cysteine engineered antibody comprises the formation of at least one non-naturally occurring disulfide bond.
13. The cysteine engineered antibody of any of embodiments 1- 12, wherein said engineered antibody exhibits the same or greater binding affinity for a specific target as the antibody prior to cysteine engineering.
14. The cysteine engineered antibody of any of embodiments 1- 13, wherein said engineered antibody exhibits the same or lower affinity for a specific target as the antibody prior to cysteine engineering.
15. The cysteine engineered antibody of any of embodiments 1-14, wherein said engineered antibody exhibits the same or greater binding affinity as the antibody for Fc receptors as the antibody prior to cysteine engineering.
16. The cysteine engineered antibody of any of embodiments 1-15, wherein said engineered antibody induces the same or greater level of antibody dependent cellular cytotoxicity (ADCC) as the antibody prior to cysteine engineering.
17. The cysteine engineered antibody of any of embodiments 1-15, wherein said engineered antibody induces a lower level of antibody dependent cellular cytotoxicity (ADCC) as the antibody prior to cysteine engineering.
18. The cysteine engineered antibody of any of embodiments 1-17, wherein said engineered antibody induces the same or greater level of antibody dependent complement dependent cytotoxicity (CDC) as the antibody prior to cysteine engineering.
19. The cysteine engineered antibody of any of embodiments 1-17, wherein said engineered antibody induces a lower level of antibody dependent complement dependent cytotoxicity (CDC) as the antibody prior to cysteine engineering.
20. The cysteine engineered antibody of any of embodiments 1-19, wherein said engineered antibody exhibits the same or greater level of stability measured by fragmentation and/or aggregation profile as the antibody prior to cysteine engineering.
21. The cysteine engineered antibody of any of embodiments 1-20, wherein said engineered antibody exhibits a lower level of stability measured by fragmentation and/or aggregation profile as the antibody prior to cysteine engineering.
22. The cysteine engineered antibody of any of embodiments 1-21, wherein said engineered antibody exhibits reduced half-life as compared to the antibody prior to cysteine engineering.
23. The cysteine engineered antibody of any of embodiments 1-22, wherein said free thiol group is capable of chemical conjugation to a cytotoxic agent, chemotherapeutic agent, toxin, radionuclide, DNA, RNA, siRNA, microRNA, peptide nucleic acid, non-natural amino acid peptide, enzyme, fluorescent tag, or biotin.
24. The cysteine engineered antibody of embodiment 23, wherein said cytotoxic agent is selected from the group consisting of an anti-tubulin agent, a DNA minor groove binder, an anti-mitmaytansanoid, and an auristatin.
25. The cysteine engineered antibody of embodiment 23, wherein said chemotherapeutic agent is selected from the group consisting of taxol, paclitaxel, doxorubicin, methotrexate, dolastatin, vinka alkaloids, and methotrexate.
26. The cysteine engineered antibody of embodiment 23, wherein said toxin is selected from the group consisting of abrin, brucine, cicutoxin, diphtheria toxin, botulism toxin, shiga toxin, endotoxin, tetanus toxin, pertussis toxin, anthrax toxin, cholera toxin falcarinol, alfa toxin, geldanamycin, gelonin, lotaustralin, ricin, strychnine, and tetradotoxin.
27. The cysteine engineered antibody of embodiment 23, wherein said radionuclide is selected from the group consisting of chromium (⁵¹Cr), cobalt (⁵⁷Co), fluorine (¹⁸F), gadolinium (¹⁵³Gd, ¹⁵⁹Gd), germanium (⁶⁸Ge) holmium (¹⁶⁶Ho), indium (¹¹⁵In, ¹¹³In ¹¹²In, ¹¹¹In), iodine (¹³¹I, ¹²⁵I, ¹²³I, ¹²¹I), lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (⁴⁷Sc), selenium (⁷⁵Se), strontium (⁸⁵Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (¹⁶⁹Yb, ¹⁷⁵Yb), yttrium (⁹⁰Y), and zinc (⁶⁵Zn).
28. The cysteine engineered antibody of embodiment 23, wherein said antibody is an internalizing antibody.
29. The cysteine engineered antibody of any of embodiments 1-28, wherein said antibody is a monoclonal, chimeric, humanized, bispecific, or multispecific antibody.
30. An isolated nucleic acid comprising a nucleotide sequence encoding a heavy chain variable domain or a light chain variable domain of cysteine engineered antibody of any of embodiments 1-29.
31. A vector comprising the nucleic acids of embodiment 30.
32. A host cell comprising the vector of embodiment 31.
33. An antibody conjugate of the cysteine engineered antibodies of any of embodiments 1-29.
34. A pharmaceutical composition comprising the antibody conjugate of embodiment 33.
35. A method of detecting cancer, autoimmune, inflammatory, or infectious diseases or disorders in a subject in need thereof, said method comprising administering to said subject the composition of embodiment 34.
36. The method of embodiment 35 wherein said disease or disorder comprises cells that overexpress a cell surface antigen that is bound by said antibody conjugate.
37. A method of inhibiting proliferation of a target cell, said method comprising contacting said cell with an effective amount of the antibody conjugate of embodiment 33.
38. A method of inhibiting proliferation of a target cell in a subject, said method comprising administering an effective amount of the composition of embodiment 34.
39. The method of embodiment 37 or 38 wherein said target cell overexpresses a cell surface antigen that is bound by said antibody conjugate.
40. A method of treating cancer, autoimmune, inflammatory, or infectious diseases or disorders in a subject in need thereof, said method comprising administering to said subject a therapeutically effective amount of the composition of embodiment 34.
41. The method of embodiment 40 wherein said disease or disorder comprises cells that overexpress a cell surface antigen that is bound by said antibody conjugate.
42. The method of embodiment 40, wherein said method comprises killing or reducing the growth rate of cells associated with said diseases.
43. The method of embodiment 40, wherein said method comprises depleting B cells or T cells.
44. The method of embodiment 40 comprising the administration of an additional therapy, wherein said additional therapy is selected from the group consisting of chemotherapy, biological therapy, immunotherapy, radiation therapy, hormonal therapy, and surgery.
45. A method for efficiently conjugating a heterologus molecule to the cysteine engineered antibodies of any of embodiments 1-29.
46. The method of embodiment 45 wherein said method comprises conjugating said heterologus molecule to at least one position selected from the group consisting of 131, 132, 133, 134, 135, 136, 137, and 139 of the CH1 domain of the antibody.
47. The method of embodiment 45 or 46 wherein said heterologus molecule is selected from the group consisting of a cytotoxic agent, chemotherapeutic agent, toxin, radionuclide, DNA, RNA, siRNA, microRNA, peptide nucleic acid, peptide, enzyme, fluorescent tag, or biotin.
48. The method of embodiment 47 wherein said cytotoxic agent is selected from the group consisting of an anti-tubulin agent, a DNA minor groove binder, an anti-mitmaytansanoid, and an auristatin.
49. The method of embodiment 47 wherein said chemotherapeutic agent is selected from the group consisting of taxol, paclitaxel, doxorubicin, methotrexate, dolastatin, vinka alkaloids, and methotrexate.
50. The method of embodiment 47 wherein said toxin is selected from the group consisting of abrin, brucine, cicutoxin, diphtheria toxin, botulism toxin, shiga toxin, endotoxin, tetanus toxin, pertussis toxin, anthrax toxin, cholera toxin falcarinol, alfa toxin, geldanamycin, gelonin, lotaustralin, ricin, strychnine, and tetradotoxin.
51. The method of embodiment 47 wherein said radionuclide is selected from the group consisting of chromium (⁵¹Cr), cobalt (⁵⁷Co), fluorine (¹⁸F), gadolinium (¹⁵³Gd, ¹⁵⁹Gd), germanium (⁶⁸Ge), holmium (¹⁶⁶Ho), indium (¹¹⁵In, ¹¹³In, ¹¹²In, ¹¹¹In), iodine (¹¹³I, ¹²⁵I, ¹²³I, ¹²¹I), lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Per), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (⁴⁷Sc), selenium (⁷⁵Se), strontium (⁸⁵Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (¹⁶⁹Yb, ¹⁷⁵Yb), yttrium (⁹⁰Y), and zinc (⁶⁵Zn).
52. The method of any of embodiments 45-51 wherein said efficiency is at least 5% or more as measured by residual free thiol groups remaining after the conjugation reaction.
53. The method of any of embodiments 45-52 wherein said efficiency is at least 25% or more as measured by residual free thiol groups remaining after the conjugation reaction.
54. The method of any of embodiments 45-53 wherein said efficiency is at least 75% or more as measured by residual free thiol groups remaining after the conjugation reaction.
55. The cysteine engineered antibody of any of embodiments 1-29, wherein said antibody does not comprise a substitution to cysteine at position 132 and/or 138.
56. The cysteine engineered antibody of any of embodiments 1 -29 or 55 wherein said antibody comprises a substitution at position 132 and/or 138, wherein said substitution is not cysteine.
57. The cysteine engineered antibody of any of embodiments 1-29 or 55-56 wherein said antibody comprises at least one expansion of the 131-139 loop region.
58. The cysteine engineered antibody of any of embodiments 1-29 or 55-57 wherein said antibody comprises an expansion of the 131-139 loop region, wherein said expansion comprises the insertion of at least 1 to at least 15 amino acids.
59. The cysteine engineered antibody of any of embodiments 1-29 or 55-58 wherein said antibody comprises an expansion of the 131 -139 loop region, wherein said expansion occurs after a positions selected from the group consisting of residues 131, 132, 133, 134, 135, 136, 137, 138 and 139.
60. The cysteine engineered antibody of any of embodiments 1 -29 or 55-59 wherein said antibody comprises an expansion of the 131-139 loop region, wherein said expansion occurs after a positions selected from the group consisting of residues 131 , 132, 133, 134, 135, 136, 137, 138 and 139.
61. The cysteine engineered antibody of any of embodiments 1 -29 or 55-69 wherein said antibody comprises at least a first and a second expansion of the 131-139 loop region, wherein said first expansion occurs after a position selected from the group consisting of residues 131, 132. 133, 134, 135, 136, 137, 138 and 139 and wherein said second expansion occurs after said first expansion, wherein said second expansion occurs after a position selected from the group consisting of residues 131, 132, 133, 134, 135, 136, 137, 138 and 139.

### Equivalents

The foregoing written specification is considered to be sufficient to enable one skilled in the art to practice the invention. The foregoing description and Examples detail certain preferred embodiments of the invention and describes the best mode contemplated by the inventors. It will be appreciated, however, that no matter how detailed the foregoing may appear in text, the invention may be practiced in many ways and the invention should be construed in accordance with the appended claims and any equivalents thereof.

### 6. EXAMPLES

The invention is now described with reference to the following examples. These examples are provided for the purpose of illustration only and the invention should in no way be construed as being limited to these examples but rather should be construed to encompass any and all variations which become evident as a result of the teachings provided herein.

### 6.1 Example 1. Expression and characterization of cysteine engineered antibodies

A series of cysteine for serine or threonine substitutions were made to the 131-139 region of the CH1 domain of an IgG1 molecule. The cysteine engineered IgG1 molecules were generated using standard DNA recombinant technologies known to practitioners of the biological arts.( See, e.g. Sambrook et al. Molecular Cloning-A Laboratory Manual, December 2000, Cold Spring Harbor Lab Press). The 131-139 region of the CH1 domain present in an IgG1 molecule represents a flexible region which is solvent exposed (See Figure 1A). The exposure to solvent that this region displays allows for the access for conjugation reagents to the specific residues. A sequence alignment of other various antibody formats representing the equivalent positions of 131-139 in the CH1 domain of IgG1 is presented in Figure 1C. Serine and/or threonine residues contained in this region are particular candidate amino acids to be substituted with cysteine residues.

One example of a cysteine engineered antibody strategy is presented in Figure 1B. Presented in bold are the naturally occurring cysteine residues in this sequence along with the predicted disulfide bond pair pattern (solid lines). Underlined is the position of a cysteine replacement of a serine residue at position 131. Potential disulfide bonds comprising this introduced cysteine are presented as dashed lines.

In Figure 2A, the 1C1 wild type and cysteine engineered derivatives thereof were expressed, purified and subjected to PAGE analysis. The 1C1 wild type (Lane 1) antibody and various cysteine engineered derivatives thereof (Lanes 2-15) exhibited very similar molecular weight profiles under non-reducing (inset i) and reducing (inset ii) conditions.

In Figure 2B, a comparison of non-reducing peptide mapping of 1C1 wt and 1C1 Ser131Cys mutant. In this example, limited proteolysis and reversed-phase chromatography/mass spectrometry (RP-LC/MS) was used to characterize the disulfide bond patterns and free cysteine residues in wild type and cysteine engineered 1C1 and 1C6 antibodies.

Materials and Methods: Non-reducing peptide mapping. MAbs were capped with 5 mM N-Ethylmaleimide (NEM) in acidic buffer at room temperature for 20 min followed by denaturing in 10 mM phosphate buffer, 250 mM NaCl, 6 M Guanidine, pH 7.0 at 37 °C for 30 min. the denatured MAb solutions were then diluted 6 fold with 100 mM phosphate buffer, 0.1 mM EDTA, pH 7.0. Endopeptidase Lys-C was added at 1:10 enzyme to protein ratio. The reaction mixtures were incubated at 37 °C for 16 to 24 hours. Half of the reaction mixture was reduced by adding 5-10 µL of 500 mM DTT and incubated at 37 °C for 15 min. Both non-reduced and reduced digests were analyzed by LC/MS. HPLC separation was achieved using reverse-phase HPLC (Phenomenex Jupiter 5m C18 column; 250 x 2 mm) and the detected by UV-detector and an on-line LTQ Ion Trap mass spectrometer (Thermo Fisher). The RP-HPLC mobile phase A was 0.1% TFA in H2O and the mobile phase B was 0.1% TFA in acetonitrile. The flow rate is 0.2 mL/min and the gradient was 0 to 60% B in 150 min. LTQ ion trap mass spectrometer with electrospray interface was operated in positive ion mode with a m/z range of 300-2000. Each peak in the peptide maps was identified by MS full scan and zoom scan analysis. MS/MS spectra were also collected to verify the sequences of the peptides. Disulfide bond linkages were determined by peptide masses from LC-MS experiments, and confirmed by comparing the peptide maps of reduced and non-reduced digests. Free thiol-containing peptides were identified by searching for peptide masses with NEM adducts.

Results: 1C1 is an EphA2 specific antibody of the IgG1 subclass. Figure 2B Inset A is the 1C1 WT antibody which showed the regular disulfide bond linkage between heavy chain hinge region and light chain C-terminus (H11-L15) and the peptide containing Ser131 (H5). Figure 2B Inset B is the 1C1 Ser131Cys mutant which showed the decrease of the regular disulfide bond between Light and heavy chain (H11-L15) and 1C1 wt peptide H5, and the appearance of new formed disulfide bond linkages between mutated cysteine to light chain C-terminus (H5m-L15) and to hinge-region (H5m-H11). Using the method described above we determined disulfide bond linkage for 1C1, 1C1 Ser131Cys and for the other mutants. In addition we determined that the 1C1 Ser131Cys antibody forms a new interchain disulfide bridge between light and heavy chain and the cysteine in position 220 of the heavy chain is free for site-specific drug conjugation. Free thiols were also identified using the above methods. Similar results were obtained with other cysteine engineered antibodies (data not shown).

In Figure 3, plots representing the results of a Size-exclusion chromatography (SEC) analysis of the 1C6 (an EphB4 specific antibody) wild type (A) and 1C6 Ser131Cys antibodies are presented. Size exclusion chromatography is a method well known in the art to determine the apparent molecular weight of molecules (e.g. proteins) in their native state. In this example, the purified antibodies 1C6 wild type (A) or 1C6 Ser131Cys (B) were loaded onto a SEC column (TSK-GEL G3000SWXL) in a buffer containing 100 mM Sodium Sulfate, 100 mM Sodium Phosphate at pH 6.8. The column was run at a flow rate of 1 ml/min. Calibration standards included for the determination of the apparent molecular weight included: Thyroglobulin (670 kDa), Bovine gamma-globulin (158 kDa), Chicken ovalbumin (44 kDa), Equine myoglobin (17 kDa), and Vitamin B12 (1.35 kDa). As demonstrated by the very similar tracings presented in the panel (A and B), the 1C6 wild type and 1C6 1Ser131Cys exist in a monomeric state. Similar results were obtained with other cysteine engineered antibodies (data not shown).

In Figure 4, plots representing the results of a Size-exclusion chromatography (SEC) analysis of the 1C1 wild type (A) 1C1 Ser134Cys (B), 1C1 Ser131-132Cys (C), and 1C1 Ser131-132-134-136Cys (D) antibodies are presented. Size exclusion chromatography was performed as above. As demonstrated by the very similar tracings presented in the panel (A - D), the 1C1 wild type and various cysteine engineered mutants thereof exist in a monomeric state. Similar results were obtained with other cysteine engineered antibodies (data not shown).

### 6.2 Example 2. Epitope Binding Characterization of Cysteine Engineered Antibodies

In this example, the binding characteristics of a cysteine engineered antibody were compared to the parent wild type antibody.

Materials and methods: The binding assay was carried out in 1% BSA in 1X PBS, all incubation steps were carried out at room temperature using a Lab-Line Instrument titer plate shaker at a shaking speed of 6.5. Biotynilated EphB4 or EphA2 and rutherium labeled (BV tag) anti-human kappa were incubated with Streptavidin M280 Beads and with a serial dilution of 1C6, 1C6 Ser131Cys and 1C1 and 1C1 Ser131Cys, respectively. Receptor and anti-human kappa concentration was 1 µg/ml and the antibody concentration was from 1µg/ml to 7.8 ng/ml. The specific binding was revealed using the Bioveris M-series Analyzer. The machine aspirates the mixture from the plate and flows it over a electromagnet. The M280 beads stick to the platform and a wash solution is then flowed over the beads to remove any unbound antibody or receptor. A static charge was applied to the platform that travels up to the rutherium label in the sandwich causing it to emit light. The read solution acts as a final electron acceptor allowing the rutherium to continuously emit light as long as the charge is applied. The electromagnet was then disengaged and the sample was washed away. The washing and read was automatically done by the machine and was consistent between wells.

Results: In this example, an ELISA (in solution format) based antigen binding assay was performed on purified antibodies namely 1C6 WT and 1C6 Ser131Cys. These antibodies specifically recognize the EphB4 receptor. As demonstrated in Figure 5, the binding affinity profile measured in an ELISA format of the WT antibody and the cysteine engineered Ser131Cys antibody were very similar. Similarly, for 1C1 based cysteine engineered antibodies, the binding affinity profile measured in an ELISA format for the 1C1 wild type and 1C1 Ser131Cys antibodies were very similar (See Figure 6). The inclusion of a reducing agent such as 1 mM DTT had no affect on the binding profile exhibited by the cysteine engineered antibody 1C1 Ser131Cys. Similar results were obtained with other cysteine engineered antibodies (data not shown). These results demonstrate that the engineering of Cysteine residues into the CH1 domain does not alter the epitope binding characteristics of the resultant antibody as compared to the parental antibody.

### 6.3 Example 3. Stability characterization of Cysteine Engineered Antibodies

In this Example, the melting temperatures (Tm) of the parental (wild type) antibodies are compared with the cysteine engineered antibodies.

Materials and Methods: Differential scanning Calorimetry (DSC) was used to determine the temperature of melting (Tm) for wild-type and cysteine engineered antibodies. The Tm is a representation of the stability of the antibody, higher Tm relates to very stable and not aggregate antibody. DSC experiments measured the heat capacity of the antibody studied in this invention (wild-types and cysteine engineered antibodies) as a function of temperature in a range from 10°C to 110°C. DSC measurements were carried out using a Microcal VP-DSC ultrasensitive scanning microcalorimeter. DSC experiments were carried out in 25 mM Histidine-HCl pH6, 5 mM EDTA. All solutions and samples used for DSC were filtered using a 0.22 micron-filter and degassed just prior to loading into the calorimeter. For each set of measurements, a buffer-versus baseline runs were first obtained. Immediately after this, the buffer solution was removed from the sample cell. The sample cells were loaded with 0.5 ml of an antibody (wild-type and cysteine engineered) solution at concentration ranging from 0.5 to 1 mg/ml. During measurement the reference cell was filled with the sample buffer. From each sample-versus-buffer experiment, the corresponding buffer-versus-buffer baseline run was subtracted. The raw data were normalized for concentration and scan rate. The data were fitted using the Origin DSC software provided by Microcal. DSC experiments were carried out also with conjugated antibodies (with EZ-Link Biotin-HPDP (Pierce) and Z-Link iodoacetyl-PE02 Biotin and similar thermograms to the wild-type and cysteine engineered antibodies were obtained.

Results: In this Example, Differential Scanning Calorimetry (DSC) was used to determine the melt curve of various wild type and Cysteine engineered antibodies. In Figure 7 Differential Scanning Calorimetry (DSC) thermograms of the 1C6 WT antibody (A) and 1C6 Ser131Cys antibody (B) are presented. Both antibodies exhibit very similar melting temperatures (Tm) of 70°C and 69°C respectively. In Figure 8 Differential Scanning Calorimetry (DSC) thermograms of the 1C1 WT (A), 1C1 Ser131Cys (B), 1C1 Ser134Cys (C), 1C1 Ser(131-132)Cys (D), and 1C1 Ser(131-132-134-136)Cys antibodies. All of the antibodies exhibit a very similar melting temperature (Tm). Similar results were obtained with other cysteine engineered antibodies (data not shown). These results demonstrate that the engineering of cysteine residues into the CH1 domain does not alter the stability of the resultant antibodies.

### 6.4 Example 4. Biotin Conjugation of Cysteine Engineered Antibodies

In this Example, free conjugation sites on cysteine engineered antibodies are demonstrated by an increased incorporation of biotin.

Materials and Methods: EZ-Link Biotin-HPDP (Conjugation Reagent 1= CR1) and EZ-Link iodoacetyl-PEO2 Biotin (Conjugation Reagent 2= CR2) were obtained from Pierce. Wild-type and cysteine engineered antibodies were incubated for 3 h at 37°C in 100 mM phosphate buffer, 100 mM NaCl pH 8.0, 0.02 mM DTT, 5 mM EDTA under nitrogen. After this incubation the antibody samples were buffer exchanged using dialysis in 1PBS 1X, 1 mM EDTA under nitrogen. CR1 was dissolved at 2 mg/ml in 100 % DMSO and CR2 was dissolved in distilled water. Seven µg/ml of conjugation reagent and 0.5 mg/ml of both wild-type and ser131cys antibodies were mixed separately, vortexed and then incubated for 90 minutes at 4°C, 37°C, 45°C and 55°C. Unbound CR1 and CR2 were removed using either SEC (size-exclusion chromatography) or desalting columns (ZEBA, Desalt Spin Columns from Pierce). Biotin incorporation was determined using a Streptavidin binding assay. The binding assay was carried out in 1% BSA in 1X PBS, all incubation steps were carried out at room temperature using a Lab-Line Instrument titer plate shaker at speed setting of 6.5. Streptavidin M280 Beads and ruthidium (BV tag) labeled anti-human Kappa were mix with a serial dilution of biotinylated ser131cys and wild-type, respectively. Anti-human Kappa concentration was 1 µg/ml and the antibody concentration was from 1 µg/ml to 7.8 ng/ml. The specific binding was evaluated using the Bioveris M-series Analyzer.

Results: Presented in Figure 9 are the results from a biotin conjugation study of 1C6 (WT) antibody and the 1C6 Ser131Cys (Mut) antibody under various conditions. In panel A, the 1C6 and 1C6 Ser131Cys antibodies were subjected to a conjugation reaction with EZ-Link Biotin-HPDP (Pierce) at various temperatures (4°C, 37°C, 45°C, and 55°C). The resultant biotin conjugation efficiency was measured and plotted. The 1C6 Ser131Cys antibody exhibited a higher efficiency of site-specific biotin conjugation than the 1C6 antibody. In panel B, the 1C6 and 1C6 Ser131Cys antibodies were subjected to a conjugation reaction with EZ-Link iodoacetyl-PEO2 Biotin at various temperatures (4°C, 37°C, 45°C, and 55°C). The resultant site-specific biotin conjugation efficiency was measured and plotted. The 1C6 Ser131Cys antibody exhibited a higher site-specific biotin conjugation efficiency than the 1C6 antibody. These results demonstrate that cysteine engineered antibodies, such as the 1C3 Ser131Cys antibody display cysteines capable of conjugation to various agents (for example, conjugation to biotin).

### 6.5 Example 5. Characterizing Binding Affinity for Fcγ Receptors Exhibited by Cysteine Engineered Antibodies

In this Example, the binding characteristics specific for Fcγ receptors exhibited by cysteine engineered antibodies were compared to wild type antibodies.

Materials and Methods: BIAcore^{®} experiments were carried out using a BIAcore^{®} 3000 instrument (Biacore International) and using standard protocols. Briefly, 7444RU (Resonance Unit) of 1C1-wt and 7781RU of 1C1 ser131cys were coupled to the dextran matrix of a CM5 sensor chip (Pharmacia Biosensor) using a standard amine coupling kit. Excess reactive esters were quenched by injection of 70 µl of 1.0 M ethanolamine hydrochloride (pH 8.5). The FcγRs (I, IIA, IIIA, IIB) were injected at 500 nM at a flow rate of 5 µl/min. The binding levels of FcγRs are similar for 1C1 wild-type and 1C1 ser131cys. Ovalbumin was used as a negative control. After the binding experiments, the sensor chip surface for 1C1 wild-type and 1C1 ser131cys mutant were regenerated using 1 M NaCl/50 mM NaOH. The regenerated surface chips were used to determine the binding to human FcRn in either 50 mM phopahte buffer pH 6.0 containing 0.05% Tween 20 or in 50 mM phopahte buffer pH 7.4 containing 0.05% Tween 20. A solution containing human FcRn was flowed over the sensor chips at 5 µl/min. The binding level for both wild-type and mutant are similar. Ovalbumin was used as a negative control.

Results: Presented in Figure 10 are the results from a BIAcore^{®} assay measuring the relative affinities for the 1C1 WT and 1C1 Ser131Cys antibodies for various Fcγ receptors. The various Fc_{γ} receptors studied were FcγRI (A), Fc_{γ}RIIIA (B), Fc_{γ}RIIA (C), Fc_{γ}RIIB (D). The 1C1 WT and 1C1 Ser131Cys antibodies exhibit very similar binding affinities for various Fc_{γ} receptors. Also, presented in Figure 11 are the results from a BIACORE® assay measuring the relative affinities for the 1C1 WT and 1C1 Ser131Cys antibodies for the FcRn receptor at pH 6.0 and pH 7.4. The 1C1 Ser131Cys antibody binds the FcRn receptor with a similar binding profile to the 1C1 WT antibody at both pH 6.0 and pH 7.4. Similar results were obtained with the other cysteine engineered antibodies tested. These results demonstrate that the engineering of cysteine residues into the CH1 domain of an antibody does not affect the binding affinity for Fcγ receptors and thus does not affect the ability to control effector functions.

### 6.6 Example 6. Internalization of Cysteine Engineered Antibodies

In this Example, the cysteine engineered antibodies were tested for the ability to internalize upon binding a cell surface antigen.

Materials and Methods. The internalization assay was carried out using 96 well U bottom plate. The PC3 cells (PC3 cells naturally express a high level of EphA2) at concentration of 10⁶ cells/ml were incubated with control antibody (R347), 1C1 wild-type and 1C1 cysteine engineered antibodies, all at 1 mg/ml in ice for 30 minutes. After this incubation, the cells were washed twice in 1X PBS. The cells where then fixed at room temperature for 20 minutes in 3.7 % paraformaldehyde and washed twice in 1X PBS. The cells were permeabilized with 0.5% Triton X-100 in 1X PBS for 5min at room temperature and washed twice in 1X PBS. One microgram of secondary antibody (Alexa-Fluor 488 goat anti-human IgG (H+L) Molecular Probes #A11013) in 1X PBS, 2% FBS was added to the cells and incubated at room temperature in the dark for 30 min. After this incubation, the cells were washed twice in 1X PBS and directly coated on microscope treated slides. The cells where then mounted beneath a microscope coverslip (Coverslips VWR#48382-138) using DAPI-containing mounting media (VectaShield HardSet Mounting Medium with DAPI. Vector Laboratories #H-1500). The slides were then incubated overnight at 4°C and subsequently visualized using a Kikon Eclipse 55i Fluroscent fluorescent microscope.

Results: Presented in Figure 12 are the results from an antibody internalization study performed on PC3 cells. A set of controls are presented in the first panel. In (A) unstained cells are counterstained with DAPI. In (B) cells stained with secondary antibody alone are counterstained with DAPI. In (C) a control primary antibody, R347 is incubated with the cells as well as counterstaining with DAPI. In (D) the cells are incubated for one hour and subsequently stained with R347. None of the controls (A-D) exhibit any antibody specific cell staining. In (E) cells are incubated with 1C1 wt antibody at time zero and for one hour. Two representative images at one hour indicate internalization of the 1C1 WT antibody. In (F) cells are incubated with 1C1 Ser131Cys antibody at time zero and for one hour. Two representative images at one hour indicate internalization of the 1C1 Ser131Cys antibody. In (G) cells are incubated with 1C1 Ser134Cys antibody at time zero and for one hour. Two representative images at one hour indicate internalization of the 1C1 Ser134Cys antibody. In (H) cells are incubated with 1C1 Ser(131-132)Cys antibody at time zero and for one hour. Two representative images at one hour indicate internalization of the 1C1 Ser(131-132)Cys antibody. In (I) cells are incubated with 1C1 Ser(131-132-134-136)Cys antibody at time zero and for one hour. Two representative images at one hour indicate internalization of the 1C1 Ser(131-132-134-136)Cys antibody. All of the cysteine engineered antibodies internalized to a similar extent as compared to the wild type antibody. These results demonstrate that the internalization of the antibody is unaffected by the engineering of cysteine residues in the CH1 domain.

### 6.7 Example 7. Quantification Of Free Thiols In Cysteine Engineered Antibodies

In this Example, the free thiols in cysteine engineered antibodies were determined.

Materials and Methods: Quantitative determination of antibodies free sulfhydryl (-SH) groups were determined using Ellman's DNTB reagent (DNTB: 5,5'-dithio-bis-(2-nitrobenzoic acid) . DNTB was dissolved in DMF at 25mM. A solution of this compound produces a measurable yellow-colored product (TNB; the extinction coefficient of 13600 M-1 cm-1) that when released upon binding of DNTB to the free sulfhydryl absorb in the visible range at 412 nm at pH 8.0. Antibodies samples were prepared using the same methods used for conjugation. Sulfhydryl groups in the wild-type antibody and in the cysteine engineered antibodies were estimated by a simple comparison to a standard curve composed of known concentrations of a sulfhydryl-containing compound, such as cysteine. Alternatively, sulfhydryl group can be quantified using the extinction coefficient of the TNB, which is released upon DNTB binding to free thiols and his amount is directly linked to the total free sulfhydryl groups. Twenty microliters of DNTB working solution at concentration of 25 mM were diluted into 990 µl of sample at concentration of 1 mg/ml. Same volume of DNTB was diluted into 990 µl of sample dialysis buffer for the blank test tube, and for cysteine standards (if used) at a standard concentration of 10 µM. DNTB and samples were vigorously mixed and incubated at room temperature for 15 minutes at 37°C. The optical absorbance at 412 nm and at 280 nm were measured using a Agilent 8453 UV-Visible Spectroscopy and using 50 µl quartz cuvette. To calculate the free thiols present in antibodies the averaged absorbance of two independent measurements at 412 nm was divided by 13600 M-1 cm-1 (the extinction coefficient of the TNB) to get the molarity in the assay, the molarity of the antibody also determined. Free sulfhydryl was calculated by dividing the molarity of TNB by the antibody molarity in solution.

Results: Using the methods described above, a determination of the number of free thiols was performed on the 1C6 and 1C1 wild type antibodies and the 1C6 and 1C1 Ser131Cys antibodies. Integrating the absorbance readings into an integer representing the number of free thiols presented for Ellman's reagent binding results the data presented in Table 1. This data demonstrates that the resultant cysteine engineered antibodies display 2 free thiols (one for each modified CH1 domain in the antibody dimer) as predicted. Similar results were obtained with other cysteine engineered antibodies (data not shown). These results demonstrate that the cysteine engineered antibodies display free thiols as predicted.

**Table 1. Determination of free thiol groups in wild type and cysteine engineered antibodies**

| Antibody | # of free thiols |
|---|---|
| 1C6 wild type | 0 |
| 1C6 Ser131Cys | 2 |
| 1C1 wild type | 0 |
| 1C1 Ser131Cys | 2 |

### 6.8 Example 7. Cysteine engineered antibody binding specificity

In this Example, the binding specificies of various cysteine engineered antibodies were determined in a comparison with the antibody prior to cysteine engineering.

Materials and Methods: In this Example, an ELISA based assay was performed to determine the relative binding specificities to EphA2 of various cysteine engineered antibodies derived from 1C1. Recombinant mouse EphA2-Fc was coated on the ELISA plate. Each antibody was formulated at 2 µg/ml and analyzed for binding with an anti-kappa antibody conjugated to HRP. The data is an average of three independent experiments.

Results: Presented in FIGURE 13 are the results from this experiment in which the cysteine engineered antibodies displayed an equivalent binding specificity for EphA2 compared with the wild type 1C1 prior to cysteine engineering. The use of 2 unrelated antibodies (Control antibody 1 and 2) confirm the specificity of this ELISA experiment for EphA2.Also, multiple substitutions of cysteine residues do not alter the binding specificity of the antibody for its cognate antigen. These results demonstrate that the cysteine engineering of antibodies does not alter the binding specificities as compared to the antibody prior to cysteine engineering. 3 Example 9, Cysteine engineered antibodies can be conjugated to PEG.

In this Example, various cysteine engineered antibodies were conjugated to polyethylene glycol (PEG) via a maleimide linker. The free engineered cysteines present on the antibodies require uncapping io expose the free sulfhydryl group for conjugation.

Materials and Methods: The cysteine antibody mutants (1C1 WT, 1C1 Ser134Cys, 1C1 Ser136Cys, and 1C1 Ser132Cys-Ser134Cys, etc.) were prepared using traditional mammalian transient expression. During purification the samples were continuously under a stream of nitrogen gas in order to minimize cysteine oxidation by the air (oxygen). All antibody purification buffers contained at least 25 mM EDTA in order to chelate any agent that could block or bind the free cysteines. All manipulation were carried out in conjugation buffer (CB) containing: 0.1 M sodium phosphate, 0.15 M NaCl, 0.025 M EDTA, pH 7.2. pH 7.2 is optimal for maximize the specificity of Maleimide-Cysteine conjugation. The conjugation moiety (Maleimide-PEG2000) is freshly prepared each time in CB. The antibody mutants were incubated in CB with 10 mM Cysteine-HCl for 30 minutes at 37 °C under constant rotation. After cysteine treatment the free cysteine were removed by protein desalting in CB, using commercially available desalting columns (Zeba column purchased from Pierce). After desalting the cysteine antibody mutants were incubated with about 3 molar excess of conjugation reagent (in this case Maleimide- PEG2000, purchased from NOF North America Corporation). Conjugation was carried out for 2 hours at 37 <0>C under constant rotation. After conjugation the excess of Maleimide- PEG20000 is removed by protein desalting. Samples were monitored by densitometry' analysis of the ratio of coπjugated/non-eonjugated heavy chain bands as seen in the SDS- PAGE.

Results: Presented in FIGURE 14 is the SPS-PAGE analysis of the uncapping and conjugation of the cysteine engineered antibodies to maleirmde-PEG2000. Conjugation was visualized as a higher molecular weigh band seen in the lanes that, represent antibodies plus PEG (lanes 5-8, 13-16) as compared to the banding profile observed in the lanes that represent antibodies in the absence of PEG (lanes 1-4, 9-12). The cysteine engineered antibodies (1C1 Ser134Cys, 1C1 Ser136Cys, 1C1Ser132-134Cys) prior to the uncapping reaction display a low but detectable level of conjugation with PEG (lanes 2-4, 6-8). The cysteine engineered antibodies after treatment with free cysteine display a higher level of conjugation (lanes 10-12, 14-16) as compared to the cysteine engineered antibodies prior to the uncapping reaction (lanes 2-4, 6-8). The non-cysteine treatment lanes demonstrate a lowered level of PEGylation (higher molecular weight band) as compared with a treatment of the cysteine engineered antibodies with 10 mM free cysteine. Control wells containing antibodies prior to cysteine engineering exhibit no detectable level of pegylation in either condition (lanes 1, 5, 9, and 13). These results suggest that the cysteine engineered antibodies (1C1 Ser134Cys, 1C1 Ser136Cys, 1C1 Ser132-134Cys) display a free cysteine thai is partially capped. The cap of the sulfhydryl group is efficiently removed by the uncapping reaction and frees the sulfhydryl group to be reactive to a conjugate.

### 7.10 Example 10. Uncapping of cysteine engineered antibodies does not disturb overall antibody structure

In this Example, various cysteine engineered antibodies were uncapped to expose free cysteine residues for conjugation in an effort to determine the overall stability of the antibody structure.

Materials and Methods: The uncapping procedure for this Example was as follows: Parental and mutant antibodies were incubated at 37°C in PBS IX pH 7.4, 10 mM EDTA with cysteine-HCl using a molar ratio of 75 (cysteine-HCl/antibody) under constant rotation and nitrogen gas. The excess of cysteine-HCl was removed by buffer exchange and overnight dialysis in PBS IX pH 7.4, 10 mM EDTA. The dialysis was carried out at room temperature under nitrogen gas in order to minimize oxidation of the uncapped cysteines. Untreated and Uncapped antibodies were analyzed by SDS-PAGE electrophoresis to determine the antibody structure integrity.

Results: Presented in FIGURE 15 are the results from this experiment demonstrating that the uncapping protocol to free up the unpaired cysteines in the cysteine engineered antibodies does not disrupt the interchain disulfide bonds and therefore the overall antibody structure. Comparing the untreated to the cognate treated lanes, the cysteine engineered antibodies (1C1 Ser134Cys, 1C1 Thr135Cys, 1C1 Ser136Cys, 1C1 Ser139Cys) do not demonstrate any difference in SDS-PAGE profile which suggests that the antibodies have not been reduced by cysteine treatment, Also, as a control, the wild type antibody (lanes 2, 8) do not exhibit any change in SDS-PAGE profile, further complementing the data which suggests that, the overall antibody structure remains intact during and after the uncapping protocol.

### 7.11 Example 11. Cysteine engineered antibodies caø be conjugated to PEG-Biotin

In this Example, various cysteine engineered antibodies were conjugated to PEG- Biolin via a maleimide linker.

Materials and Methods: Cysteine engineered antibodies and the wild type control antibodies were prepared as presented above. Conjugation was carried out at 37°C in PBS IX pH 7.4, 10 mM EDTA using Maleimide∼PEG2-Biotin and using a molar ration of 1 :6 (Maleimide-PEG2"Biolin"Antibody), for 2 hours at 37 °C under constant rotation. The non-reacted Maleiτnide-PEG2-Biotin was removed by extensive dialysis in PBS IX pH 7.4, 10 mM EDTA at 4 °C. Antibodies were analyzed by Western Blot analysis and visualized for Bio tin. The antibody was present at a concentration of 1 mg/ml. However, similar results were obtained with antibody concentrations of up to 5 mg/ml.

Results: Presented in FIGUPvE 16 are the results from a biotin conjugation reaction with various cysteine engineered antibodies. Lane 1 is a control antibody labeled with biotin to visualize the predicted size of an antibody with biotin conjugated to a free cysteine. The 1C1 wild- type antibody did not display biotin conjugation due to the lack of free cysteines (lane 3). The various cysteine engineered antibodies (1C1 Ser134Cys, 1C1 Thr135Cys, 1C1 Ser136Cys, 1C1 Ser139Cys) displayed efficient conjugation to biotin at the expected molecular weight (lanes 4-7). These results demonstrate that the cysteine engineered antibodies 1C1 Ser134Cys, 1C1 Thr135Cys, 1C1 Ser136Cys, 1C1 Ser139Cys display an exposed free cysteine capable of conjugation to biotin.

### 7.12 Example 12. Conjugated cysteine engineered antibodies retain binding to congnate antigens.

In this Example, various cysteine engineered antibodies conjugated to Biotin were tested for the retention of binding specificity as compared to a control, non- conjugated antibody.

Materials and Methods: Cysteine engineered antibodies were prepared as presented in Example 11. The ELISA plate was prepared with recombinant !y produced EphA2-FLAG coated at 2 µg/nil. The various antibodies were incubated with the ELISA plate, washed and detected with anti-Strepavidin conjugated to HRP.

Results: Presented in FIGURE 17 are the results from an ELJSA plate binding experiment in which the various cysteine engineered antibodies conjugated to biotin were tested for retention of cognate antigen specificity. Control antibodies could not be visualized on the plate as the do not contain conjugated biotin for detection. In this experiment, biotin conjugated cysteine engineered antibodies 1C1 Ser134Cys, 1C1 Thr135Cys, 1C1 Ser136Cys, 1C1 Ser139Cys retained binding for EphA2. These results suggest that conjugation of the various cysteine engineered antibodies does not adversely affect binding to their cognate antigens.

### 7.13 Example 13. Quantification of biotin conjugation to cysteine engineered

In this Example, various cysteine engineered antibodies conjugated to Biotin were analyzed for the specific content of Biotin per antibody molecule.

Materials and Methods: Cysteine engineered antibodies were prepared as presented in Example 11. Standard mass spectrometry analytical techniques were employed to determine the apparent mass of the respective antibodies. The intact mass of the unconjugated and conjugated antibodies were determined. The difference between the unconjugated mass and the conjugated mass was determined by spectrometry. Using the predicted mass of two biotin molecules as approximately 1051.24 Da., the difference of the unconjugated and conjugated mass may reflect the additional biotin molecules.

**Table 2: Biotin content of various cysteine engineered antibodies**

| Antibody | Unconjugated Mass (Da) | Conjugated Mass (Da) | *D mass (Da) | Number of Biotins |
|---|---|---|---|---|
| 1C1 Wild type + Biotin | 149440.7 | 149439.0 | 1.7 | 0 |
| 1C1 Ser134Cys + Biotin | 149472.8 | 150527.0 | 1054.2 | 2 |
| 1C1 Thr135Cys + Biotin | 149444.0 | 150497.0 | 1053.0 | 2 |
| 1C1 Ser136Cys + Biotin | 149471.7 | 150533.0 | 1061.3 | 2 |
| 1C1 Thr139Cys + Biotin | 149453.0 | 150511.0 | 1058.0 | 2 |

**Results: Presented in Table 2 are the results from a mass spectrometry analysis** of various antibodies conjugated to biotin. The ICl wild type antibody (even when treated with biotin) displays no significant difference of the unconjugated mass as compared to the conjugated mass suggesting that there are no biotin molecules conjugated to that antibody. The 1C1 Ser134Cys, 1C1 Thr135Cys, 1C1 Ser136Cys, 1C1 Ser139Cys all display a difference in mass between the conjugated mass and the unconjugated mass. This difference is approximately equal to the predicted mass of two biotin molecules of 1051.24 Da. Thus, due to the homodimeric nature of antibody heavy chains, the single cysteine substitution in the heavy chain of each antibody.

### 7.14 Example 14. Conjugation to cysteine engineered antibodies is site-specific and highly efficient

In this Example the efficiency and position of biotln conjugation to various cysteine engineered antibodies was determined.

Materials and Methods: Cysteine engineered antibodies were prepared as presented Example 1 1 . Standard peptide mapping techniques were employed to determine tbe relative position and efficiency of the biotin conjugation reaction. Based on the primary sequence of the antibody a theoretical mass and peptide fragmentation profile was determined. This theoretical mass was compared with the observed mass displayed by the peptides and the difference was determined. The predicted mass change exhibited by the biotin molecule conjugated to a specific peptide was identified. In addition, the relative intensity of the biotin containing peptide and the non-conjugated peptide was determined as the efficiency of conjugation. The conjugation efficiency results are presented below in Table 3.

**Table 3. Conjugation efficiency of various cysteine engineered antibodies in complex with biotin**

| Antibody | Conjugation efficiency |
|---|---|
| 1C1 Wild type | 0% |
| 1C1 Ser134Cys | 53% |
| 1C1 Thr135Cys | 48% |
| 1C1 Ser136Cys | 63% |
| 1C1 Thr139Cys | 70% |

Results: In the peptide mapping analysis of various antibodies conjugated with bio tin, tbe specific peptide to which the biotin was conjugated was identified. Specifically, for each antibody, ICl Ser134Cys, ICl Thrl35Cys, ICI Scτl36Cys, ICl Thrl39Cys, tbe biotin was conjugated to the predicted unpaired cysteine. Further, the relative proportions of conjugated and unconjugated biotin species were analyzed to determine the conjugation efficiency. Presented in Table 3 are the conjugation efficiencies of tbe various cysteine engineered antibodies. The wild-type antibody displayed no conjugation as it does not retain a site readily available for conjugation. The cysteine engineered antibodies displayed biotin conjugation efficiencies ranging from 48% to 70%. These data demonstrate that site- specific conjugation occurs at a high level of efficiency for the various cysteine engineered antibodies.

While the foregoing invention has been described in some detail for purposes of clarity and understanding, it will be clear to one skilled in the art from a reading of this disclosure that various changes in form and detail can be made without departing from the true scope of the invention. For example, all the techniques and apparatus described above may be used in various combinations.

## Claims

1. A cysteine engineered antibody, wherein the cysteine engineered antibody comprises a substitution of one or more amino acids to a cysteine residue in the 131-139 region of the heavy chain of an antibody as defined by the EU Index numbering system, wherein the cysteine engineered antibody comprises at least one free thiol group.

2. The cysteine engineered antibody of claim 1, wherein the substituted amino acids are chosen from 131, 132, 134, 135, 136, and 139 of the antibody heavy chain, according to the EU Index numbering system.

3. The cysteine engineered antibody of claim 1 or 2, wherein said free thiol group is capable of chemical conjugation to a cytotoxic agent, chemotherapeutic agent, toxin, radionuclide, DNA, RNA, siRNA, microRNA, peptide nucleic acid, non-natural amino acid, peptide, enzyme, fluorescent tag, or biotin.

4. The cysteine engineered antibody of claim 3, wherein said cytotoxic agent is chosen from an anti-tubulin agent, a DNA minor groove binder, an anti-mitmaytansanoid, and an auristatin.

5. The cysteine engineered antibody of claim 3, wherein said chemotherapeutic agent is chosen from taxol, paclitaxel, doxorubicin, methotrexate, dolastatin, vinka alkaloids, methotrexate, and duocarmycin.

6. The cysteine engineered antibody of claim 3, wherein said toxin is chosen from abrin, brucine, cicutoxin, diphtheria toxin, botulism toxin, shiga toxin, endotoxin, tetanus toxin, pertussis toxin, anthrax toxin, cholera toxin, falcarinol, alfa toxin, geldanamycin, gelonin, lotaustralin, ricin, strychnine, and tetradotoxin.

7. The cysteine engineered antibody of claim 3, wherein said radionuclide is chosen from chromium (⁵¹Cr), cobalt (⁵⁷Co), fluorine (¹⁸F), gadolinium (¹⁵³Gd, ¹⁵⁹Gd), germanium (⁶⁸Ge), holmium (¹⁶⁶Ho), indium (¹¹⁵In, ¹¹³In, ¹¹²In, ¹¹¹In), iodine (¹³¹I, I¹²⁵, I^{123,} I¹²¹), lanthanium (¹⁴⁰La), luetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (⁴⁷Sc), selenium (⁷⁵Se), strontium (⁸⁵Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (⁹⁰Yb, ¹⁷⁵Yb), yttrium (⁹⁰Y), and zinc (⁶⁵Zn).

8. An isolated nucleic acid comprising a nucleotide sequence encoding a heavy chain variable domain of an antibody of any of claims 1-7.

9. A vector comprising the nucleic acid of claim 8.

10. A host cell comprising the vector of claim 9.

11. An antibody conjugate of the cysteine engineered antibody of any of claims 1-7.

12. A pharmaceutical composition comprising the antibody conjugate of claim 11.

13. The pharmaceutical composition of claim 12 for use in treating a disease or disorder, in a subject in need thereof, wherein the disease or disorder is cancer, an autoimmune disease or disorder, an inflammatory disease or disorder, or an infectious disease or disorder.

14. The cysteine engineered antibody of any of claims 1-7, wherein said antibody comprises inclusion of additional amino acid(s), wherein said inclusion of additional amino acid(s) occurs after a position chosen from residues 131, 132, 133, 134, 135, 136, 137, 138 and 139.

15. The cysteine engineered antibody of claim 14, wherein said antibody further comprises a second inclusion of additional amino acid(s) in the 131-139 loop region, wherein said second inclusion of additional amino acid(s) occurs after said first inclusion of additional amino acid(s), wherein said second inclusion of additional amino acid(s) occurs after a position chosen from residues 131, 132, 133, 134, 135, 136, 137, 138 and 139.

## Patentansprüche

1. Cystein-manipulierter Antikörper, wobei der Cystein-manipulierte Antikörper eine Substitution einer oder mehrerer Aminosäuren zu einem Cysteinrest in dem Bereich 131-139 der schweren Kette eines Antikörpers, wie definiert durch das EU-Index-Nummerierungssystem, umfasst, wobei der Cystein-manipulierte Antikörper wenigstens eine freie Thiolgruppe umfasst.

2. Cystein-manipulierter Antikörper gemäß Anspruch 1, wobei die substituierten Aminosäuren ausgewählt sind aus 131, 132, 134, 135, 136 und 139 der schweren Kette des Antikörpers gemäß dem EU-Index-Nummerierungssystem.

3. Cystein-manipulierter Antikörper gemäß Anspruch 1 oder 2, wobei die freie Thiolgruppe zur chemischen Konjugation an ein zytotoxisches Mittel, chemotherapeutisches Mittel, Toxin, Radionuklid, eine DNA, RNA, siRNA, mikroRNA, Peptidnukleinsäure, nichtnatürliche Aminosäure, ein Peptid, Enzym, eine Fluoreszenzmarkierung oder Biotin fähig ist.

4. Cystein-manipulierter Antikörper gemäß Anspruch 3, wobei das zytotoxische Mittel ausgewählt ist aus einem Anti-Tubulin-Mittel, einem DNA-Kleine-Furche-Binder, einem Anti-Mitmaytansanoid und einem Auristatin.

5. Cystein-manipulierter Antikörper gemäß Anspruch 3, wobei das chemotherapeutische Mittel ausgewählt ist aus Taxol, Paclitaxel, Doxorubicin, Methotrexat, Dolastatin, Vinka-Alkaloiden, Methotrexat und Duocarmycin.

6. Cystein-manipulierter Antikörper gemäß Anspruch 3, wobei das Toxin ausgewählt ist aus Abrin, Brucin, Cicutoxin, Diphtherietoxin, Botulismustoxin, Shigatoxin, Endotoxin, Tetanustoxin, Pertussistoxin, Anthraxtoxin, Choleratoxin, Falcarinol, Alfatoxin, Geldanamycin, Gelonin, Lotaustralin, Ricin, Strychnin und Tetradotoxin.

7. Cystein-manipulierter Antikörper gemäß Anspruch 3, wobei das Radionuklid ausgewählt ist aus Chrom (⁵¹Cr), Cobalt (⁵⁷Co), Fluor (¹⁸F), Gadolinium (¹⁵³Gd, ¹⁵⁹Gd), Germanium (⁶⁸Ge), Holmium (¹⁶⁶Ho), Indium (¹¹⁵In, ¹¹³In, ¹¹²In, ¹¹¹In), Iod (¹³¹I, ¹²⁵I, ¹²³I, ¹²¹I), Lanthan (¹⁴⁰La), Lutetium (¹⁷⁷Lu), Mangan (⁵⁴Mn), Molybdän (⁹⁹Mo), Palladium (¹⁰³Pd), Phosphor (³²P), Praseodym (¹⁴²Pr), Promethium (¹⁴⁹Pm), Rhenium (¹⁸⁶Re, ¹⁸⁸Re), Rhodium (¹⁰⁵Rh), Ruthenium (⁹⁷Ru), Samarium (¹⁵³Sm), Scandium (⁴⁷Sc), Selen (⁷⁵Se), Strontium (⁸⁵Sr), Schwefel (³⁵S), Technetium (⁹⁹Tc), Thallium (²⁰¹Ti), Zinn (¹¹³Sn, ¹¹⁷Sn), Tritium (³H), Xenon (¹³³Xe), Ytterbium (⁹⁰Yb, ¹⁷⁵Yb), Yttrium (⁹⁰Y) und Zink (⁶⁵Zn).

8. Isolierte Nukleinsäure, umfassend eine Nukleotidsequenz, die eine schwere-Kette-variable-Domäne eines Antikörpers gemäß einem der Ansprüche 1-7 kodiert.

9. Vektor, umfassend die Nukleinsäure gemäß Anspruch 8.

10. Wirtszelle, umfassend den Vektor gemäß Anspruch 9.

11. Antikörperkonjugat des Cystein-manipulierten Antikörpers gemäß einem der Ansprüche 1-7.

12. Pharmazeutische Zusammensetzung, umfassend das Antikörperkonjugat gemäß Anspruch 11.

13. Pharmazeutische Zusammensetzung gemäß Anspruch 12 für die Verwendung bei der Behandlung einer Erkrankung oder Störung bei einem Subjekt mit Bedarf daran, wobei die Erkrankung oder Störung Krebs, eine Autoimmunerkrankung oder -Störung, eine entzündliche Erkrankung oder Störung oder eine infektiöse Erkrankung oder Störung ist.

14. Cystein-manipulierter Antikörper gemäß einem der Ansprüche 1-7, wobei der Antikörper die Einfügung zusätzlicher Aminosäure(n) umfasst, wobei die Einfügung zusätzlicher Aminosäure(n) nach einer Position ausgewählt aus den Resten 131, 132, 133, 134, 135, 136, 137, 138 und 139 auftritt.

15. Cystein-manipulierter Antikörper gemäß Anspruch 14, wobei der Antikörper ferner eine zweite Einfügung zusätzlicher Aminosäure(n) in dem Schleifenbereich 131-139 umfasst, wobei die zweite Einfügung zusätzlicher Aminosäure(n) nach der ersten Einfügung zusätzlicher Aminosäure(n) auftritt, wobei die zweite Einfügung zusätzlicher Aminosäure(n) nach einer Position ausgewählt aus den Resten 131, 132, 133, 134, 135, 136, 137, 138 und 139 auftritt.

## Revendications

1. Anticorps cystéine-modifié, lequel anticorps cystéine-modifié comprend une substitution d'un ou plusieurs acides aminés au niveau d'un résidu de cystéine dans la région 131-139 de la chaîne lourde d'un anticorps comme défini par le système de numérotation de l'index EU, lequel anticorps cystéine-modifié comprend au moins un groupe thiol libre.

2. Anticorps cystéine-modifié selon la revendication 1, dans lequel les acides aminés substitués sont choisis parmi 131, 132, 134, 135, 136 et 139 de la chaîne lourde d'anticorps, conformément au système de numérotation de l'index EU.

3. Anticorps cystéine-modifié selon la revendication 1 ou 2, dans lequel ledit groupe thiol libre est capable d'une conjugaison chimique à un agent cytotoxique, un agent chimiothérapeutique, une toxine, un radionucléide, un ADN, un ARN, un ARNsi, un microARN, un acide nucléique peptidique, un acide aminé non naturel, un peptide, une enzyme, un marqueur fluorescent, ou la biotine.

4. Anticorps cystéine-modifié selon la revendication 3, dans lequel ledit agent cytotoxique est choisi parmi un agent anti-tubuline, un liant au sillon mineur d'ARN, un anti-mitmaytansanoïde, et une auristatine.

5. Anticorps cystéine-modifié selon la revendication 3, dans lequel ledit agent chimiothérapeutique est choisi parmi le taxol, le paclitaxel, la doxorubicine, le méthotrexate, la dolastatine, les alcaloïdes de pervenche, le méthotrexate, et la duocarmycine.

6. Anticorps cystéine-modifié selon la revendication 3, dans lequel ladite toxine est choisie parmi l'abrine, la brucine, la cicutoxine, la toxine diphtérique, la toxine botulique, la toxine dysentérique, les endotoxines, la toxine tétanique, la toxine de la coqueluche, la toxine de l'anthrax, la toxine cholérique, le falcarinol, les aflatoxines, la geldanamycine, la gélonine, la lautaustraline, la ricine, la strychnine, et la tétradotoxine.

7. Anticorps cystéine-modifié selon la revendication 3, dans lequel ledit radionucléide est choisi parmi le chrome (⁵¹Cr), le cobalt (⁵⁷Co), le fluor (¹⁸F), le gadolinium (¹⁵³Gd, ¹⁵⁹Gd), le germanium (⁶⁸Ge), l'holmium (¹⁰⁰Ho), l'indium (¹¹⁵In, ^{113I}n, ¹¹²In, ¹¹¹In), l'iode (¹³¹I, I¹²⁵, I¹²³, I¹²¹), le lanthane (¹⁴⁰La), le lutécium (¹⁷⁷Lu), le manganèse (⁵⁴Mn), le molybdène (⁹⁹Mo), le palladium (¹⁰³Pd), le phosphore (³²P), le praséodyme (¹⁴²Pr), le prométhium (¹⁴⁹Pm), le rhénium (¹⁸⁶Re, ¹⁸⁸Re), le rhodium (¹⁰⁵Rh), le ruthénium (⁹⁷Ru), le samarium (¹⁵³Sm), le scandium (⁴⁷Sc), le sélénium (⁷⁵Se), le strontium (⁸⁵Sr), le soufre (³⁵S), le technétium (⁹⁹Tc), le thallium (²⁰¹Ti), l'étain (¹¹³Sn, ¹¹⁷Sn), le tritium (³H), le xénon (¹³³Xe), l'ytterbium (⁹⁰Yb, ¹⁷⁵Yb), l'yttrium (⁹⁰Y) et le zinc (⁶⁵Zn).

8. Acide nucléique isolé comprenant une séquence de nucléotides codant un domaine variable de chaîne lourde d'un anticorps de l'une quelconque des revendications 1 à 7.

9. Vecteur comprenant l'acide nucléique de la revendication 8.

10. Cellule hôte comprenant le vecteur de la revendication 9.

11. Conjugué d'anticorps de l'anticorps cystéine-modifié de l'une quelconque des revendications 1 à 7.

12. Composition pharmaceutique comprenant le conjugué d'anticorps de la revendication 11.

13. Composition pharmaceutique selon la revendication 12, pour utilisation dans le traitement d'une maladie ou d'un trouble, chez un sujet en ayant besoin, dans laquelle la maladie ou le trouble est un cancer, une maladie ou un trouble auto-immun, une maladie ou un trouble inflammatoire, ou une maladie ou un trouble infectieux.

14. Anticorps cystéine-modifié selon l'une quelconque des revendications 1 à 7, lequel anticorps comprend l'inclusion d'un ou plusieurs acides aminés additionnels, dans lequel ladite inclusion du ou des acides aminés additionnels a lieu après une position choisie parmi les résidus 131, 132, 133, 134, 135, 136, 137, 138 et 139.

15. Anticorps cystéine-modifié selon la revendication 14, lequel anticorps comprend en outre une deuxième inclusion d'un ou plusieurs acides aminés additionnels dans la région de boucle 131-139, dans lequel ladite deuxième inclusion du ou des acides aminés additionnels a lieu après ladite première inclusion d'un ou plusieurs acides aminés additionnels, et dans lequel ladite deuxième inclusion du ou des acides aminés additionnels a lieu après une position choisie parmi les résidus 131, 132, 133, 134, 135, 136, 137, 138 et 139.
